# EUROPEAN PATENT APPLICATION

(11) **EP 4 786 492 A1**
(43) Date of publication of application: **05.08.2026**
(21) Application number: 24870838.0
(22) Date of filing: 26.09.2024
(51) Int. Cl.: C07K 16/28, A61K 39/395, C07K 19/00, A61P 35/00

(54) **MONOCLONAL ANTIBODY WHICH SPECIFICALLY RECOGNIZES DELTA-LIKE LIGAND 3, AND APPLICATION THEREOF**

(30) Priority: 27.09.2023 CN 202311254160; 28.11.2023 CN 202311604853
(71) Applicant: Simcere Zaiming Pharmaceutical Co., Ltd., Haikou, Hainan 570311 (CN)
(72) Inventor: HU, Yingying, Shanghai 201318 (CN); FU, Yayuan, Shanghai 201318 (CN); TANG, Renhong, Shanghai 201318 (CN)
(74) Representative: Müller-Boré & Partner Patentanwälte PartG mbB
(86) International application number: PCT/CN2024/121296
(87) International publication number: WO 2025/067294

(57) **Abstract**

The present invention relates to a DLL3 antibody and use thereof. In particular, the present disclosure relates to an antibody or an antigen-binding fragment thereof specifically binding to DLL3, an encoding nucleic acid thereof, a recombinant vector, a host cell, a preparation method, a pharmaceutical composition, and use in treating a disease, for example in treating a tumor. The present disclosure has important significance for developing DLL3 target-based therapeutic drugs and detection reagents.

## Description

The present invention claims priority to the Chinese Patent Application No. 202311254160.4 entitled "MONOCLONAL ANTIBODY SPECIFICALLY RECOGNIZING DELTA-LIKE LIGAND 3 AND USE THEREOF" and filed on September 27, 2023, and the Chinese Patent Application No. 202311604853.1 entitled "MONOCLONAL ANTIBODY SPECIFICALLY RECOGNIZING DELTA-LIKE LIGAND 3 AND USE THEREOF" and filed on November 28, 2023, which are incorporated herein by reference in their entireties along with the appendix.

### TECHNICAL FIELD

The present invention relates to the field of tumor immunotherapy or diagnosis, and in particular, to a monoclonal antibody specifically recognizing delta-like ligand 3 (DLL3) and use thereof.

### BACKGROUND

Delta-like ligand 3 (DLL3) is a member of the Notch ligand family. There are two types of spliceosomes. For the major spliceosome, the sequence homology of human and monkey extracellular segments is 91%, and the sequence homology of human and murine extracellular segments is 84%. DLL3 is distributed mainly in the Golgi apparatus and is partially localized at the cell membrane. Under physiological state, it mainly participates in the process of forming the somite in the embryonic development. DLL3-knockout mice show craniocerebral or neuronal developmental defects, and DLL3-mutant people show defects such as vertebral and rib hypoplasia. Unlike other ligands of the Notch family, which activate signaling pathways, DLL3 can interact with DLL1 or Notch1 in cis or trans manner to inhibit the activation of signaling pathways.

Neuroendocrine lung cancer accounts for 20% of the total lung cancer cases, with small cell lung cancer accounting for about 14%. Most patients have concurrent hematogenous metastasis, and only about 1/ 3 limited-stage patients have foci limited to the chest. The current standard first-line therapy for patients with extensive-stage small cell lung cancer includes radiotherapy, combined use of carboplatin + etoposide + atezolizumab, combined use of carboplatin + etoposide + durvalumab, and combined use of cisplatin + etoposide + durvalumab. Untreated patients are very sensitive to chemotherapy and radiotherapy, but most patients relapse within a short period of time due to a small number of cancer cells that remain and the insensitivity to tumor stem cell therapy. For the patients receiving the first-line therapy, the median progression-free survival is 2-3 months, the median overall survival is 8-13 months, while the 5-year survival is only less than 5%. For relapsed or refractory patients, there is currently a lack of effective treatment options. The recommended second-line therapy for patients having a relapse within 6 months includes topotecan and lurbinectedin, and other recommended therapy includes the antibody pembrolizumab and the like. For patients having a relapse after more than 6 months, it is recommended to maintain the original therapy. In view of the current state of treatment, there is a need to provide new effective therapies for patients.

DLL3 is barely expressed in normal tissues, and in the nervous system and tissues such as pancreas and testis, its mRNA transcription level is high, but its protein expression level is low. Meanwhile, DLL3 is substantially localized in the cell matrix in normal tissues. DLL3 has high expression level in small cell lung cancer sample and is distributed in cell membrane and cell matrix, and it has a tendency to be related to disease grading, but this tendency is not significant. DLL3 is barely expressed in normal lung tissues and lung squamous cell carcinoma. In addition, DLL3 is also expressed in other neuroendocrine-related tumors, such as melanoma, glioblastoma multiforme, small cell carcinoma of the bladder, and the like. According to StemCentrx, the H-score of DLL3 expression is over 100 in samples from 65% of patents with large cell neuroendocrine carcinoma of the lung, 72% of untreated small cell lung cancer patients, and 85% of relapsed and refractory small cell lung cancer patients. According to Abbvie, 83% of the samples from small cell lung cancer patients were DLL3 positive, with 32% of the samples being DLL3 strong positive (50% of cells being positive). Thus, DLL3 can be considered one of the effective targets for small cell lung cancer or neuroendocrine cancers. ADC products, such as Rova-T, have provided a powerful proof-of-concept for the clinical efficacy of DLL3, but because ADC products rely on antigen expression on the cell membrane surface and toxin selection, positive data on overall survival haven't been obtained in clinical phase III. The present invention is intended to obtain antibody sequences targeting DLL3 to produce more pharmaceutical forms targeting DLL3.

### SUMMARY

By providing a novel antibody or antigen-binding fragment thereof specifically binding to DLL3 that regulates Notch signaling through the inhibition of the function of DLL3, the present invention provides a novel tumor therapy.

In a first aspect of the present invention, provided is an antibody or an antigen-binding fragment thereof specifically binding to human delta-like ligand 3 (DLL3), wherein the antibody or the antigen-binding fragment thereof comprises a light chain variable region (VL) and a heavy chain variable region (VH), wherein
(1) the light chain variable region comprises an LCDR1, an LCDR2, and an LCDR3, and the LCDR1, the LCDR2, and the LCDR3 are an LCDR1, an LCDR2, and an LCDR3 of a VL domain set forth in any one of SEQ ID NOs:19, 21, 23, 25, 27, 29, 31, 33, 35, 37, 39, 41, 43, 45, 47, 49, 338-339, 343-346, 349-355, 359-361, 364-365, and 374-375; and
(2) the heavy chain variable region comprises an HCDR1, an HCDR2, and an HCDR3, and the HCDR1, the HCDR2, and the HCDR3 are an HCDR1, an HCDR2, and an HCDR3 of a VH domain set forth in any one of SEQ ID NOs:18, 20, 22, 24, 26, 28, 30, 32, 34, 36, 38, 40, 42, 44, 46, 48, 340-342, 347, 356, 362-363, 366-370, and 376-377.

In some specific embodiments, the LCDR1, the LCDR2, and the LCDR3 are determined according to the Kabat numbering scheme, the IMGT numbering scheme, and/or the Chothia numbering scheme.

In some specific embodiments, the HCDR1, the HCDR2, and the HCDR3 are determined according to the Kabat numbering scheme, the IMGT numbering scheme, and/or the Chothia numbering scheme.

In some specific embodiments, the antibody or the antigen-binding fragment thereof comprises the following LCDR1, LCDR2, LCDR3, HCDR1, HCDR2, and HCDR3:
(1) according to the Kabat numbering scheme, LCDR1-3 and HCDR1-3 have the amino acid sequences set forth in SEQ ID NOs: 50-145, 357-358, 371-373, and 378 or a sequence combination having 1, 2, 3, or more amino acid insertions, deletions, and/or substitutions compared with the amino acid sequences set forth in SEQ ID NOs: 50-145;
(2) according to the IMGT numbering scheme, LCDR1-3 and HCDR1-3 have the amino acid sequences set forth in SEQ ID NOs: 146-241 or a sequence combination having 1, 2, 3, or more amino acid insertions, deletions, and/or substitutions compared with the amino acid sequences set forth in SEQ ID NOs: 146-241;
(3) according to the Chothia numbering scheme, LCDR1-3 and HCDR1-3 have the amino acid sequences set forth in SEQ ID NOs: 242-337 or a sequence combination having 1, 2, 3, or more amino acid insertions, deletions, and/or substitutions compared with the amino acid sequences set forth in SEQ ID NOs: 242-337.

In some specific embodiments, provided is the antibody or the antigen-binding fragment thereof, wherein:
(1) the light chain variable region comprises the sequences set forth in SEQ ID NOs: 19, 21, 23, 25, 27, 29, 31, 33, 35, 37, 39, 41, 43, 45, 47, 49, 338-339, 343-346, 349-355, 359-361, 364-365, and 374-375 or sequences having 80%, 85%, 90%, 95%, 96%, 97%, 98%, 99%, or higher identity to the sequences set forth in SEQ ID NOs: 19, 21, 23, 25, 27, 29, 31, 33, 35, 37, 39, 41, 43, 45, 47, 49, 338-339, 343-346, 349-355, 359-361, 364-365, and 374-375; and
(2) the heavy chain variable region comprises the sequences set forth in SEQ ID NOs: 18, 20, 22, 24, 26, 28, 30, 32, 34, 36, 38, 40, 42, 44, 46, 48, 340-342, 347, 356, 362-363, 366-370, and 376-377 or sequences having 80%, 85%, 90%, 95%, 96%, 97%, 98%, 99%, or higher identity to the sequences set forth in SEQ ID NOs: 18, 20, 22, 24, 26, 28, 30, 32, 34, 36, 38, 40, 42, 44, 46, 48, 340-342, 347, 356, 362-363, 366-370, and 376-377.

In some specific embodiments, provided is the antibody or the antigen-binding fragment thereof, wherein the antibody or the antigen-binding fragment thereof is chimeric, humanized, or fully human-derived.

In some specific embodiments, provided is the antibody or the antigen-binding fragment thereof, wherein the antibody or the antigen-binding fragment thereof is capable of binding to human, monkey, or murine delta-like ligand 3 (DLL3).

In some specific embodiments, provided is the antibody or the antigen-binding fragment thereof, wherein the antibody or the antigen-binding fragment thereof may further comprise a constant region sequence in any one of human or murine IgG1, IgG2, IgG3, IgG4, IgA, IgM, IgE, and IgD antibodies, preferably a constant region sequence of a human or murine IgG1, IgG2, IgG3, or IgG4 antibody or a constant region sequence of a human or murine IgG1, IgG2, IgG3, or IgG4 antibody with a mutation;
further, the antibody or the antigen-binding fragment thereof is also conjugated to a therapeutic agent or a tracer;
preferably, the therapeutic agent is selected from a radioisotope, a chemotherapeutic agent, a cytotoxic agent, or an immunomodulator, and the tracer is selected from a radiocontrast medium, a paramagnetic ion, a metal, a fluorescent label, a chemiluminescent label, an ultrasound contrast agent, and a photosensitizer;
more preferably, the cytotoxic agent is selected from alkaloids, methotrexate, anthracycline antibiotics (doxorubicin), pyrrolobenzodiazepine (PBD) or gemcitabine, cytarabine, tegafur, ifosfamide, dacarbazine, and oxaliplatin; more preferably, the cytotoxic agent is taxanes.

In some specific embodiments, the antigen-binding fragment is selected from one or more of a F(ab')₂, a Fab', a Fab, an Fv, an scFv, a nanobody, or an affibody.

In a second aspect of the present invention, provided is a multispecific antigen-binding molecule, wherein the multispecific antigen-binding molecule comprises the DLL3 antibody or the antigen-binding fragment thereof, an antigen-binding molecule that binds to an antigen other than DLL3, and/or an antigen-binding molecule that binds to a DLL3 epitope different from a DLL3 epitope to which the DLL3 antibody or the antigen-binding fragment thereof binds;
preferably, the additional antigen-binding molecule is an antibody or an antigen-binding fragment thereof;
preferably, the multispecific antigen-binding molecule may be bispecific, trispecific, or tetraspecific;
preferably, the multispecific antigen-binding molecule may be divalent, trivalent, tetravalent, pentavalent, or hexavalent.

In a third aspect of the present invention, provided is an isolated nucleic acid fragment, wherein the nucleic acid fragment encodes any one of the antibodies or antigen-binding fragments thereof described above or the multispecific antigen-binding molecule described above.

In a fourth aspect of the present invention, provided is a recombinant vector, wherein the recombinant vector comprises the isolated nucleic acid fragment described above.

In a fifth aspect of the present invention, provided is a host cell, wherein the host cell comprises the recombinant vector described above; preferably, the cell is a prokaryotic cell or a eukaryotic cell, e.g., a bacterium (*E. coli*), a fungus (yeast), an insect cell, or a mammalian cell (a CHO cell line or a 293T cell line).

In a sixth aspect of the present invention, provided is a method for preparing a product comprising the DLL3 antibody or the antigen-binding fragment thereof or the multispecific antigen-binding molecule described above, wherein the method comprises: culturing the host cell and isolating an antibody, an antigen-binding fragment, or a multispecific antigen-binding molecule expressed by the cell.

In a seventh aspect of the present invention, provided is a pharmaceutical composition, wherein the pharmaceutical composition comprises the DLL3 antibody or the antigen-binding fragment thereof, the multispecific antigen-binding molecule, the nucleic acid fragment, the vector, or a product prepared according to the foregoing method; optionally, the pharmaceutical composition further comprises a pharmaceutically acceptable carrier, a diluent, or an adjuvant.

In some specific embodiments, the pharmaceutical composition further comprises an additional antineoplastic agent.

In an eighth aspect of the present invention, provided is a method for treating a tumor or cancer, wherein the method comprises administering to a subject an effective amount of the antibody or the antigen-binding fragment thereof, the multispecific antigen-binding molecule, the nucleic acid fragment, the vector, a product prepared according to the foregoing method, or the pharmaceutical composition; preferably, the tumor or cancer is selected from a solid tumor, a hematologic tumor, or a DLL3-expressing infiltrative cancer;
preferably, the tumor or cancer is selected from at least one of small cell lung cancer, glioma, pancreatic cancer, melanoma, breast cancer, pituitary tumor, endometrioma, acute myeloid leukemia, liver cancer, bladder cancer, colon cancer, prostate cancer, kidney cancer, and esophageal cancer.

In a ninth aspect of the present invention, provided is use of the antibody or the antigen-binding fragment thereof, the multispecific antigen-binding molecule, the nucleic acid fragment, the vector, a product prepared according to the foregoing method, or the pharmaceutical composition in preparing a medicament for treating a tumor or cancer; preferably, the tumor or cancer is selected from a solid tumor, a hematologic tumor, or a DLL3-expressing infiltrative cancer;
preferably, the tumor or cancer is selected from at least one of small cell lung cancer, glioma, pancreatic cancer, melanoma, breast cancer, pituitary tumor, endometrioma, acute myeloid leukemia, liver cancer, bladder cancer, colon cancer, prostate cancer, kidney cancer, and esophageal cancer.

In a tenth aspect of the present invention, provided is a kit, wherein the kit comprises the antibody or the antigen-binding fragment thereof, the multispecific antigen-binding molecule, the nucleic acid fragment, the vector, a product prepared according to the foregoing method, or the pharmaceutical composition.

In an eleventh aspect of the present invention, provided is a method for detecting expression of DLL3 in a biological sample, wherein the method comprises contacting the biological sample with the antibody or the antigen-binding fragment thereof under conditions that allow the antibody or the antigen-binding fragment thereof and DLL3 to form a complex; preferably, the method further comprises detecting formation of the complex and indicating presence or an expression level of DLL3 in the sample.

In a twelfth aspect of the present invention, provided is use of the antibody or the antigen-binding fragment thereof in preparing a DLL3 detection reagent.

### Terminology and Definitions

Unless otherwise defined herein, scientific and technical terms used in correlation with the present invention shall have the meanings that are commonly understood by those skilled in the art.

Furthermore, unless otherwise stated herein, terms used in the singular form herein shall include the plural form, and vice versa. More specifically, as used in this specification and the appended claims, unless otherwise clearly indicated, the singular forms "a", "an", and "the" include referents in the plural form.

The terms "include", "comprise", and "have" herein are used interchangeably and are intended to indicate the inclusion of a solution, implying that there may be elements other than those listed in the solution. Meanwhile, it should be understood that the descriptions "include", "comprise", and "have" used herein also provide the solution of "consist of ...".

The term "and/or" used herein includes the meanings of "and", "or", and "all or any other combination of elements linked by the term".

Delta-like ligand 3 (DLL3) is a single transmembrane protein attached to the cell surface and is a member of the Notch ligand family. The human DLL3 gene is localized on chromosome 19q13 and has an open reading frame length of approximately 1800 bp. The human DLL3 protein consists of 619 amino acids, and its complete structure comprises one DSL domain, one intracellular domain, and six epidermal growth factor-like domains. The DSL gene sequence at the N-terminus of the extracellular domain is highly conserved in the ligand family, and the extracellular domain is an essential functional domain for binding to the Notch receptor. DLL3 has a short intracellular domain and its function remains unclear. Researches show that DLL3 is highly expressed in SCLC and other neuroendocrine tumors, but is rarely expressed in normal tissues. Activation of DLL3 may exert a pro-cancer or anti-cancer effect. DLL3 is widely expressed in human cancers, including small cell lung cancer, glioma, pancreatic cancer, melanoma, breast cancer, pituitary tumor, endometrioma, acute myeloid leukemia, liver cancer, bladder cancer, colon cancer, prostate cancer, kidney cancer, esophageal cancer, and the like.

The term "specifically bind to" herein refers to that an antigen-binding molecule (e.g., an antibody) specifically binds to an antigen and substantially identical antigens, generally with high affinity, but does not bind to unrelated antigens with high affinity. The affinity is generally reflected in an equilibrium dissociation constant (KD), where a relatively low KD indicates a relatively high affinity. In the case of antibodies, high affinity generally means having a KD of about 10⁻⁶ M or less, 10⁻⁷ M or less, about 10⁻⁸ M or less, or about 10⁻⁹ M or less. The KD is calculated as follows: KD = Kd/Ka, where Kd represents the dissociation rate and Ka represents the association rate. The equilibrium dissociation constant KD can be measured by methods well known in the art, such as surface plasmon resonance (e.g., Biacore) or equilibrium dialysis.

The term "antigen-binding molecule" herein is used in its broadest sense and refers to a molecule that specifically binds to an antigen. Illustratively, the antigen-binding molecule includes, but is not limited to, an antibody or an antibody mimetic. "Antibody mimetic" refers to an organic compound or a binding domain that is capable of specifically binding to an antigen, but is not structurally related to an antibody. Illustratively, the antibody mimetic includes, but is not limited to, affibody, affitin, affilin, a designed ankyrin repeat protein (DARPin), a nucleic acid aptamer, and a Kunitz domain peptide.

The term "antibody" herein is used in its broadest sense and refers to a polypeptide or a combination of polypeptides that comprises sufficient sequence from an immunoglobulin heavy chain variable region and/or sufficient sequence from an immunoglobulin light chain variable region to be capable of specifically binding to an antigen. "Antibody" herein encompasses various forms and various structures as long as they exhibit the desired antigen binding activity. The "antibody" herein includes alternative protein scaffolds or artificial scaffolds having grafted complementarity determining regions (CDRs) or CDR derivatives. Such scaffolds include antibody-derived scaffolds comprising mutations introduced to, for example, stabilize the three-dimensional structure of the antibody, and fully synthetic scaffolds comprising, for example, biocompatible polymers. See, e.g., Korndorfer, I. P., Beste, G. & Skerra, A. (2003). Proteins, 53, 121-129.; Roque, A. C. A., Lowe, C. R. & Taipa, M. A. Antibodies and genetically engineered related molecules: production and purification. Biotechnol. Prog. 20, 639-654 (2004) (which is incorporated herein by reference). Such scaffolds may also include non-antibody-derived scaffolds, such as scaffold proteins known in the art to be useful for grafting CDRs, including but not limited to, tenascin, fibronectin, peptide aptamers, and the like.

The term "antibody" includes intact antibodies and any antigen-binding fragments (i.e., "antigen-binding moiety") or single chains thereof. The "antibody" refers to a glycoprotein comprising at least two heavy (H) chains and two light (L) chains that are linked together by disulfide bonds, or an antigen-binding moiety thereof. Each heavy chain consists of a heavy chain variable region (abbreviated herein as VH) and a heavy chain constant region. The heavy chain constant region consists of three domains: CH1, CH2, and CH3. Each light chain consists of a light chain variable region (abbreviated herein as VL) and a light chain constant region. The light chain constant region consists of one domain CL. The VH region and the VL region can be further subdivided into hypervariable regions called complementarity determining regions (CDRs), which are scattered among regions that are more conserved, termed framework regions (FRs). Each VH or VL consists of three CDRs and four FRs, which are arranged from the N-terminus to the C-terminus in the following order: FR1, CDR1, FR2, CDR2, FR3, CDR3, and FR4. The variable regions of the heavy and light chains comprise binding domains that can interact with antigens. The constant regions of the antibody may mediate the binding of the immunoglobulin to a host tissue or factor, and the host tissue or factor includes various cells (e.g., effector cells) of the immune system and the first component (C1q) of the classical complement system. The heavy chain constant regions of immunoglobulins differ in their amino acid composition and arrangement, and thus in their antigenicity. Accordingly, the "immunoglobulin" herein can be divided into five classes, or isoforms of immunoglobulins, i.e., IgM, IgD, IgG, IgA, and IgE, with their corresponding heavy chains being µ, δ, γ, α, and ε chains, respectively. The Ig of the same class may also be divided into different subclasses according to the differences in the amino acid composition of the hinge regions and the number and location of disulfide bonds in the heavy chains. For example, IgG may be divided into IgG1, IgG2, IgG3, and IgG4, and IgA may be divided into IgA1 and IgA2. The light chains can be divided into κ or λ chains according to the differences in the constant regions. Each of the five classes of Ig may have a κ chain or λ chain.

The "antibody" herein also includes antibodies that do not comprise a light chain, e.g., heavy chain antibodies (HCAbs) produced by *Camelus dromedarius*, *Camelus bactrianus*, *Lama glama*, *Lama guanicoe*, *Vicugna pacos*, and the like, as well as immunoglobulin new antigen receptors (IgNARs) found in *Chondrichthyes*, e.g., shark.

The term "antibody" herein may be derived from any animal, including but not limited to human and non-human animals which may be selected from primates, mammals, rodents, and vertebrates, such as *Camelidae* species, *Lama glama*, *Lama guanicoe*, *Vicugna pacos*, sheep, rabbits, mice, rats, or *Chondrichthyes* (e.g., shark).

The term "heavy chain antibody" herein refers to an antibody lacking a light chain of a conventional antibody. The term specifically includes, but is not limited to, homodimeric antibodies comprising a VH antigen-binding domain and CH2 and CH3 constant domains in the absence of a CH1 domain.

The term "multispecific" herein refers to the ability of an antibody or an antigen-binding fragment thereof to bind to, e.g., different antigens or to at least two different epitopes on the same antigen. Thus, the terms such as "bispecific", "trispecific", and "tetraspecific" refer to the number of different epitopes to which an antibody can bind. For example, conventional monospecific IgG antibodies have two identical antigen-binding sites (paratopes) and therefore can only bind to the same epitope (rather than to different epitopes). In contrast, multispecific antibodies have at least two different types of paratopes/binding sites and can therefore bind to at least two different epitopes. As used herein, the "complementarity determining region" refers to the antigen-binding site of an antibody. In addition, a single "specificity" may refer to one, two, three, or more identical complementarity determining regions in a single antibody (the actual number of complementarity determining regions/binding sites in a single antibody molecule is referred to as the "valency"). For example, a single native IgG antibody is monospecific and bivalent, since it has two identical paratopes. Accordingly, a multispecific antibody comprises at least two (different) complementarity determining regions/binding sites. Thus, the term "multispecific antibody" refers to an antibody having more than one paratope and the ability to bind to two or more different epitopes. The term "multispecific antibody" particularly includes bispecific antibodies as defined above, but typically also includes proteins, e.g., antibodies and scaffolds specifically binding to three or more different epitopes, i.e., antibodies having three or more paratopes/binding sites.

The term "valent" herein refers to the presence of a specified number of binding sites in an antibody/antigen-binding molecule. Thus, the terms "monovalent", "divalent", "tetravalent", and "hexavalent" refer to the presence of one binding site, two binding sites, four binding sites, and six binding sites, respectively, in an antibody/antigen-binding molecule.

The "full-length antibody" and "intact antibody" are used interchangeably herein and refer to an antibody having a structure substantially similar to that of a natural antibody.

The terms "antigen-binding fragment" and "antibody fragment" herein are used interchangeably and refer to a fragment that does not have the entire structure of an intact antibody, but comprises only a portion of the intact antibody or a variant of the portion that retains the ability to bind to an antigen. Illustratively, the "antigen-binding fragment" or "antibody fragment" used herein includes, but is not limited to, a Fab, a F(ab')₂, a Fab', a Fab'-SH, an Fd, an Fv, an scFv, a diabody, an affibody, and a single domain antibody.

The term "chimeric antibody" herein refers to an antibody having a variable region sequence of an immunoglobulin derived from one organism (e.g., rat, mouse, rabbit, or *Vicugna pacos*) and a constant region of an immunoglobulin derived from a different organism (e.g., human). Methods for producing the chimeric antibody are known in the art. See, e.g., Patent US5807715A; Morrison, 1985, Science 229(4719): 1202-1207. Transfectomas Provide Novel Chimeric Antibodies; Gillies et al., J Immunol Methods. 1989 Dec 20, 125(1-2):191-202; the above is incorporated herein by reference.

The term "humanized antibody" herein refers to a genetically engineered non-human antibody that has an amino acid sequence modified to increase the homology to the sequence of a human antibody. Generally, all or part of the CDRs of a humanized antibody is derived from a non-human antibody (donor antibody), and all or part of the non-CDRs (e.g., variable region FRs and/or constant regions) is derived from a human immunoglobulin (receptor antibody). The humanized antibody generally retains or partially retains the desired properties of the donor antibody, including but not limited to, antigen specificity, affinity, reactivity, the ability to increase the activity of immune cells, the ability to enhance an immune response, or the like.

The term "fully human antibody" herein refers to an antibody having variable regions in which both the FRs and CDRs are derived from human germline immunoglobulin sequences. Furthermore, if the antibody comprises constant regions, the constant regions are also derived from human germline immunoglobulin sequences. The fully human antibody herein may include amino acid residues that are not encoded by human germline immunoglobulin sequences (e.g., mutations introduced by random or site-specific mutagenesis *in vitro* or by somatic mutations *in vivo*). However, "fully human antibody" herein does not include antibodies in which CDR sequences derived from the germline of another mammalian species (e.g., mouse) have been grafted onto human framework sequences.

The term "variable region" herein refers to a region of a heavy or light chain of an antibody involved in the binding of the antibody to an antigen. The "heavy chain variable region" is used interchangeably with "VH" and "HCVR", and the "light chain variable region" is used interchangeably with "VL" and "LCVR". The heavy and light chain variable domains of natural antibodies generally have similar structures, each domain containing four conserved framework regions (FRs) and three hypervariable regions (HVRs). See, e.g., Kindt et al., Kuby Immunology, 6th ed.,W.H.Freeman and Co., p.91(2007), which is incorporated herein by reference. A single VH or VL domain may be sufficient to provide antigen binding specificity.

The terms "complementarity determining region" and "CDR" are used interchangeably herein and generally refer to hypervariable regions (HVRs) found in both the light and heavy chain variable domains. The highly conserved portions of the variable domains are called framework regions (FRs). As known in the art, the amino acid positions representing the hypervariable regions of an antibody may vary according to the context and various definitions known in the art. Some positions within a variable domain may be considered to be hybrid hypervariable positions in that such positions may be considered to be within a hypervariable region according to one set of standards (such as IMGT or KABAT) but outside a hypervariable region according to a different set of standards (such as KABAT or IMGT). One or more of such positions may also be found in extended hypervariable regions. The present disclosure includes antibodies comprising modifications in such hybrid hypervariable positions. The heavy chain variable region CDRs may be abbreviated as HCDRs and the light chain variable region CDRs as LCDRs. The variable domains of native heavy and light chains each comprise four framework regions, mainly in a sheet configuration, connected by three CDRs (CDR1, CDR2, and CDR3) that form loops connecting the sheet structures, and in some cases form part of the sheet structures. The CDRs in each chain are held tightly together by the FR regions, in the order of FR1-CDR1-FR2-CDR2-FR3-CDR3-FR4, and contribute to the formation of the antigen-binding site of the antibody with CDRs from other antibody chains (see Kabat et al., Sequences of Proteins of Immunological Interest, National Institute of Health, Bethesda, Md. 1987, which is incorporated herein by reference).

For further description of the CDRs, see Kabat et al., J. Biol. Chem., 252: 6609-6616 (1977); Kabat et al., United States Department of Health and Human Services, Sequences of proteins of immunological interest (1991); Chothia et al., J. Mol. Biol. 196: 901-917 (1987); Al-Lazikani B. et al., J. Mol. Biol., 273: 927-948 (1997); MacCallum et al., J. Mol. Biol. 262: 732-745 (1996); Abhinandan and Martin, Mol. Immunol., 45: 3832-3839 (2008); Lefranc M.P. et al., Dev. Comp. Immunol., 27: 55-77 (2003); and Honegger and Plückthun, J. Mol. Biol., 309: 657-670 (2001). The "CDR" herein may be labeled and defined in a manner well known in the art, including but not limited to Kabat numbering scheme, Chothia numbering scheme, or IMGT numbering scheme; the tool websites used include, but are not limited to, AbRSA site (http://cao.labshare.cn/AbRSA/cdrs.php), abYsis site (www.abysis.org/abysis/sequence_input/key_annotation/key_annotation.cgi), and IMGT site (http://www.imgt.org/3Dstructure-DB/cgi/DomainGapAlign.cgi#results). The CDR herein includes overlaps and subsets of amino acid residues defined in different ways. (the above is incorporated herein by reference).

The term "Kabat numbering scheme" herein generally refers to the immunoglobulin alignment and numbering scheme proposed by Elvin A. Kabat (see, e.g., Kabat et al., Sequences of Proteins of Immunological Interest, 5th Ed. Public Health Service, National Institutes of Health, Bethesda, Md., 1991, which is incorporated herein by reference).

The term "Chothia numbering scheme" herein generally refers to the immunoglobulin numbering scheme proposed by Chothia et al., which is a classical rule for identifying CDR region boundaries based on the position of structural loop regions (see, e.g., Chothia & Lesk (1987) J. Mol. Biol. 196: 901-917; Chothia et al., (1989) Nature 342: 878-883, which is incorporated herein by reference).

The term "IMGT numbering scheme" herein generally refers to a numbering scheme based on the international ImMunoGeneTics information system (IMGT) initiated by Lefranc et al. See Lefranc et al., Dev. Comparat. Immunol. 27: 55-77, 2003, which is incorporated herein by reference.

The term "heavy chain constant region" herein refers to the carboxyl-terminal portion of an antibody heavy chain that is not directly involved in the binding of the antibody to an antigen, but exhibits effector functions, such as interaction with an Fc receptor; the heavy chain constant region has a more conserved amino acid sequence relative to the variable domain of the antibody. The "heavy chain constant region" may be selected from a CH1 domain, a hinge region, a CH2 domain, a CH3 domain, and a variant or fragment thereof. The "heavy chain constant region" includes a "full-length heavy chain constant region" having a structure substantially similar to that of a natural antibody constant region, while the "heavy chain constant region fragment" includes only "a portion of the full-length heavy chain constant region". Illustratively, a typical "full-length antibody heavy chain constant region" consists of the CH1 domain-hinge region-CH2 domain-CH3 domain. When the antibody is IgE, it further comprises a CH4 domain; when the antibody is a heavy chain antibody, it does not comprise a CH1 domain. Illustratively, a typical "heavy chain constant region fragment" may be selected from an Fc domain or a CH3 domain.

The term "light chain constant region" herein refers to the carboxyl-terminal portion of an antibody light chain that is not directly involved in the binding of the antibody to an antigen. The light chain constant region may be selected from a constant κ domain and a constant λ domain.

The term "Fc region" herein is used to define the C-terminal region of an antibody heavy chain containing at least a portion of the constant region. The "Fc region" includes native Fc regions and variant Fc regions. Illustratively, the human IgG heavy chain Fc region may extend from Cys226 or Pro230 to the carboxyl-terminus of the heavy chain. However, an antibody produced by a host cell may undergo post-translational cleavage of one or more, particularly one or two, amino acids from the C-terminus of the heavy chain. Therefore, an antibody produced by a host cell via the expression of a specific nucleic acid molecule encoding a full-length heavy chain may include a full-length heavy chain, or may include cleaved variants of the full-length heavy chain. It may be such a situation when the last two C-terminal amino acids of the heavy chain are glycine (G446) and lysine (K447, in accordance with the Kabat EU index). Therefore, the C-terminal lysine (Lys447), or the C-terminal glycine (Gly446) and lysine (Lys447) of the Fc region, may or may not be present. Typically, the IgG Fc region comprises IgG CH2 and IgG CH3 domains; optionally, the IgG Fc region may further comprise a complete or partial hinge region, but does not comprise a CH1 domain. The "CH2 domain" of the human IgG Fc region typically extends from an amino acid residue at about position 231 to an amino acid residue at about position 340. In one embodiment, a carbohydrate chain is attached to the CH2 domain. The CH2 domain herein may be a native CH2 domain or a variant CH2 domain. The "CH3 domain" comprises the residues in the Fc region at the C-terminus of the CH2 domain (i.e., from an amino acid residue at about position 341 to an amino acid residue at about position 447 of the IgG). The CH3 region herein may be a CH3 domain of a native sequence or a variant CH3 domain (e.g., a CH3 domain having a "protuberance" ("knob") introduced in one strand and a "cavity" ("hole") correspondingly introduced in the other strand; see U.S. Pat. No.5,821,333, which is explicitly incorporated herein by reference). As described herein, such variant CH3 domains may be used to promote the heterodimerization of two non-identical antibody heavy chains.

Unless otherwise specified herein, the numbering of amino acid residues in the Fc region or constant region conforms to the EU numbering scheme, also known as the EU index, as described in Kabat et al., Sequences of Proteins of Immunological Interest, 5th Ed., Public Health Service, National Institutes of Health, Bethesda, MD, 1991, which is explicitly incorporated herein by reference.

The term "Fc variant" herein refers to a change in the structure or functionality of an Fc caused by the presence of one or more amino acid substitution, insertion, or deletion mutations at appropriate sites on the Fc. The "inter-Fc variant interaction" refers to the formation of space-filling effects, electrostatic steering, hydrogen bonding, hydrophobic interactions, etc., between mutationally designed Fc variants. The inter-Fc variant interaction contributes to the formation of stable heterodimeric proteins. Preferred mutational designs are those of the "knob-into-hole" format.

Techniques for designing mutations in Fc variants have been widely used in the art to produce bispecific antibodies or heterodimeric Fc fusion protein forms. Representatives are the "knob-into-hole" format proposed by Cater, et al. (Protein Engineering, vol. 9 no.7, pp. 617-621, 1996); the Fc-containing heterodimeric form produced by Amgen Inc. using electrostatic steering (US 20100286374 A1); the heterodimeric form (SEEDbodies) formed by IgG/Ig chain exchange proposed by Jonathan H. Davis, et al. (Protein Engineering, Design&Selection pp. 1-8, 2010); the bispecific molecule produced by the DuoBody (Science, 2007.317(5844)) platform of Genmab A/S; the heterodimeric protein form given by Xencor by combining structural calculation and Fc amino acid mutation and different mechanisms of action (mAbs 3:6, 546-557; November/December 2011); the heterodimeric protein form produced by Alphamab Oncology, Suzhou, via the charge network-based Fc modification method (CN201110459100.7); and other genetic engineering methods to give heterodimeric functional protein by means based on Fc amino acid change or functional modification. The knob/hole structures on the Fc variant fragment described herein refer to that the two Fc fragments are mutated separately and can be bound by the "knob-into-hole" format after mutation. The "knob-into-hole" model of Cater et al., is preferred for site-directed mutagenesis on the Fc region, such that the resulting first and second Fc variants can bind together in the "knob-into-hole" form to give a heterodimer. The selection of a particular immunoglobulin Fc region from a particular immunoglobulin class and subclass is within the knowledge of those skilled in the art. Preferred are the Fc regions of human IgG1, IgG2, IgG3, or IgG4 antibodies, and more preferred is the Fc region of human IgG1 antibody. One of the first Fc variant or the second Fc variant is randomly selected for the knob mutation and the other for hole mutation. (The above is incorporated herein by reference).

The term "conservative amino acid" herein generally refers to amino acids that belong to the same class or have similar characteristics (e.g., charge, side chain size, hydrophobicity, hydrophilicity, backbone conformation, and rigidity). Illustratively, the amino acids in each of the following groups belong to conservative amino acid residues of each other, and substitutions of amino acid residues within the groups belong to conservative amino acid substitutions:
1) alanine (A), serine (S), and threonine (T);
2) aspartic acid (D) and glutamic acid (E);
3) asparagine (N) and glutamine (Q);
4) arginine (R), lysine (K), and histidine (H);
5) isoleucine (I), leucine (L), methionine (M), and valine (V); and
6) phenylalanine (F), tyrosine (Y), and tryptophan (W).

The term "identity" can be acquired by a calculation as follows: to determine the percent "identity" of two amino acid sequences or two nucleic acid sequences, the sequences are aligned for optimal comparison purposes (e.g., for optimal alignment, gaps can be introduced in one or two of the first and second amino acid sequences or nucleic acid sequences, or non-homologous sequences can be discarded for comparison). Amino acid residues or nucleotides at corresponding amino acid positions or nucleotide positions are then compared. When a position in the first sequence is occupied by the same amino acid residue or nucleotide at the corresponding position in the second sequence, the molecules are identical at this position. The percent identity between two sequences varies with the identical positions shared by the sequences, taking into account the number of gaps that need to be introduced and the length of each gap for optimal alignment of the two sequences.

A mathematical algorithm can be used to compare two sequences and calculate the percent identity between the sequences. For example, the percent identity between two amino acid sequences is determined with the Needlema and Wunsch algorithm ((1970) J. Mol. Biol., 48: 444-453; available at www.gcg.com) which has been integrated into the GAP program of the GCG software package, using the Blossum 62 matrix or PAM250 matrix and gap weight of 16, 14, 12, 10, 8, 6, or 4 and length weight of 1, 2, 3, 4, 5, or 6. For another example, the percent identity between two nucleotide sequences is determined with the GAP program of the GCG software package (available at www.gcg.com), using the NWSgapdna.CMP matrix and gap weight of 40, 50, 60, 70, or 80 and length weight of 1, 2, 3, 4, 5, or 6. A particularly preferred parameter set (and one that should be used unless otherwise stated) is a Blossum62 scoring matrix with a gap penalty of 12, a gap extension penalty of 4, and a frameshift gap penalty of 5. The percent identity between two amino acid sequences or nucleotide sequences can also be determined with a PAM120 weighted remainder table, a gap length penalty of 12, and a gap penalty of 4, using the E. Meyers and W. Miller algorithm ((1989) CABIOS, 4: 11-17) which has been incorporated into the ALIGN program (version 2.0). (The above is incorporated herein by reference).

Additionally or alternatively, the nucleic acid sequences and protein sequences described herein can be further used as "query sequences" for searches against public databases to, e.g., identify sequences of other family members or correlated sequences. For example, such searches can be performed using the NBLAST and XBLAST programs (version 2.0) of Altschul et al., (1990) J. Mol. Biol., 215: 403-10. BLAST nucleotide searches can be performed with the NBLAST program, with a score of 100 and a word length of 12, to obtain nucleotide sequences homologous to the nucleic acid molecule of the present disclosure. BLAST protein searches can be performed using the XBLAST program, with a score of 50 and a word length of 3, to obtain amino acid sequences homologous to the protein molecule of the present disclosure. To obtain gapped alignment results for the purpose of comparison, gapped BLAST can be used as described in Altschul et al. (1997) Nucleic Acids Res. 25: 3389-3402. When using the BLAST and gapped BLAST programs, the default parameters of the respective programs (e.g., XBLAST and NBLAST) can be used. See www.ncbi.nlm.nih.gov.

The term "nucleic acid" herein includes any compound and/or substance that comprises a polymer of nucleotides. Each nucleotide consists of a base, in particular a purine or pyrimidine base (i.e., cytosine (C), guanine (G), adenine (A), thymine (T), or uracil (U)), a sugar (i.e., deoxyribose or ribose), and a phosphate group. Generally, a nucleic acid molecule is described as a sequence of bases, whereby the bases represent the primary structure (linear structure) of the nucleic acid molecule. The sequence of bases is generally expressed as 5' to 3'. In this context, the term "nucleic acid molecule" encompasses deoxyribonucleic acid (DNA), including, e.g., complementary DNA (cDNA) and genomic DNA; ribonucleic acid (RNA), in particular messenger RNA (mRNA); the synthetic forms of DNA or RNA; and polymers comprising a mixture of two or more of these molecules. The nucleic acid molecule may be linear or cyclic. Furthermore, the term "nucleic acid molecule" includes both sense and antisense strands, as well as single- and double-stranded forms. Moreover, the nucleic acid molecules described herein may contain naturally occurring or non-naturally occurring nucleotides. Examples of non-naturally occurring nucleotides include modified nucleotide bases having derived sugar or phosphate backbone linkages or chemically modified residues. The nucleic acid molecule also encompasses DNA and RNA molecules suitable for use as a vector for direct expression of the antibodies of the present disclosure *in vitro* and/or *in vivo*, e.g., in a host or a patient. Such DNA (e.g., cDNA) or RNA (e.g., mRNA) vectors may be unmodified or modified. For example, mRNA can be chemically modified to enhance the stability of the RNA vector and/or the expression of the encoded molecule such that the mRNA can be injected into a subject to produce antibodies *in vivo* (see, e.g., Stadler et al., Nature Medicine 2017, published online, June 12, 2017, doi: 10.1038/nm.4356 or EP2101823B1), which is explicitly incorporated herein by reference.

The "Isolated" nucleic acid herein refers to a nucleic acid molecule that has been separated from components of its natural environment. The isolated nucleic acid includes a nucleic acid molecule contained in such a cell: the cell generally contains the nucleic acid molecule, but the nucleic acid molecule is present extrachromosomally or at a chromosomal location different from its natural chromosomal location.

The term "vector" herein refers to a nucleic acid molecule capable of amplifying another nucleic acid to which it has been linked. The term includes vectors that serve as self-replicating nucleic acid structures as well as vectors integrated into the genome of a host cell into which they have been introduced. Certain vectors are capable of directing the expression of nucleic acids to which they are operably linked. Such vectors are called "expression vectors" herein.

The term "host cell" herein refers to a cell into which an exogenous nucleic acid has been introduced, including the progeny of such a cell. Host cells include "transformants" and "transformed cells", which include primary transformed cells and progenies derived therefrom, regardless of the number of passages. Progenies may not be exactly the same as parent cells in terms of nucleic acid content, and may contain mutations. Mutant progenies having the same function or biological activity that are screened or selected from the primary transformed cells are included herein.

The term "pharmaceutical composition" herein refers to a formulation that exists in a form allowing the biological activity of the active ingredient contained therein to be effective and does not contain additional ingredients having unacceptable toxicity to a subject to which the pharmaceutical composition is administered.

The term "pharmaceutically acceptable carrier" herein includes any and all solvents, dispersion media, coatings, surfactants, antioxidants, preservatives (e.g., antibacterial agents and antifungal agents), isotonic agents, absorption retardants, salts, preservatives, drug stabilizers, binders, excipients, disintegrants, lubricants, sweeteners, flavorants, dyes, and the like, and combinations thereof, as known to those skilled in the art (see. E.g., Remington's Pharmaceutical Sciences, 18th Ed. MackPrinting Company, 1990, pp. 1289-1329). Except insofar as any conventional carrier is incompatible with the active ingredient, use in the therapeutic or pharmaceutical compositions is contemplated.

The term "treatment" used herein refers to surgical or therapeutic treatment for the purpose of preventing or slowing (reducing) the progression of an undesired physiological or pathological change, e.g., cancer or tumor. Beneficial or desired clinical results include, but are not limited to, alleviation of symptoms, decrease of severity of disease, stabilization (i.e., not worsening) of state of disease, delay or slowing of disease progression, amelioration or palliation of state of disease, and remission of state of disease (whether partial or total), whether detectable or undetectable. Subjects in need of treatment include those already with a disorder or disease, as well as those who are susceptible to a disorder or disease or those who intend to prevent a disorder or disease. When referring to terms such as slowing, alleviation, decrease, palliation, and remission, their meanings also include elimination, disappearance, nonoccurrence, etc.

The term "subject" herein refers to an organism that receives the treatment for a particular disease or disorder described herein. Illustratively, the "subject" includes mammals, such as humans, primates (e.g., monkey), or non-primate mammals, that receive the treatment for a disease or disorder.

The term "effective amount" herein refers to an amount of a therapeutic agent that is effective to prevent or alleviate symptoms of a disease or the progression of the disease when administered to a cell, tissue, or subject alone or in combination with another therapeutic agent. "Effective amount" also refers to an amount of a compound that is sufficient to alleviate symptoms, e.g., to treat, cure, prevent, or alleviate related medical disorders, or to increase the rates at which such disorders are treated, cured, prevented, or alleviated. When the active ingredient is administered alone to an individual, a therapeutically effective dose refers to the amount of the ingredient alone. When a combination is used, a therapeutically effective dose refers to the combined amounts of the active ingredients that produce the therapeutic effect, whether administered in combination, sequentially, or simultaneously.

The term "cancer" herein refers to or describes a physiological condition in mammals that is typically characterized by unregulated cell growth. Included in this definition are benign and malignant cancers. The term "tumor" or "neoplasm" herein refers to all neoplastic cell growth and proliferation, whether malignant or benign, and all pre-cancerous and cancerous cells and tissues. The terms "cancer" and "tumor" are not mutually exclusive when referred to herein.

### BRIEF DESCRIPTION OF THE DRAWINGS

FIGs. 1A-1B shows the assay on binding activity of control antibodies for human or monkey DLL3 protein by enzyme-linked immunosorbent assay (ELISA), where FIG. 1A is the binding activity of the control antibodies for human DLL3-his protein, and FIG. 1B is the binding activity of the control antibodies for monkey DLL3-his protein;
FIG. 2 shows the assay on the expression levels of human DLL3 protein in the CHO-K1 recombinant cell lines by flow cytometry fluorescence sorting (FACS);
FIG. 3 shows the assay on the expression levels of monkey DLL3 protein in the CHO-K1 recombinant cell lines by FACS;
FIG. 4 shows the assay on the expression levels of mouse DLL3 protein in the CHO-K1 recombinant cell lines by FACS;
FIGs. 5A-5E show the assay on the binding activity of chimeric antibodies for human DLL3 protein by ELISA;
FIGs. 6A-6E show the assay on the binding activity of chimeric antibodies for SHP-77 cells by FACS;
FIGs. 7A-7E show the assay on the binding activity of chimeric antibodies for CHO K1-cyno DLL3 cells, a cell line over-expressing monkey DLL3, by FACS;
FIGs. 8A-8G show the assay on the binding activity of humanized antibodies for human DLL3 protein by ELISA;
FIGs. 9A-9F show the assay on the binding activity of humanized antibodies for SHP-77 cells by FACS;
FIGs. 10A-10F show the assay on the binding activity of humanized antibodies for NCI-H82 cells by FACS;
FIGs. 11A-11F show the assay on the binding activity of humanized antibodies for the human DLL3 recombinant cell line by FACS;
FIGs. 12A-12F show the assay on the binding activity of humanized antibodies for the monkey DLL3 recombinant cell line by FACS;
FIGs. 13A-13F show the assay on the binding activity of humanized antibodies for the mouse DLL3 recombinant cell line by FACS.

### DETAILED DESCRIPTION

The present disclosure is further described below with reference to specific examples; the advantages and features of the present disclosure will become more apparent with the description. Experimental procedures without specified conditions in the examples are conducted according to conventional conditions or conditions recommended by the manufacturers. Reagents or instruments without specified manufacturers used herein are conventional products that are commercially available.

The examples herein are illustrative only, and do not limit the scope of the present disclosure in any way. It will be appreciated by those skilled in the art that various modifications or substitutions may be made to the technical solutions of the present disclosure in form and details without departing from the spirit and scope of the present disclosure, and that these modifications and substitutions shall fall within the protection scope of the present disclosure.

### Example 1: Preparation of Control Antibodies and Full-Length Antigens, Identification of Endogenous Cells, and Preparation of Cell Strains Over-expressing DLL3

### 1.1. Preparation of control antibodies

SC16.56, AMG757-4, 2G1, and BI3 are antibodies recognizing human DLL3, and they have strong binding activity for human DLL3 protein and also can specifically bind to recombinant or endogenous cell lines expressing DLL3.

The sequences of the heavy chain variable region and light chain variable region of SC16.56 were obtained according to patent US20200138969A1, the sequences of the heavy chain variable region and light chain variable region of AMG757-4 were obtained according to patent WO2017021349A1, the sequences of the heavy chain variable region and light chain variable region of 2G1 were obtained according to patent WO2020180591A1, and the sequences of the heavy chain variable region and light chain variable region of BI3 were obtained according to patent WO2019234220A1.

SC16.56 and BI3 expressed the complete IgG format. The VH and VL of each of AMG757-4 and 2G1 and human IgG1 Fc were linked in an order from N-terminus to C-terminus to form an scFv-human IgG1 Fc (scFv-hFc). The sequences of SC16.56 VH, SC16.56 VL, SC16.56 HC, SC16.56 LC, AMG757-4 scFv, AMG757-4 scFv-hFc, 2G1 scFv, 2G1 scFv-hFc, BI3 VH, BI3 VL, BI3 HC, and BI3 LC are shown in Table 1.

Nucleotide sequences were cloned into a pTT5 vector (purchased from Youbio) separately, and plasmids were prepared according to established standard molecular biology methods. For specific method, see Sambrook, J., Fritsch, E. F., and Maniatis, T. (1989). Molecular Cloning: A Laboratory Manual, Second Edition (Plainview, New York: Cold Spring Harbor Laboratory Press), which is incorporated herein by reference. The expression vector and the transfection reagent PEI (purchased from Polysciences, Cat. No. 24765-1) were added to OPTI-MEM (Gibco, Cat. No. 11058021). The mixture was well mixed and let stand for 15 min. Expi293F cells (Thermofisher, Cat. No. A14527) were added, and the system was incubated on a shaker at 37 °C and 5% CO₂ at 120 rpm. On day 2 of the transfection, OPM-293 ProFeed (Shanghai OPM Biosciences Co., Ltd., Cat. No. F081918-001) and 6 g/L glucose (Sigma, Cat. No. G7528) were added. On day 6 of the transfection, the cell supernatant was collected. The culture supernatant was loaded on a Protein A chromatography column (Protein A packing: AT Protein A Diamond, purchased from Bestchrom), washed with phosphate-buffered saline (PBS, pH 7.4), then washed with 20 mM PB, 1 M NaCl (pH 7.2), and finally eluted with a citrate buffer at pH 3.4. An Fc-tagged antibody eluted from the Protein A chromatography column was collected, neutralized with 1/10 column volume of 1 M Tris at pH 8.0, and dialyzed with PBS at 4 °C overnight. Protein aggregates were removed using molecular sieves (BXK16/26, purchased from Bestchrom). The concentration of the antibody was determined using Nanodrop, and the purity of the antibody was determined using HPLC-SEC. The endotoxin content of the antibody was detected by using an endotoxin assay kit, and finally, the control antibody was subjected to sterile filtration through a 0.22 µM filter, subpackaged, and stored at -80 °C.

**Table 1. Amino acid sequences of control antibodies**

| Sequence name | Sequence No. | Amino acid sequence |
|---|---|---|
| SC16.56 VH | SEQ ID NO.1 | QVQLVQSGAEVKKPGASVKVSCKASGYTFTNYGMNWVRQAPGQGLEW MGWINTYTGEPTYADDFKGRVTMTTDTSTSTAYMELRSLRSDDTAVYYC ARIGDSSPSDYWGQGTLVTVSS |
| SC16.56 VL | SEQ ID NO.2 | EIVMTQSPATLSVSPGERATLSCKASQSVSNDVVWYQQKPGQAPRLLIYY ASNRYTGIPARFSGSGSGTEFTLTISSLQSEDFAVYYCQQDYTSPWTFGQG TKLEIK |
| SC16.56 HC | SEQ ID NO.3 | QVQLVQSGAEVKKPGASVKVSCKASGYTFTNYGMNWVRQAPGQGLEW MGWINTYTGEPTYADDFKGRVTMTTDTSTSTAYMELRSLRSDDTAVYYC ARIGDSSPSDYWGQGTLVTVSSASTKGPSVFPLAPSSKSTSGGTAALGCLV KDYFPEPVTVSWNSGALTSGVHTFPAVLQSSGLYSLSSVVTVPSSSLGTQT YICNVNHKPSNTKVDKKVEPKSCDKTHTCPPCPAPELLGGPSVFLFPPKP KDTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQPR EPQVYTLPPSREEMTKNQVSLTCLVKGFYPSDIAVEWESNGQPENNYKTT PPVLDSDGSFFLYSKLTVDKSRWQQGNVFSCSVMHEALHNHYTQKSLSL SPGK |
| SC16.56 LC | SEQ ID NO.4 | EIVMTQSPATLSVSPGERATLSCKASQSVSNDVVWYQQKPGQAPRLLIYY ASNRYTGIPARFSGSGSGTEFTLTISSLQSEDFAVYYCQQDYTSPWTFGQG TKLEIKRTVAAPSVFIFPPSDEQLKSGTASVVCLLNNFYPREAKVQWKVD NALQSGNSQESVTEQDSKDSTYSLSSTLTLSKADYEKHKVYACEVTHQG LSSPVTKSFNRGEC |
| AMG757-4 scFv | SEQ ID NO.5 | QVQLQESGPGLVKPSETLSLTCTVSGGSISSYYWSWIRQPPGKCLEWIGY VYYSGTTNYNPSLKSRVTISVDTSKNQFSLKLSSVTAADTAVYYCASIAVT GFYFDYWGQGTLVTVSSGGGGSGGGGSGGGGSEIVLTQSPGTLSLSPGER VTLSCRASQRVNNNYLAWYQQRPGQAPRLLIYGASSRATGIPDRFSGSGS GTDFTLTISRLEPEDFAVYYCQQYDRSPLTFGCGTKLEIK |
| AMG757-4 scFv-hFc | SEQ ID NO.6 | QVQLQESGPGLVKPSETLSLTCTVSGGSISSYYWSWIRQPPGKCLEWIGY VYYSGTTNYNPSLKSRVTISVDTSKNQFSLKLSSVTAADTAVYYCASIAVT GFYFDYWGQGTLVTVSSGGGGSGGGGSGGGGSEIVLTQSPGTLSLSPGER VTLSCRASQRVNNNYLAWYQQRPGQAPRLLIYGASSRATGIPDRFSGSGS GTDFTLTISRLEPEDFAVYYCQQYDRSPLTFGCGTKLEIKEPKSSDKTHTCP PCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVS NKALPAPIEKTISKAKGQPREPQVYTLPPSREEMTKNQVSLTCLVKGFYPS DIAVEWESNGQPENNYKTTPPVLDSDGSFFLYSKLTVDKSRWQQGNVFS CSVMHEALHNHYTQKSLSLSPGK |
| 2G1 scFv | SEQ ID NO.7 | QLQLQESGPGLVKPSETLSLTCTVSGGSISSSSYYWGWIRQPPGKGLEWIG SIYYSGNIYHNPSLKSRVSISVDTSKNQFSLRLSSVTAADTAVYYCAREIIV GATHFDYWGQGTLVTVSSGGGGSGGGGSGGGGSGGGGSAIQMTQSPSS |
| | | LSASVGDRVTITCRASQGIRNDLGWYQQKPGKAPELLIYAASSLQSGVPS RFSGSGSGTDFTLTISSLQPEDFATYYCLQDYNYPLTFGPGTKVDIK |
| 2G1 scFv-hFc | SEQ ID NO.8 | QLQLQESGPGLVKPSETLSLTCTVSGGSISSSSYYWGWIRQPPGKGLEWIG SIYYSGNIYHNPSLKSRVSISVDTSKNQFSLRLSSVTAADTAVYYCAREIIV GATHFDYWGQGTLVTVSSGGGGSGGGGSGGGGSGGGGSAIQMTQSPSS LSASVGDRVTITCRASQGIRNDLGWYQQKPGKAPELLIYAASSLQSGVPS RFSGSGSGTDFTLTISSLQPEDFATYYCLQDYNYPLTFGPGTKVDIKEPKSA DKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKE YKCKVSNKALPAPIEKTISKAKGQPREPQVYTLPPSREEMTKNQVSLTCL VKGFYPSDIAVEWESNGQPENNYKTTPPVLDSDGSFFLYSKLTVDKSRWQ QGNVFSCSVMHEALHNHYTQKSLSLSPGK |
| BI3 VH | SEQ ID NO.9 | QVQLVQSGAEVKKPGASVKVSCKASGYTFTSYYVHWVRQAPGQGLEW MVIINPGGGTTSYAQKFLGRVTMTRDTSTNTVYMELKSLRSEDTAVYYC ARGEAVTGNYFYYGMDVWGQGTTVTVSS |
| BI3 VL | SEQ ID NO.10 | DIQMTQSPSAMSASVGDRVTITCRASQGISNYLVWFQQKPGKAPKRLIYA VSSLYSGVPSRFSGSGSGTEFTLTISSLQPEDFATYYCLQHDSYPYTFGQGT KLEIK |
| BI3 HC | SEQ ID NO.11 | QVQLVQSGAEVKKPGASVKVSCKASGYTFTSYYVHWVRQAPGQGLEW MVIINPGGGTTSYAQKFLGRVTMTRDTSTNTVYMELKSLRSEDTAVYYC ARGEAVTGNYFYYGMDVWGQGTTVTVSSASTKGPSVFPLAPSSKSTSGG TAALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTIS KAKGQPREPQVYTLPPSREEMTKNQVSLTCLVKGFYPSDIAVEWESNGQP ENNYKTTPPVLDSDGSFFLYSKLTVDKSRWQQGNVFSCSVMHEALHNHY TQKSLSLSPGK |
| BI3 LC | SEQ ID NO.12 | DIQMTQSPSAMSASVGDRVTITCRASQGISNYLVWFQQKPGKAPKRLIYA VSSLYSGVPSRFSGSGSGTEFTLTISSLQPEDFATYYCLQHDSYPYTFGQGT KLEIKRTVAAPSVFIFPPSDEQLKSGTASVVCLLNNFYPREAKVQWKVDN ALQSGNSQESVTEQDSKDSTYSLSSTLTLSKADYEKHKVYACEVTHQGLS SPVTKSFNRGEC |

### 1.2 Preparation of full-length human DLL3 protein antigen and cynomolgus monkey DLL3 protein antigen

The amino acid sequence of human DLL3 protein extracellular segment (ECD) (UniProt ID: Q9NYJ7; the specific sequence is set forth in SEQ ID NO: 13) and the amino acid sequence of cynomolgus monkey DLL3 protein extracellular segment (UniProt ID: A0A2K5WSR4; the specific sequence is set forth in SEQ ID NO: 14) were each coupled to a His tag and cloned into a pTT5 vector. The expression method was the same as that of the preparation of the control antibodies described in section 1.1 above. The cell components were removed by centrifugation to obtain a culture supernatant. The protein in the cell culture supernatant was purified using a Ni affinity chromatography column (purchased from GE Healthcare). The column was equilibrated with 3-5 column volumes of an equilibration buffer (PBS, pH 7.4), and the clarified culture supernatant was loaded on the Ni column with the flow rate controlled at 5 mL/min. After the sample loading, the Protein A column was washed with an equilibrium buffer, and the volume of the equilibrium buffer was 3-5 times that of the Ni affinity chromatography column. The impure proteins were eluted with PBS + 10 mM imidazole and PBS + 20 mM imidazole separately, and the elution was monitored with a nucleic acid protein detector (A280 UV absorption peak). The protein was then eluted using PBS + 250 mM imidazole, collected, and then dialyzed into PBS using a dialysis cassette (purchased from Thermo Scientific) at 4 °C. The protein was subjected to sterile filtration using a 0.22 µm filter (purchased from Millipore) and aseptically stored, thus obtaining the purified protein. Meanwhile, human DLL3-His protein (purchased from Acro, Cat. No. DL3-H52H4) and cynomolgus monkey DLL3-His protein (purchased from Acro, Cat. No. DL3-C52H3) were purchased commercially.

**Table 2. Sequences of human, monkey, and mouse DLL3 antigens and sequence of EpCAM transmembrane and intracellular regions**

| Antigen name | Sequence No. | Sequence information |
|---|---|---|
| Human DLL3 extracellular segment | SEQ ID NO.13 | AGVFELQIHSFGPGPGPGAPRSPCSARLPCRLFFRVCLKPGLSEEAAESP CALGAALSARGPVYTEQPGAPAPDLPLPDGLLQVPFRDAWPGTFSFIIE TWREELGDQIGGPAWSLLARVAGRRRLAAGGPWARDIQRAGAWELRF SYRARCEPPAVGTACTRLCRPRSAPSRCGPGLRPCAPLEDECEAPLVCR AGCSPEHGFCEQPGECRCLEGWTGPLCTVPVSTSSCLSPRGPSSATTGC LVPGPGPCDGNPCANGGSCSETPRSFECTCPRGFYGLRCEVSGVTCAD GPCFNGGLCVGGADPDSAYICHCPPGFQGSNCEKRVDRCSLQPCRNG GLCLDLGHALRCRCRAGFAGPRCEHDLDDCAGRACANGGTCVEGGG AHRCSCALGFGGRDCRERADPCAARPCAHGGRCYAHFSGLVCACAP GYMGARCEFPVHPDGASALPAAPPGLRPGDPQRYL |
| Cynomolgus monkey DLL3 extracellular segment | SEQ ID NO.14 | AGVFELQIHSFGPGPGPGAPRSPCSARGPCRLFFRVCLKPGLSEEAAES PCALGAALSARGPVYTEQPEAPAPDLPLPNGLLQVPFRDAWPGTFSLII ETWREELGDQIGGPAWSLLARVTRRRRLAAGGPWARDIQRAGAWELR FSYRARCELPAVGTACTRLCRPRSAPSRCGPGLRPCAPLEDECEAPPVC RAGCSLEHGFCEQPGECRCLEGWTGPLCMVPVSTSSCLGLRGPSSTTT GCLVPGPGPCDGNPCANGGSCSETPGSFECTCPRGFYGLRCEVSGVTC ADGPCFNGGLCVGGADPDSAYICHCPPGFQGSNCEKRVDRCSLQPCR NGGLCLDLGHALRCRCRAGFAGPRCEHDLDDCAGRACANGGTCVEG GGAHRCSCALGFGGRNCRERADPCAARPCAHGGRCYAHFSGLVCAC APGYMGARCEFPVHPDGVSALPAAPPGLRPGDPQR |
| EpCAM transmembrane and intracellular regions | SEQ ID NO.15 | AGVIAVIVVVVIAIVAGIVVLVISRKKRMAKYEKAEIKEMGEMHRELN A |
| Mouse DLL3 extracellular segment | SEQ ID NO.16 | HSFGPGPGLGTPRSPCNARGPCRLFFRVCLKPGVSQEATESLCALGAA LSTSVPVYTEHPGESAAALPLPDGLVRVPFRDAWPGTFSLVIETWREQL GEHAGGPAWNLLARVVGRRRLAAGGPWARDVQRTGTWELHFSYRA RCEPPAVGAACARLCRSRSAPSRCGPGLRPCTPFPDECEAPSVCRPGCS PEHGYCEEPDECRCLEGWTGPLCTVPVSTSSCLNSRVPGPASTGCLLPG PGPCDGNPCANGGSCSETSGSFECACPRGFYGLRCEVSGVTCADGPCF NGGLCVGGEDPDSAYVCHCPPGFQGSNCEKRVDRCSLQPCQNGGLCL DLGHALRCRCRAGFAGPRCEHDLDDCAGRACANGGTCVEGGGSRRC SCALGFGGRDCRERADPCASRPCAHGGRCYAHFSGLVCACAPGYMG VRCEFAVRPDGADAVPAAPRGLRQADPQRFL |

### 1.3 Assay on binding reactions of control antibodies with human DLL3-his protein and monkey DLL3-his protein

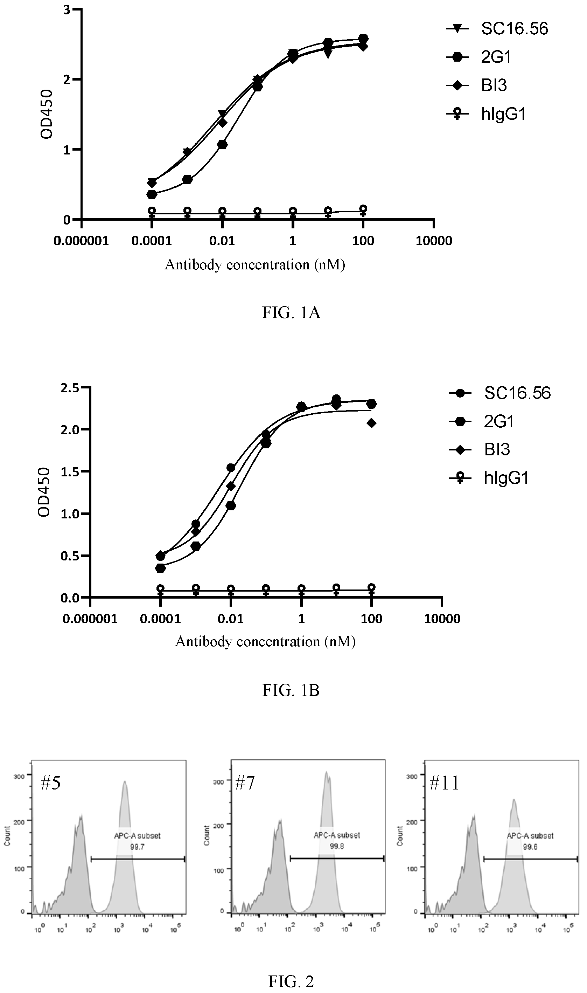

The antigen-antibody binding activity was detected by ELISA. The specific method is as follows: The antigen protein was diluted with PBS to a final concentration of 0.5 µg/mL, and the diluted solution was added to a 96-well ELISA plate at 50 µL/well. The plate was sealed with a plastic film and incubated at 4 °C overnight. The next day, the plate was washed twice with PBST, and a blocking buffer [PBS + 2% (w/w) BSA] was added for blocking at room temperature for 2 h. The blocking buffer was discarded, and then the control antibody (diluted in a 10-fold gradient from an initial concentration of 100 nM) or a negative control antibody was added at 50 µL/well. After incubation at room temperature for 1 h, the plate was washed 3 times with PBS. A horseradish peroxidase (HRP)-labeled secondary antibody (purchased from Merck, Cat. No. AP113P) was added, and the mixture was incubated at room temperature for 1 h. The plate was then washed 5 times with PBS. A TMB substrate was added at 50 µL/well, and the resulting mixture was incubated at room temperature for 10 min. Then, a stop solution (1.0 M HCl) was added at 50 µL/well. OD_{450 nm} values were read using an ELISA plate reader (Multimode Plate Reader, EnSight, purchased from Perkin Elmer). Specific results are shown in FIGs. 1A-1B; the control antibodies exhibit good binding activity for human DLL3 protein and monkey DLL3 protein. hIgG1 was the negative control.

### 1.4 Preparation of CHO-K1 recombinant cell strains expressing human DLL3 protein

The nucleotide sequences encoding the amino acid sequence of human DLL3 extracellular segment (UniProt ID: Q9NYJ7; the specific sequence is set forth in SEQ ID NO: 13) and the amino acid sequence of EpCAM transmembrane and intracellular regions (UniProt ID: P16422; the specific sequence is set forth in SEQ ID NO: 15, see Table 2 for specific sequence information) were cloned into a pLVX-IRES-Puro vector (purchased from Youbio, Cat. No. VT1464) and subjected to lentiviral packaging, and then the virus was used to infect the CHO-K1 cells. After the CHO-K1 cells were infected with the virus for 72 h, the cells were assayed by flow cytometry using the known DLL3 antibody 2G1. When it was confirmed that the transfected cells began to express human DLL3 protein, 10 µg/mL Puromycin (purchased from Gibco, Cat. No. A1113802) was added for screening. After the cells recovered, subcloning was performed in a 96-well plate by the limiting dilution method, and the cells were cultured at 37 °C and 5% (v/v) CO₂. After about 2 weeks, some of the monoclonal wells were selected for expansion in a 6-well plate. The expanded clones were screened again by flow cytometry using the 2G1 antibody. Monoclonal cell lines with better growth and higher fluorescence intensity were selected and further expanded, and the cells were assayed again by flow cytometry and then cryopreserved in liquid nitrogen, thus obtaining the stably transfected cell strains expressing human DLL3. The specific results are shown in Table 3 and FIG. 2, in which the IgG subtype control was a human IgG1 control. Table 3 shows that a series of CHO-K1 monoclonal cell lines with positive expression of human DLL3 had been obtained. In FIG. 2, the abscissa represents the fluorescence intensity of the cells, and the ordinate represents the number of the cells. The results show that #5, #7, and #11 were all cell strains with high expression of human DLL3.

**Table 3. FACS assay results for CHO-K1 recombinant cell lines expressing human DLL3 protein**

| No. | Clone No. of stably transfected cell line | Mean fluorescence intensity of cells | |
|---|---|---|---|
| | | IgG subtype control | DLL3 antibody |
| 1 | CHO-K1 hDLL3 #5 | 39.4 | 2003 |
| 2 | CHO-K1 hDLL3 #7 | 39.4 | 2379 |
| 3 | CHO-K1 hDLL3 #11 | 39.4 | 1634 |

### 1.5 Preparation of CHO-K1 recombinant cell strains expressing monkey DLL3 protein

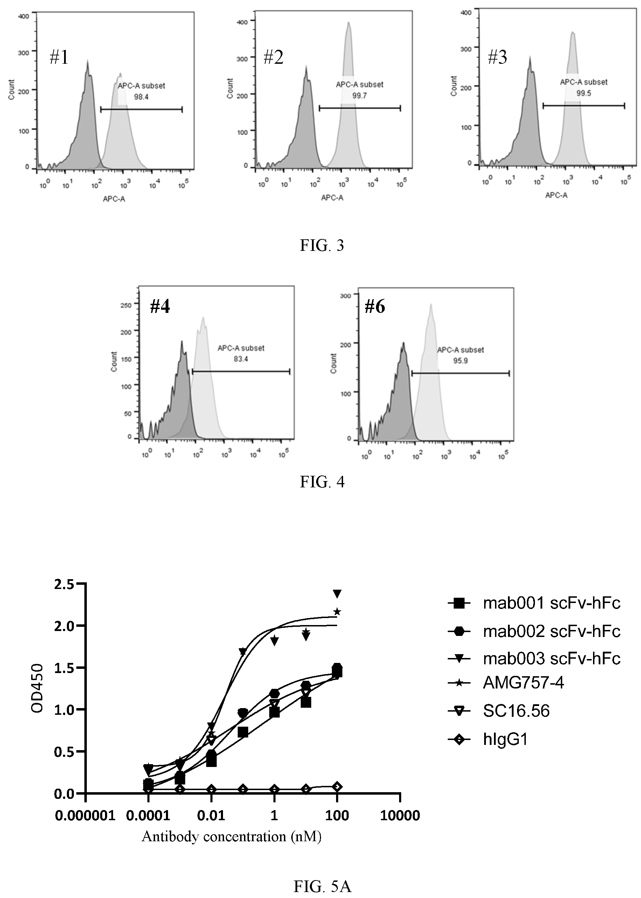

The nucleotide sequences encoding the amino acid sequence of monkey DLL3 extracellular segment (UniProt ID: A0A2K5WSR4; the specific sequence is set forth in SEQ ID NO: 14) and the amino acid sequences of EpCAM transmembrane and intracellular regions (see Table 2) were cloned into a pLVX-IRES-Puro vector, and stably transfected cell strains expressing monkey DLL3 were obtained by using the method for constructing recombinant cells in section 1.4 above. The specific results are shown in Table 4 and FIG. 3, in which the IgG subtype control was a human IgG1 control. Table 4 shows that a series of CHO-K1 monoclonal cell lines, #1, #2, and #3, with positive expression of monkey DLL3 had been obtained. In FIG. 3, the abscissa represents the fluorescence intensity of the cells, and the ordinate represents the number of the cells. The results show that #1, #2, and #3 were all cell strains with high expression of monkey DLL3.

**Table 4. FACS assay results for CHO-K1 recombinant cell lines expressing monkey DLL3 protein**

| No. | Clone No. of stably transfected cell line | Mean fluorescence intensity of cells | |
|---|---|---|---|
| | | IgG subtype control | DLL3 antibody |
| 1 | CHO K1-cyno DLL3 #1 | 31.5 | 734 |
| 2 | CHO K1-cyno DLL3 #2 | 31.5 | 1563 |
| 3 | CHO K1-cyno DLL3 #3 | 31.5 | 1581 |

### 1.6 Preparation of CHO-K1 recombinant cell strains expressing mouse DLL3 protein

The nucleotide sequences encoding the amino acid sequence of mouse DLL3 extracellular segment (UniProt ID: O88516; the specific sequence is set forth in SEQ ID NO: 16) and the amino acid sequences of EpCAM transmembrane and intracellular regions (see Table 2) were cloned into a pLVX-IRES-Puro vector, and stably transfected cell strains #4 and #6 expressing mouse DLL3 were obtained by using the method for constructing recombinant cells in section 1.4. The specific results are shown in Table 5 and FIG. 4, in which the IgG subtype control was a human IgG1 control. Table 5 shows that a series of CHO-K1 monoclonal cell lines with positive expression of mouse DLL3 had been obtained. In FIG. 4, the abscissa represents the fluorescence intensity of the cells, and the ordinate represents the number of the cells. The results show that both #4 and #6 were cell strains with high expression of mouse DLL3.

**Table 5. FACS assay results for CHO-K1 recombinant cell lines expressing mouse DLL3 protein**

| No. | Clone No. of stably transfected cell line | Mean fluorescence intensity of cells | |
|---|---|---|---|
| | | IgG subtype control | DLL3 antibody |
| 1 | CHO K1-mDLL3 #4 | 23.7 | 249 |
| 2 | CHO K1-mDLL3 #6 | 25.3 | 349 |

### Example 2: Preparation of Hybridoma Antibody Against DLL3

### 2.1 Mouse immunization and serum titer assay

In the animal immunization experiment, there were nine experimental groups. The experimental animals were 6-8 week-old female Balb/c, SJL, MRL/lpr, and C57 BL/6J mice (purchased from Shanghai SLAC Laboratory Animal Co., Ltd.) and were housed in an SPF environment. Blood was collected from orbits of the mice before immunization to obtain the negative serum.

For the first to third immunization groups, at the primary immunization, human DLL3 antigen was emulsified with Alum (purchased from Thermo, Cat. No. 77161) and CpG (synthesized by Sangon Biotech, Cat. No. ODN1826) and injected intraperitoneally (0.1 mL), and the human DLL3 antigen was also emulsified with TiterMax (purchased from Sigma, Cat. No. T2684) and CpG and injected subcutaneously and intraplantarly at multiple sites, that is, 50 µg of immunogen was injected into each mouse. A booster immunization was performed every week thereafter, and during each booster immunization, the antigen was emulsified with Alum or TiterMax and injected subcutaneously at multiple sites, that is, 25 µg of immunogen was injected into each mouse; a total of six booster immunizations were performed. The third group was further subjected to three booster immunizations with CHO cells expressing DLL3 protein, and the immunization method is as follows: the cells were emulsified with TiterMax and physiological saline and then injected intraperitoneally (0.1 mL), and then a suspension of cells in CpG was injected intraperitoneally, and a total of 1E+7 cells were injected into each mouse.

For the fourth and fifth immunization groups, at the primary immunization, the cells were emulsified with TiterMax and physiological saline and then injected intraperitoneally (0.1 mL), and then a suspension of cells in CpG was injected intraperitoneally, and a total of 1E+7 cells were injected into each mouse. At the second immunization, the antigen was emulsified with Alum and CpG and injected subcutaneously at multiple sites, and a total of 25 µg of immunogen was injected. A booster immunization was performed every week thereafter, and the booster immunizations were performed using the cells and protein alternately.

For the sixth to ninth immunization groups, at the primary immunization, the cells were emulsified with TiterMax and physiological saline and then injected intraperitoneally (0.1 mL), and then a suspension of cells in CpG was injected intraperitoneally, and a total of 1E+7 cells were injected into each mouse. At the second immunization, the antigen was emulsified with Alum and CpG and injected subcutaneously at multiple sites, and a total of 25 µg of immunogen was injected. A booster immunization was performed every week thereafter, and the booster immunizations were performed using the cells and protein alternately.

For the immunization groups above, blood was collected from orbits of the mice after the second, sixth, and seventh booster immunizations, and serum batches were named TB1, TB2 and TB3, respectively. The binding titers of the antibody in the mouse serum for the human DLL3-His protein were assayed by ELISA. The assay results show that the serum of the immunized mice exhibited different degrees of binding to the immunogens and showed antigen-antibody reactions.

Mice with high antibody titer in serum were selected for splenocyte fusion after the booster immunizations. A booster immunization was performed 3 days prior to the splenocyte fusion, and an antigen solution prepared with physiological saline was injected subcutaneously, intraplantarly, and intraperitoneally at 50 µg/mouse.

### 2.2 Splenocyte fusion and hybridoma screening

ACK Lysing Buffer (purchased from Gibco, Cat. No. A1049201) was added to lyse red blood cells mixed in splenocytes and thereby to obtain a splenocyte suspension. The cells were washed 3 times by centrifugation at 1000 rpm with a DMEM (purchased from Gibco, Cat. No. 12800017) basal medium and then mixed with mouse myeloma cells SP2/0 (purchased from ATCC) at a ratio of 2:1 in number of viable cells, and the resulting mixture was subjected to cell fusion by BTX ECM2001+ efficient electrofusion (see METHODS IN ENZYMOLOGY, VOL. 220). The fused cells were diluted with a DMEM medium containing 20% fetal bovine serum (purchased from ExCell Bio, Cat. No. FND500) and 1× HAT (purchased from Sigma, Cat. No. H0262-10VL), where the percentage was a percentage by mass. Then the cells were added to a 96-well cell culture plate at 2 × 10⁴ cells/200 µL/well, and the plate was put into an incubator at 37 °C and 5% CO₂, where the percentage was a percentage by volume. After 14 days, cell fusion plate supernatants were screened by ELISA, and human DLL3 protein was allowed to bind to positive clones, which were then seeded in a 24-well plate and expanded in DMEM containing 10% HT (purchased from Sigma, Cat. No. H0137-10VL) fetal bovine serum at 37 °C and 5% CO₂. After culturing for 3 days, the culture solution resulting from expansion in the 24-well plate was centrifuged, and the supernatant was collected and assayed for binding activity by ELISA using the monkey DLL3 protein and for binding activity by FACS using CHO-K1 hDLL3. Based on the screening results for the 24-well plate, hybridoma cells from the hybridoma cell culture supernatants that were positive for the binding were selected as eligible positive clones, and the hybridoma cells from the positive wells were subcloned in a 6-well plate using Medium D (purchased from STEMCELL, Cat. No. 03810). After 7 days of subcloning, monoclones were picked and cultured in a DMEM medium containing 10% FBS and 1× HT at 37 °C and 5% CO₂ for 2 days. Primary screening was performed by ELISA, and single positive monoclones were selected and seeded in a 24-well plate for further culturing. After 3 days, the binding activity was assayed by ELISA using human DLL3 protein and monkey DLL3 protein, the binding activity was assayed by FACS using CHO-K1 hDLL3 cells, SHP-77 cells, or NCI-H82 cells, and the binding specificity was confirmed by FACS using negative cells CHO-K1 or FlpinCHO.

Based on assay results for the sample from the 24-well plate, the optimal clone was selected and expanded in a DMEM medium containing 10% FBS at 37 °C and 5% CO₂, and the expanded clone was cryopreserved in liquid nitrogen to obtain the hybridoma cell provided in the present invention.

### Example 3: Amino Acid Sequencing for Light and Heavy Chain Variable Regions of Hybridoma Positive Clones

Hybridoma cells in a logarithmic growth phase were collected, then fully lysed with Trizol (Invitrogen, with Cat. No. 15596-018), and stored at -80 °C. Amino acid sequencing for light and heavy chain variable regions of hybridoma positive clones was completed. The variable region sequences are shown in Table 6. Sequencing results were analyzed by using MOE software, then an evolutionary tree was constructed based on amino acid sequences of proteins encoding variable regions, and similar sequences were excluded, thus obtaining candidate clones. The light chain variable region sequence and the heavy chain variable region sequence of each candidate clone were cloned in an order of VL-linker-VH into an expression vector pTT5 containing a signal peptide and the Fc fragment of a human antibody IgG1 (sequence of Fc: 
EPKSSDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTIS
KAKGQPREPQVYTLPPSREEMTKNQVSLTCLVKGFYPSDIAVEWESNGQPENNYKTTPPVL
DSDGSFFLYSKLTVDKSRWQQGNVFSCSVMHEALHNHYTQKSLSLSPGK, SEQ ID NO: 17)
 to obtain expression vectors for human-murine chimeric antibodies, and the antibodies were prepared according to the method in section 1.1 of Example 1. 16 scFv-hFc chimeric antibodies were obtained, and their VH and VL sequences are shown in Table 6 and their CDR sequences are shown in Table 7.

**Table 6. Amino acid sequences of antibody variable regions**

| Heavy and light chains | | Sequence No. | Amino acid sequence |
|---|---|---|---|
| mab001 | VH | SEQ ID NO.18 | QIQLVQSGPELKKPGETVKISCKASGYTFTTYGMSWVKQAPGKG LKWMGWINTYSGVPTYADDFKGRFAFSLETSASTAYLQINNLKN EDTATYFCARIGDYDYGEYWGQGTLVTVSA |
| | VL | SEQ ID NO.19 | DVVMTQTPLTLAVTTGQPASISCKSSQSLLDSDGKTYLNWLLQR PGQSPKRLLYLVSQLDSGVPDRFSGSGSGTDFTLKISRVEAEDLG VYYCWQGTHFPYTFGGGTKLEIK |
| mab002 | VH | SEQ ID NO.20 | QIQLVQSGPELKKPGETVKISCKASGYTFTNYGMNWLKQAPGK GLKWMGWINTYTGESTYADDFKGRFAFSLETSASTVYLQINNLK NEDMATYFCARMGDGYYDFTMDYWGQGTSVTVSS |
| | VL | SEQ ID NO.21 | QIVLTQSPAIMSASPGEKVTMTCSARSSVSYMQWYQQKSGTSPK RWIYDTSKLASGVPARFSGSGSGTSYSLTISTMEAEDAATYYCQQ WSSDPPTFGGGTKLEIK |
| mab003 | VH | SEQ ID NO.22 | QVQLQQSGTELMKPGASVKLSCKATGYTFTGNWIEWVKQRPGH GLEWIGEILPGTGSTQYNEKFKGKATFTADPSSNTAYMQLSSLTT EDSAIYYCARWGSTVVAYDYWGQGTTLTVSS |
| | VL | SEQ ID NO.23 | DVQITQSPSYLAASPGETITINCRASKSISKYLAWYQEKPGKTNK LLIYSGSTLHSGIPSRFSGSGSGTDFTLTISSLEPEDFAIYYCQQHN EYPLTFGGGTKLELK |
| mab004 | VH | SEQ ID NO.24 | QVQLQQPGAELVKPGASVKLSCKASGYTFTSYWINWVKQRPGQ GLEWIGNIYPGSSSPNYNEKFKSKATLTVDTSSSTAYMQLSSLTSD DSAVYYCARSLLYAMDYWGQGTSVTVSS |
| | VL | SEQ ID NO.25 | DIVLTQSPASLAVSLGQRATISCRASESVDYDGDSYMNWYQQKP GQPPKLLIYVASNLESGIPARFSGSGSGTDFILNIHPVEEEDAATYY CLQSNEAPYTFGSGTKLEIK |
| mab005 | VH | SEQ ID NO.26 | QVQLQQSGAELMKPGASVTLSCKATGYTLPGYWIEWVKQRPGH GLEWIGEILPGSSSANYNEKFKGKATFTADTSSNTAYMQLSSLTT EDSAIYYCASTYGTSLDYWGQGTTLTVSS |
| | VL | SEQ ID NO.27 | DIVLTQSPATLSVTPGDSVSLSCRASQSVSNDLHWYQQKSHESPR LLIKYASQSISGIPSRFSGSGSGTDFTLTINSVETEDFGMYFCQQSN NWPYTFGGGTKLEMK |
| mab006 | VH | SEQ ID NO.28 | QVQLQQPGAELVRPGTSVKLSCKASGYTFTSYWMHWVRQRPG QGLEWIGVIDPSDSLANYNQEFKGKATLTVDTSSSTAYMQLSSLT SEDSAVYYCARDSTVVAPMDYWGQGTSVTVSS |
| | VL | SEQ ID NO.29 | DVQITQSPSYLAASPGETITINCRATKSISKYLAWYQEKPGKTNK LLIFSGSTLQSGIPSRFRGSGSGTDFTLTISSLEPEDFAMYYCQQHH EYPYTFGGGTKLEIK |
| mab007 | VH | SEQ ID NO.30 | QVQLQQPGAELVRPGTSVKLSCKASGYTFTSYWMHWVKQRPG QGLEWIGVIDPSDSLANYNQEFKGKATLTVDTSSSTAYMQLSSLT SEDSAVYYCARDSTVVAPMDYWGQGTSVTVSS |
| | VL | SEQ ID NO.31 | DVQITQSPSYLAASPGETITINCRASTSISKYLAWYQERPGKTNKL LIYSGSTLQSGIPSRFRGSGSGTDFTLTISSLEPEDFAMYYCQQHH VYPYTFGGGTKLEIK |
| mab008 | VH | SEQ ID NO.32 | EVQLQQSGPVLVKPGASVKMSCKASGYTFTDYYMNWVKRSHG KSLEWIGIINPYNGGTSYNQKFRGKATLTVDKSSNTAYMELNSLT SEDSAVYYCSRYDYDYDVEGAMDYWGQGTSVTVSS |
| | VL | SEQ ID NO.33 | DIVMTQSQKFMSTSVGDRVSITCKASQNVGTAVAWYQQKPGQSP KLLIFSASIRYTGVPDRFTGSGSGTDFTLTISNMQSEDLADYFCQQ YSSSPYTFGGGTKLEIK |
| mab009 | VH | SEQ ID NO.34 | EVQLQQSGTVLARPGASVKMSCKTSGYTFTSYWMHWVKQSPG QGLEWIGTIYPGNSDTRYNQKFKDKAYVTAVTSASTAYMELSSLT NEESAVYYCTRSYDYGDLDYWGQGTTLTVSS |
| | VL | SEQ ID NO.35 | DIVMTQSPATLSVTPGDRVSLSCRASQTISDYLHWYQQRSHESPR LLIKYASQSISGIPSRFSGSGSGSDFTLSINSVEPEDVGVYYCQNG HSFPLTFGAGTKLELK |
| mab010 | VH | SEQ ID NO.36 | QVQLQQSGAELARPGASMKLSCKASGYTFTSYGISWVKQITGQ GLEWIGEIFPRSGNTYDNENFRGKATLTADKSSSTAYLEVRSLTSE DSAVYFCARTTVFAPYYFDYWGQGTTLTVSS |
| | VL | SEQ ID NO.37 | DIQMTQSPASLSVSVGETVTITCRASENINSNLAWYQQKQGKSPQ LLVYAATNLADGVPSRFSGSGSGTQFSLKISGLQSEDFGSYYCQH FWGLPWTFGGGTKLEIR |
| mab011 | VH | SEQ ID NO.38 | EVQLQQSGPELVKPGASVKISCKASGYTFTDYYMNWVKQSHGK SLEWIGDISPNYGGTNYNQKFKGKATLTVDKSSSTACMELRSLTS EDSAVYYCTRSFYYYGTGYDWYFDVWGTGTTVTVSS |
| | VL | SEQ ID NO.39 | DIVLTQSPATLSVTPGDSVSLSCRASQSISNNLHWYQQKSHESPRL LIKYASQSISGIPSRFSGSGSGTDFTLSINSVETEDFGMYFCQQSNS WPWTFGGGTKLEIK |
| mab012 | VH | SEQ ID NO.40 | QVQLQQSGPELVKPGASVKISCKASGYTFTDYYIHWVKQRPGQ GLEWIGWTLPGSGSPYYNEKFKGKATLTVDKSSSTAYMLLSSLTS EDSAVYFCARWHYYYGSSYHFDYWGQGTTLTVSS |
| | VL | SEQ ID NO.41 | DVLMTQTPLSLPVSLGDQASISCRSSQSIVHSDGNTYLEWYLQKP GQSPKLLIYKVSNRFSGVPDRFSGSGSGTDFTLKISRVEAEDLGF YYCFQGSHVPFTFGSGTKLEIK |
| mab013 | VH | SEQ ID NO.42 | QVQLQQSGAELVKPGASVRISCKASGYAFSNYWINWVKQRPGK GLEWIGQINPGDGDTIYNGKFKGKATLTADKSSSTAYMQLSSLTS EDSAVYFCEREGRNYGDFAYWGQGTTLTVSS |
| | VL | SEQ ID NO.43 | DIVLTQSPASLAVSLGQRATISCRASKSVSTSGFSYMHWYQQKPG QPPKLLIYLASNLESGVPARFSGSGSGTDFTLNIHPVEEEDAATYY CQHSRELPFTFGSGTKLEIK |
| mab014 | VH | SEQ ID NO.44 | EVQLHQSGPELVKPGTSVKISCKASGYTFTDYYMNWVKQSHGK SLEWIGDINPNHGDTGYNQKFTGKATLTVDKSSSTAYMELRSLTS EDSAVYYCARNFFYYGTTYGWYFDVWGTGTTVTVSS |
| | VL | SEQ ID NO.45 | DIQMTQSSSSFSVSLGDRVTITCKASEDIYNRLAWYQQKPGNTPR LLISGATSLETGVPSRFSGSGSGKDFTLSITSLQTEDVATYYCQQY WSLPWTFGGGTKLEIK |
| mab015 | VH | SEQ ID NO.46 | EVKLEESGGGLVQPGGSMKLSCVASGITFSNFWMNWVRQSPEK GLEWVAQIRLKSDDYATHYAESVKGRFTISRDDSKGSVYLQMNN LRTEDTGIHYCTTGYYFDYWGQGTTLTVSS |
| | VL | SEQ ID NO.47 | NIVMTQSPKSMSMSVGERVTLSCKASENVGTYVSWFQQKPEQS PKLLIYGASNRYTGVPDRFTGSGSATDFTLTISSVQAEDLADYHC GQFYSFPCTFGGGTKLEIK |
| mab016 | VH | SEQ ID NO.48 | EVNLEESGGGLVQPGGSMKLSCVASGFAFSNFWMQWVRQSPEK GLEWVAQIRLKSDNYAAHYAESVKGRFTISRDDSKSSVYLQLNN LRAEDTGTYYCTQGNWVGFAYWGQGTLVTVSA |
| | VL | SEQ ID NO.49 | DIVMTQSQKFMSTTIGDRVSITCKASQNVGTTVAWYQQKPGQSP NLLLYSTSNRFTGVPDRFTGSGSGTDFTLTITNMQSEDLADYFCQ QYNNYPLTFGGGTKLEIK |

**Table 7. Heavy and light chain CDR sequences of DLL3 chimeric antibodies**

| **Kabat analysis** | | | | | | |
|---|---|---|---|---|---|---|
| **Fv** | **Sequence No.** | **CDR1** | **Sequence No.** | **CDR2** | **Sequence No.** | **CDR3** |
| mab001-V H | SEQ ID NO.50 | TYGMS | SEQ ID NO.51 | WINTYSGVP TYADDFKG | SEQ ID NO.52 | IGDYDYGEY |
| mab001-VL | SEQ ID NO.53 | KSSQSLLDSDGK TYLN | SEQ ID NO.54 | LVSQLDS | SEQ ID NO.55 | WQGTHFPYT |
| mab002-V H | SEQ ID NO.56 | NYGMN | SEQ ID NO.57 | WINTYTGES TYADDFKG | SEQ ID NO.58 | MGDGYYDFT MDY |
| mab002-VL | SEQ ID NO.59 | SARSSVSYMQ | SEQ ID NO.60 | DTSKLAS | SEQ ID NO.61 | QQWSSDPPT |
| mab003-V H | SEQ ID NO.62 | GNWIE | SEQ ID NO.63 | EILPGTGSTQ YNEKFKG | SEQ ID NO.64 | WGSTVVAYD Y |
| mab003-VL | SEQ ID NO.65 | RASKSISKYLA | SEQ ID NO.66 | SGSTLHS | SEQ ID NO.67 | QQHNEYPLT |
| mab004-V H | SEQ ID NO.68 | SYWIN | SEQ ID NO.69 | NIYPGSSSPN YNEKFKS | SEQ ID NO.70 | SLLYAMDY |
| mab004-VL | SEQ ID NO.71 | RASESVDYDGD SYMN | SEQ ID NO.72 | VASNLES | SEQ ID NO.73 | LQSNEAPYT |
| mab005-V H | SEQ ID NO.74 | GYWIE | SEQ ID NO.75 | EILPGSSSAN YNEKFKG | SEQ ID NO.76 | TYGTSLDY |
| mab005-VL | SEQ ID NO.77 | RASQSVSNDLH | SEQ ID NO.78 | YASQSIS | SEQ ID NO.79 | QQSNNWPYT |
| mab006-V H | SEQ ID NO.80 | SYWMH | SEQ ID NO.81 | VIDPSDSLAN YNQEFKG | SEQ ID NO.82 | DSTVVAPMD Y |
| mab006-VL | SEQ ID NO.83 | RATKSISKYLA | SEQ ID NO.84 | SGSTLQS | SEQ ID NO.85 | QQHHEYPYT |
| mab007-V H | SEQ ID NO.86 | SYWMH | SEQ ID NO.87 | VIDPSDSLAN YNQEFKG | SEQ ID NO.88 | DSTVVAPMD Y |
| mab007-VL | SEQ ID NO.89 | RASTSISKYLA | SEQ ID NO.90 | SGSTLQS | SEQ ID NO.91 | QQHHVYPYT |
| mab008-V H | SEQ ID NO.92 | DYYMN | SEQ ID NO.93 | IINPYNGGTS YNQKFRG | SEQ ID NO.94 | YDYDYDVEG AMDY |
| mab008-VL | SEQ ID NO.95 | KASQNVGTAVA | SEQ ID NO.96 | SASIRYT | SEQ ID NO.97 | QQYSSSPYT |
| mab009-V H | SEQ ID NO.98 | SYWMH | SEQ ID NO.99 | TIYPGNSDTR YNQKFKD | SEQ ID NO.100 | SYDYGDLDY |
| mab009-VL | SEQ ID NO.101 | RASQTISDYLH | SEQ ID NO.102 | YASQSIS | SEQ ID NO.103 | QNGHSFPLT |
| mab010-V H | SEQ ID NO. 104 | SYGIS | SEQ ID NO. 105 | EIFPRSGNTY DNENFRG | SEQ ID NO.106 | TTVFAPYYF DY |
| mab010-VL | SEQ ID NO. 107 | RASENINSNLA | SEQ ID NO.108 | AATNLAD | SEQ ID NO.109 | QHFWGLPWT |
| mab011-VH | SEQ ID NO.110 | DYYMN | SEQ ID NO.111 | DISPNYGGTN YNQKFKG | SEQ ID NO.112 | SFYYYGTGY DWYFDV |
| mab011-VL | SEQ ID NO.113 | RASQSISNNLH | SEQ ID NO.114 | YASQSIS | SEQ ID NO.115 | QQSNSWPWT |
| mab012-V H | SEQ ID NO.116 | DYYIH | SEQ ID NO.117 | WTLPGSGSP YYNEKFKG | SEQ ID NO.118 | WHYYYGSSY HFDY |
| mab012-VL | SEQ ID NO.119 | RSSQSIVHSDGN TYLE | SEQ ID NO.120 | KVSNRFS | SEQ ID NO.121 | FQGSHVPFT |
| mab013-V H | SEQ ID NO.122 | NYWIN | SEQ ID NO.123 | QINPGDGDTI YNGKFKG | SEQ ID NO.124 | EGRNYGDFA Y |
| mab013-VL | SEQ ID NO.125 | RASKSVSTSGFS YMH | SEQ ID NO.126 | LASNLES | SEQ ID NO.127 | QHSRELPFT |
| mab014-V H | SEQ ID NO.128 | DYYMN | SEQ ID NO.129 | DINPNHGDT GYNQKFTG | SEQ ID NO.130 | NFFYYGTTY GWYFDV |
| mab014-VL | SEQ ID NO.131 | KASEDIYNRLA | SEQ ID NO. 132 | GATSLET | SEQ ID NO.133 | QQYWSLPWT |
| mab015-V H | SEQ ID NO.134 | NFWMN | SEQ ID NO.135 | QIRLKSDDYA THYAESVKG | SEQ ID NO.136 | GYYFDY |
| mab015-VL | SEQ ID NO.137 | KASENVGTYVS | SEQ ID NO.138 | GASNRYT | SEQ ID NO.139 | GQFYSFPCT |
| mab016-V H | SEQ ID NO.140 | NFWMQ | SEQ ID NO.141 | QIRLKSDNYA AHYAESVKG | SEQ ID NO.142 | GNWVGFAY |
| mab016-VL | SEQ ID NO.143 | KASQNVGTTVA | SEQ ID NO.144 | STSNRFT | SEQ ID NO.145 | QQYNNYPLT |

| **IMGT** | | | | | | |
|---|---|---|---|---|---|---|
| **Fv** | **Sequence No.** | **CDR1** | **Sequence No.** | **CDR2** | **Sequence No.** | **CDR3** |
| mab001-V H | SEQ ID NO.146 | GYTFTTYG | SEQ ID NO.147 | INTYSGVP | SEQ ID NO.148 | ARIGDYDYG EY |
| mab001-VL | SEQ ID NO.149 | QSLLDSDGKTY | SEQ ID NO.150 | LV | SEQ ID NO.151 | WQGTHFPYT |
| mab002-V H | SEQ ID NO.152 | GYTFTNYG | SEQ ID NO.153 | INTYTGES | SEQ ID NO.154 | ARMGDGYY DFTMDY |
| mab002-VL | SEQ ID NO.155 | SSVSY | SEQ ID NO.156 | DT | SEQ ID NO.157 | QQWSSDPPT |
| mab003-V H | SEQ ID NO.158 | GYTFTGNW | SEQ ID NO.159 | ILPGTGST | SEQ ID NO.160 | ARWGSTVVA YDY |
| mab003-VL | SEQ ID NO.161 | KSISKY | SEQ ID NO.162 | SG | SEQ ID NO.163 | QQHNEYPLT |
| mab004-V H | SEQ ID NO.164 | GYTFTSYW | SEQ ID NO.165 | IYPGSSSP | SEQ ID NO.166 | ARSLLYAMD Y |
| mab004-VL | SEQ ID NO.167 | ESVDYDGDSY | SEQ ID NO.168 | VA | SEQ ID NO.169 | LQSNEAPYT |
| mab005-V H | SEQ ID NO.170 | GYTLPGYW | SEQ ID NO.171 | ILPGSSSA | SEQ ID NO.172 | ASTYGTSLD Y |
| mab005-VL | SEQ ID NO.173 | QSVSND | SEQ ID NO.174 | YA | SEQ ID NO.175 | QQSNNWPYT |
| mab006-V H | SEQ ID NO.176 | GYTFTSYW | SEQ ID NO.177 | IDPSDSLA | SEQ ID NO.178 | ARDSTVVAP MDY |
| mab006-VL | SEQ ID NO.179 | KSISKY | SEQ ID NO.180 | SG | SEQ ID NO.181 | QQHHEYPYT |
| mab007-V H | SEQ ID NO.182 | GYTFTSYW | SEQ ID NO.183 | IDPSDSLA | SEQ ID NO.184 | ARDSTVVAP MDY |
| mab007-VL | SEQ ID NO.185 | TSISKY | SEQ ID NO.186 | SG | SEQ ID NO.187 | QQHHVYPYT |
| mab008-V H | SEQ ID NO.188 | GYTFTDYY | SEQ ID NO.189 | INPYNGGT | SEQ ID NO.190 | SRYDYDYDV EGAMDY |
| mab008-VL | SEQ ID NO.191 | QNVGTA | SEQ ID NO.192 | SA | SEQ ID NO.193 | QQYSSSPYT |
| mab009-V H | SEQ ID NO.194 | GYTFTSYW | SEQ ID NO.195 | IYPGNSDT | SEQ ID NO.196 | TRSYDYGDL DY |
| mab009-VL | SEQ ID NO.197 | QTISDY | SEQ ID NO.198 | YA | SEQ ID NO.199 | QNGHSFPLT |
| mab010-V H | SEQ ID NO.200 | GYTFTSYG | SEQ ID NO.201 | IFPRSGNT | SEQ ID NO.202 | ARTTVFAPY YFDY |
| mab010-VL | SEQ ID NO.203 | ENINSN | SEQ ID NO.204 | AA | SEQ ID NO.205 | QHFWGLPWT |
| mab011-VH | SEQ ID NO.206 | GYTFTDYY | SEQ ID NO.207 | ISPNYGGT | SEQ ID NO.208 | TRSFYYYGT GYDWYFDV |
| mab011-VL | SEQ ID NO.209 | QSISNN | SEQ ID NO.210 | YA | SEQ ID NO.211 | QQSNSWPWT |
| mab012-V H | SEQ ID NO.212 | GYTFTDYY | SEQ ID NO.213 | TLPGSGSP | SEQ ID NO.214 | ARWHYYYG SSYHFDY |
| mab012-VL | SEQ ID NO.215 | QSIVHSDGNTY | SEQ ID NO.216 | KV | SEQ ID NO.217 | FQGSHVPFT |
| mab013-V H | SEQ ID NO.218 | GYAFSNYW | SEQ ID NO.219 | INPGDGDT | SEQ ID NO.220 | EREGRNYGD FAY |
| mab013-VL | SEQ ID NO.221 | KSVSTSGFSY | SEQ ID NO.222 | LA | SEQ ID NO.223 | QHSRELPFT |
| mab014-V H | SEQ ID NO.224 | GYTFTDYY | SEQ ID NO.225 | INPNHGDT | SEQ ID NO.226 | ARNFFYYGT TYGWYFDV |
| mab014-VL | SEQ ID NO.227 | EDIYNR | SEQ ID NO.228 | GA | SEQ ID NO.229 | QQYWSLPWT |
| mab015-V H | SEQ ID NO.230 | GITFSNFW | SEQ ID NO.231 | IRLKSDDYAT | SEQ ID NO.232 | TTGYYFDY |
| mab015-VL | SEQ ID NO.233 | ENVGTY | SEQ ID NO.234 | GA | SEQ ID NO.235 | GQFYSFPCT |
| mab016-V H | SEQ ID NO.236 | GFAFSNFW | SEQ ID NO.237 | IRLKSDNYAA | SEQ ID NO.238 | TQGNWVGFA Y |
| mab016-VL | SEQ ID NO.239 | QNVGTT | SEQ ID NO.240 | ST | SEQ ID NO.241 | QQYNNYPLT |

| **Chothia analysis** | | | | | | |
|---|---|---|---|---|---|---|
| mab001-V H | SEQ ID NO.242 | GYTFTTY | SEQ ID NO.243 | NTYSGV | SEQ ID NO.244 | IGDYDYGEY |
| mab001-VL | SEQ ID NO.245 | KSSQSLLDSDGK TYLN | SEQ ID NO.246 | LVSQLDS | SEQ ID NO.247 | WQGTHFPYT |
| mab002-V H | SEQ ID NO.248 | GYTFTNY | SEQ ID NO.249 | NTYTGE | SEQ ID NO.250 | MGDGYYDFT MDY |
| mab002-VL | SEQ ID NO.251 | SARSSVSYMQ | SEQ ID NO.252 | DTSKLAS | SEQ ID NO.253 | QQWSSDPPT |
| mab003-V H | SEQ ID NO.254 | GYTFTGN | SEQ ID NO.255 | LPGTGS | SEQ ID NO.256 | WGSTVVAYD Y |
| mab003-VL | SEQ ID NO.257 | RASKSISKYLA | SEQ ID NO.258 | SGSTLHS | SEQ ID NO.259 | QQHNEYPLT |
| mab004-V H | SEQ ID NO.260 | GYTFTSY | SEQ ID NO.261 | YPGSSS | SEQ ID NO.262 | SLLYAMDY |
| mab004-VL | SEQ ID NO.263 | RASESVDYDGD SYMN | SEQ ID NO.264 | VASNLES | SEQ ID NO.265 | LQSNEAPYT |
| mab005-V H | SEQ ID NO.266 | GYTLPGY | SEQ ID NO.267 | LPGSSS | SEQ ID NO.268 | TYGTSLDY |
| mab005-VL | SEQ ID NO.269 | RASQSVSNDLH | SEQ ID NO.270 | YASQSIS | SEQ ID NO.271 | QQSNNWPYT |
| mab006-V H | SEQ ID NO.272 | GYTFTSY | SEQ ID NO.273 | DPSDSL | SEQ ID NO.274 | DSTVVAPMD Y |
| mab006-VL | SEQ ID NO.275 | RATKSISKYLA | SEQ ID NO.276 | SGSTLQS | SEQ ID NO.277 | QQHHEYPYT |
| mab007-V H | SEQ ID NO.278 | GYTFTSY | SEQ ID NO.279 | DPSDSL | SEQ ID NO.280 | DSTVVAPMD Y |
| mab007-VL | SEQ ID NO.281 | RASTSISKYLA | SEQ ID NO.282 | SGSTLQS | SEQ ID NO.283 | QQHHVYPYT |
| mab008-V H | SEQ ID NO.284 | GYTFTDY | SEQ ID NO.285 | NPYNGG | SEQ ID NO.286 | YDYDYDVEG AMDY |
| mab008-VL | SEQ ID NO.287 | KASQNVGTAVA | SEQ ID NO.288 | SASIRYT | SEQ ID NO.289 | QQYSSSPYT |
| mab009-V H | SEQ ID NO.290 | GYTFTSY | SEQ ID NO.291 | YPGNSD | SEQ ID NO.292 | SYDYGDLDY |
| mab009-VL | SEQ ID NO.293 | RASQTISDYLH | SEQ ID NO.294 | YASQSIS | SEQ ID NO.295 | QNGHSFPLT |
| mab010-V H | SEQ ID NO.296 | GYTFTSY | SEQ ID NO.297 | FPRSGN | SEQ ID NO.298 | TTVFAPYYF DY |
| mab010-VL | SEQ ID NO.299 | RASENINSNLA | SEQ ID NO.300 | AATNLAD | SEQ ID NO.301 | QHFWGLPWT |
| mab011-VH | SEQ ID NO.302 | GYTFTDY | SEQ ID NO.303 | SPNYGG | SEQ ID NO.304 | SFYYYGTGY DWYFDV |
| mab011-VL | SEQ ID NO.305 | RASQSISNNLH | SEQ ID NO.306 | YASQSIS | SEQ ID NO.307 | QQSNSWPWT |
| mab012-V H | SEQ ID NO.308 | GYTFTDY | SEQ ID NO.309 | LPGSGS | SEQ ID NO.310 | WHYYYGSSY HFDY |
| mab012-VL | SEQ ID NO.311 | RSSQSIVHSDGN TYLE | SEQ ID NO.312 | KVSNRFS | SEQ ID NO.313 | FQGSHVPFT |
| mab013-V H | SEQ ID NO.314 | GYAFSNY | SEQ ID NO.315 | NPGDGD | SEQ ID NO.316 | EGRNYGDFA Y |
| mab013-VL | SEQ ID NO.317 | RASKSVSTSGFS YMH | SEQ ID NO.318 | LASNLES | SEQ ID NO.319 | QHSRELPFT |
| mab014-V H | SEQ ID NO.320 | GYTFTDY | SEQ ID NO.321 | NPNHGD | SEQ ID NO.322 | NFFYYGTTY GWYFDV |
| mab014-VL | SEQ ID NO.323 | KASEDIYNRLA | SEQ ID NO.324 | GATSLET | SEQ ID NO.325 | QQYWSLPWT |
| mab015-V H | SEQ ID NO.326 | GITFSNF | SEQ ID NO.327 | RLKSDDYA | SEQ ID NO.328 | GYYFDY |
| mab015-VL | SEQ ID NO.329 | KASENVGTYVS | SEQ ID NO.330 | GASNRYT | SEQ ID NO.331 | GQFYSFPCT |
| mab016-V H | SEQ ID NO.332 | GFAFSNF | SEQ ID NO.333 | RLKSDNYA | SEQ ID NO.334 | GNWVGFAY |
| mab016-VL | SEQ ID NO.335 | KASQNVGTTVA | SEQ ID NO.336 | STSNRFT | SEQ ID NO.337 | QQYNNYPLT |

### Example 4: Identification of DLL3 Human-Murine Chimeric Antibodies

### 4.1 Assay on binding of chimeric antibodies to human DLL3 protein by ELISA

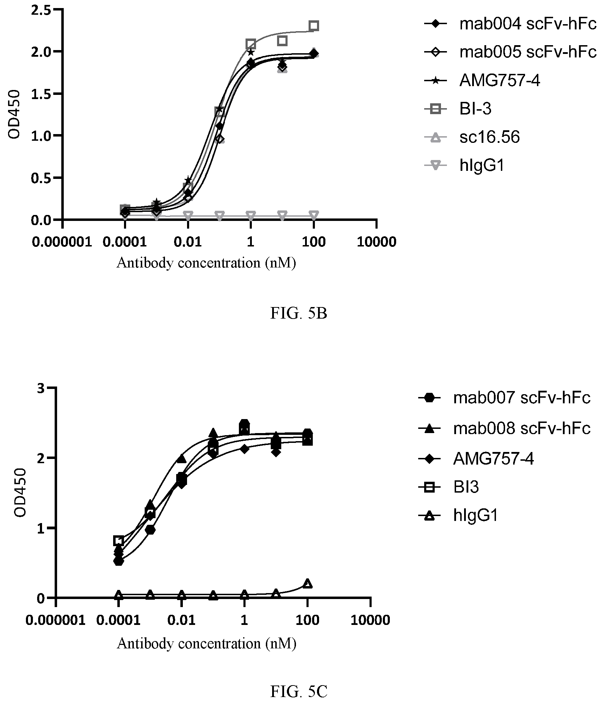

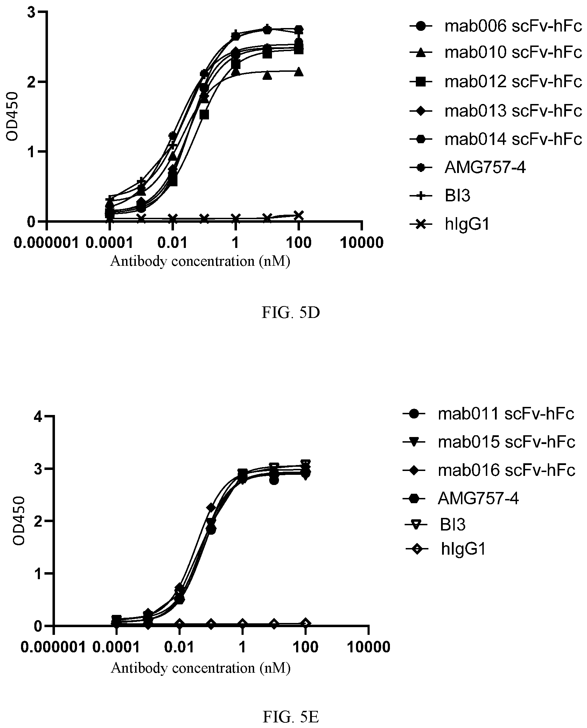

The chimeric antibodies obtained above were subjected to ELISA assay and data analysis according to the method in section 1.3 of Example 1. An ELISA plate reader (Multimode Plate Reader, EnSight, purchased from Perkin Elmer) was used to read OD_{450 nm} values, and the results for the binding activity of the chimeric antibodies for human DLL3 protein are shown in FIGs. 5A-5E. The results show that the chimeric antibodies exhibit good binding activity for human DLL3 protein. The IgG control was hIgG1, and data in the table are OD_{450 nm} values.

### 4.2. Assay on binding of chimeric antibodies to human DLL1 and DLL4 proteins by ELISA

The chimeric antibodies obtained above were subjected to ELISA assay and data analysis according to the method in section 1.3 of Example 1. An ELISA plate reader (Multimode Plate Reader, EnSight, purchased from Perkin Elmer) was used to read OD_{450 nm} values, and the maximum values of the binding of the chimeric antibodies to human DLL1 (purchased from Acro, Cat. No. DL1-H52H8) or DLL4 (purchased from Acro, Cat. No. DL4-H5227) protein at concentrations of 0.1, 1, 10, and 100 nM are shown in Table 8. The results show that mab010 scFv-hFc bound to hDLL1, mab013 scFv-hFc and mab015 scFv-hFc bound to hDLL1 weakly, and mab015 scFv-hFc bound to hDLL4. The remaining chimeric antibodies barely bound to human DLL1 and DLL4. The negative control was hIgG1, the positive controls were an anti-DLL1 antibody (purchased from R&D, Cat. No. MAB0108-100) and an anti-DLL4 antibody (purchased from R&D, Cat. No. MAB1506), and the data in the table are OD_{450 nm} values.

**Table 8. ELISA assay results for binding reactions of chimeric antibodies with human DLL1 and DLL4 proteins**

| Antibody | Emax of OD_{450 nm} binding value | |
|---|---|---|
| | hDLL1 | hDLL4 |
| mab001 scFv-hFc | 0.05 | 0.05 |
| mab002 scFv-hFc | 0.05 | 0.05 |
| mab003 scFv-hFc | 0.06 | 0.05 |
| hIgG1 | 0.05 | 0.07 |
| SC16.56 | 0.05 | 0.05 |
| AMG757-4 | 0.05 | 0.05 |
| Anti-DLL1 antibody | 1.74 | - |
| Anti-DLL4 antibody | - | 1.77 |
| mab004 scFv-hFc | 0.05 | 0.04 |
| mab005 scFv-hFc | 0.05 | 0.04 |
| hIgG1 | 0.04 | 0.05 |
| SC16.56 | 0.05 | 0.05 |
| AMG757-4 | 0.05 | 0.05 |
| BI3 | 0.05 | 0.05 |
| Anti-DLL1 antibody | 1.56 | - |
| Anti-DLL4 antibody | - | 1.09 |
| mab007 scFv-hFc | 0.07 | 0.06 |
| mab008 scFv-hFc | 0.09 | 0.08 |
| hIgG1 | 0.05 | 0.05 |
| AMG757-4 | 0.05 | 0.05 |
| BI3 | 0.05 | 0.05 |
| Anti-DLL1 antibody | 1.55 | - |
| Anti-DLL4 antibody | - | 1.20 |
| mab006 scFv-hFc | 0.08 | 0.07 |
| mab010 scFv-hFc | 1.16 | 0.06 |
| mab012 scFv-hFc | 0.08 | 0.08 |
| mab013 scFv-hFc | 0.32 | 0.07 |
| mab014 scFv-hFc | 0.08 | 0.06 |
| hIgG1 | 0.07 | 0.07 |
| AMG757-4 | 0.06 | 0.05 |
| BI3 | 0.07 | 0.05 |
| Anti-DLL1 antibody | 1.42 | - |
| Anti-DLL4 antibody | - | 1.99 |
| mab011 scFv-hFc | 0.06 | 0.06 |
| mab015 scFv-hFc | 0.51 | 1.39 |
| mab016 scFv-hFc | 0.05 | 0.04 |
| hIgG1 | 0.04 | 0.04 |
| AMG757-4 | 0.04 | 0.04 |
| BI3 | 0.05 | 0.04 |
| Anti-DLL1 antibody | 1.08 | - |
| Anti-DLL4 antibody | - | 1.16 |

### 4.3 Assay on binding activity of chimeric antibodies for SHP77 cells endogenously expressing DLL3 by FACS

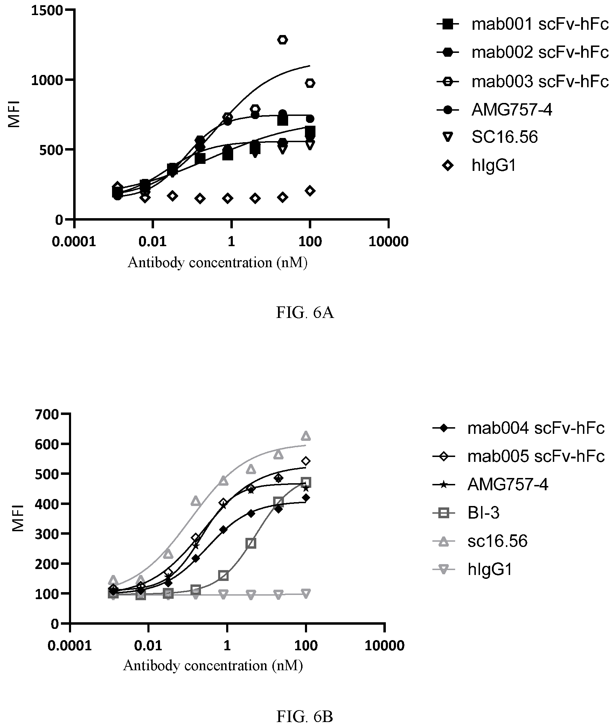

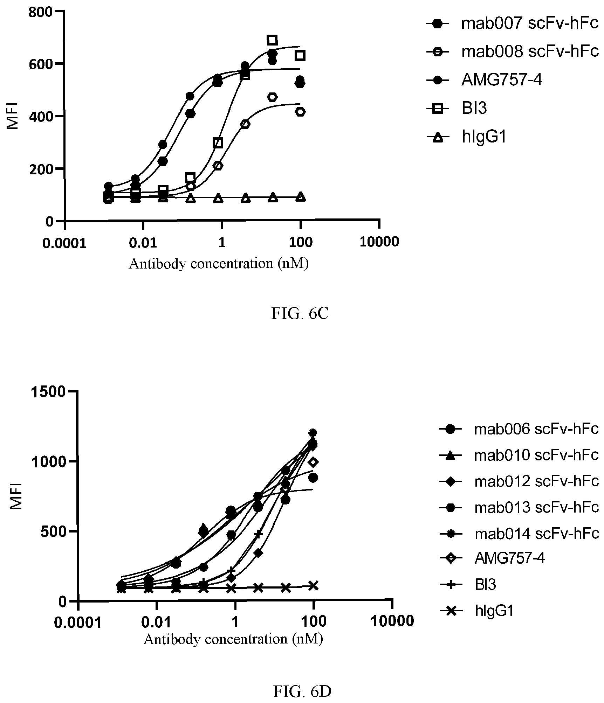

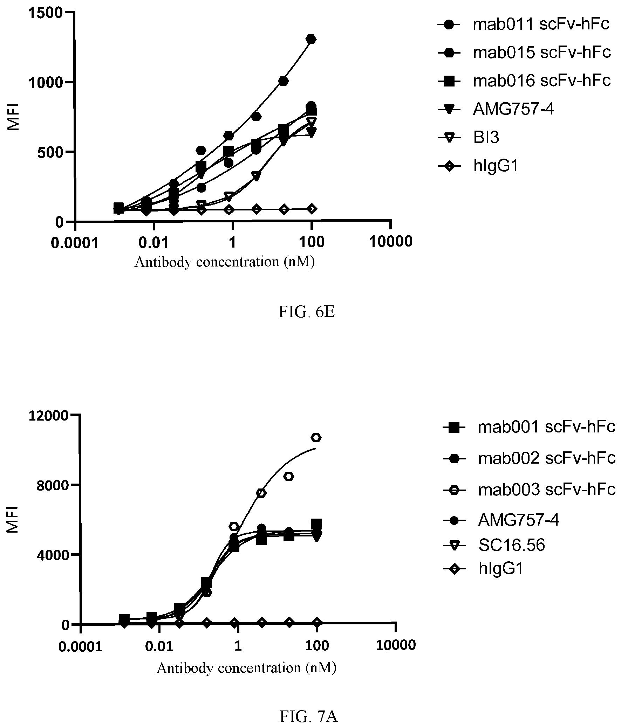

SHP77 is a human small cell lung cancer cell with moderate expression of DLL3 protein on the cell surface. The desired SHP77 cells were expanded to the logarithmic growth phase in a T-75 cell culture flask, the medium was removed by pipetting, and the cells were washed twice with a PBS buffer and digested with trypsin. Then, a complete medium was added to stop the digestion, and the cells were pipetted to form a single cell suspension. After counting, the cells were centrifuged, and the cell pellet was resuspended in an FACS buffer (PBS + 2% fetal bovine serum) to 2 × 10⁶ cells/mL. The cell resuspension was added to a 96-well FACS reaction plate at 50 µL/well, and a chimeric antibody sample to be tested was added at 50 µL/well. The mixture was incubated at 4 °C for 1 h. After the cells were washed 3 times by centrifugation with a PBS buffer, a goat anti-human IgG H+L antibody (Jackson, Cat. No. 109605088) was added at 50 µL/well. The mixture was incubated on ice for 1 h. After the cells were washed 3 times by centrifugation with a PBS buffer and resuspended in 100 µL of PBS, assay and analysis were performed by FACS (FACS Canto^{™}, purchased from BD). Data analysis was performed by software (FlowJo) to obtain the mean fluorescence intensity (MFI) of the cells. The results are shown in Table 9 and FIGs. 6A-6E, which indicate that the chimeric antibodies all can bind to SHP77 cells.

**Table 9. FACS assay results for binding activity of chimeric antibodies for SHP77 cells**

| Antibody | Antibody concentration (nM) | | | | | | | |
|---|---|---|---|---|---|---|---|---|
| | 100 | 20 | 4 | 0.8 | 0.16 | 0.032 | 0.0064 | 0.00128 |
| mab001 scFv-hFc | 631 | 709 | 505 | 461 | 435 | 362 | 247 | 196 |
| mab002 scFv-hFc | 596 | 548 | 535 | 498 | 518 | 371 | 255 | 197 |
| mab003 scFv-hFc | 976 | 1285 | 789 | 732 | 566 | 351 | 197 | 182 |
| AMG757-4 | 721 | 757 | 748 | 702 | 562 | 341 | 219 | 167 |
| SC16.56 | 529 | 500 | 476 | 465 | 432 | 334 | 235 | 188 |
| hIgG1 | 204 | 158 | 151 | 151 | 149 | 168 | 155 | 234 |
| mab004 scFv-hFc | 420 | 382 | 367 | 313 | 217 | 135 | 110 | 107 |
| mab005 scFv-hFc | 543 | 486 | 451 | 404 | 287 | 172 | 125 | 117 |
| AMG757-4 | 452 | 484 | 445 | 394 | 259 | 155 | 117 | 112 |
| BI3 | 471 | 406 | 268 | 160 | 113 | 100 | 96 | 101 |
| SC16.56 | 628 | 566 | 517 | 478 | 411 | 234 | 146 | 146 |
| hIgG1 | 98 | 95 | 94 | 96 | 96 | 94 | 95 | 94 |
| mab007 scFv-hFc | 521 | 634 | 563 | 525 | 406 | 227 | 135 | 103 |
| mab008 scFv-hFc | 412 | 468 | 365 | 208 | 130 | 98 | 90 | 82 |
| AMG757-4 | 534 | 606 | 588 | 542 | 473 | 291 | 160 | 133 |
| BI3 | 627 | 686 | 553 | 295 | 163 | 116 | 104 | 92 |
| hIgG1 | 90 | 88 | 86 | 86 | 86 | 90 | 88 | 91 |
| mab006 scFv-hFc | 876 | 718 | 664 | 643 | 497 | 267 | 152 | 112 |
| mab010 scFv-hFc | 1145 | 855 | 707 | 619 | 523 | 278 | 161 | 113 |
| mab012 scFv-hFc | 1100 | 729 | 337 | 164 | 110 | 98 | 94 | 94 |
| mab013 scFv-hFc | 1115 | 927 | 743 | 472 | 239 | 138 | 109 | 98 |
| mab014 scFv-hFc | 1197 | 829 | 681 | 461 | 237 | 137 | 104 | 96 |
| AMG757-4 | 987 | 794 | 677 | 632 | 482 | 278 | 158 | 116 |
| BI3 | 1132 | 836 | 473 | 214 | 131 | 104 | 94 | 91 |
| hIgG1 | 105 | 90 | 91 | 88 | 90 | 91 | 90 | 90 |
| mab011 scFv-hFc | 822 | 608 | 508 | 417 | 239 | 146 | 103 | 90 |
| mab015 scFv-hFc | 1301 | 1001 | 745 | 612 | 506 | 268 | 144 | 96 |
| mab016 scFv-hFc | 790 | 653 | 546 | 502 | 391 | 202 | 121 | 96 |
| AMG757-4 | 631 | 599 | 542 | 502 | 335 | 183 | 121 | 98 |
| BI3 | 703 | 567 | 3117 | 173 | 110 | 88 | 83 | 82 |
| hIgG1 | 85 | 82 | 83 | 81 | 81 | 79 | 77 | 94 |

### Example 5: Assay on Cross-Binding Activity of DLL3 Chimeric Antibodies

### (A) Assay on binding activity of chimeric antibodies for monkey DLL3 by FACS

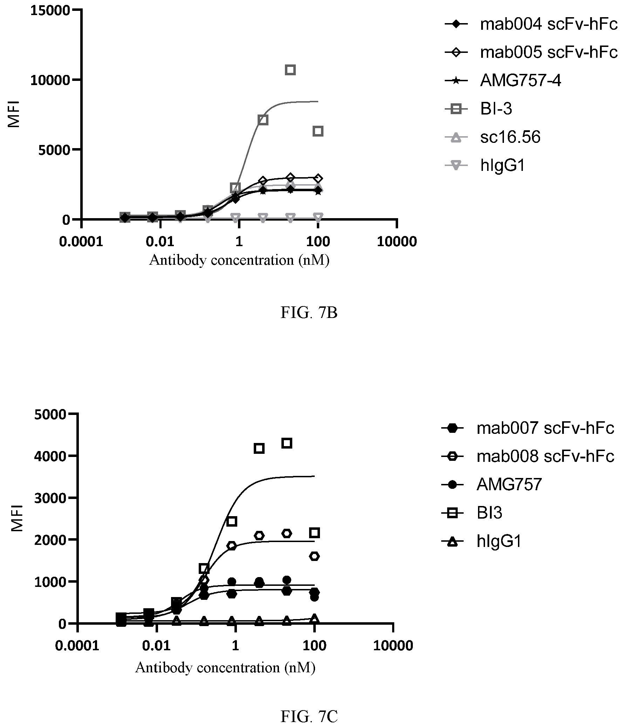

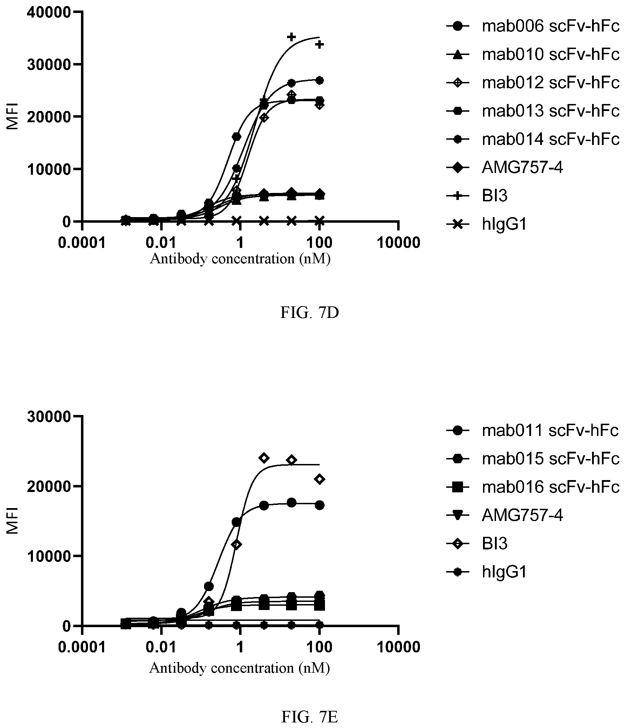

Reference is made to section 4.3 in Example 4 for the assay method. FACS results for the binding of the chimeric antibodies to CHO K1-cyno DLL3 cells, a cell line over-expressing monkey DLL3, are shown in Table 10 and FIGs. 7A-7E, and the results show that the chimeric antibodies all can bind to CHO K1-cyno DLL3 cells. The IgG control was hIgG1, and the data in the Table are mean fluorescence intensity MFI values.

**Table 10. FACS assay results for binding reactions of chimeric antibodies with CHO K1-cyno DLL3 cells**

| Antibody | Antibody concentration (nM) | | | | | | | |
|---|---|---|---|---|---|---|---|---|
| | 100 | 20 | 4 | 0.8 | 0.16 | 0.032 | 0.0064 | 0.00128 |
| mab001 scFv-hFc | 5681 | 5023 | 4789 | 4418 | 2370 | 894 | 393 | 260 |
| mab002 scFv-hFc | 5191 | 5052 | 4982 | 4660 | 2251 | 897 | 346 | 201 |
| mab003 scFv-hFc | 10643 | 8425 | 7468 | 5567 | 1817 | 566 | 243 | 163 |
| AMG757-4 | 5067 | 5269 | 5460 | 4943 | 2165 | 737 | 304 | 167 |
| SC16.56 | 4931 | 5008 | 5037 | 4513 | 1970 | 690 | 314 | 161 |
| hIgG1 | 59 | 60 | 56 | 59 | 64 | 69 | 68 | 77 |
| mab004 scFv-hFc | 2095 | 2155 | 2098 | 1431 | 470 | 213 | 132 | 120 |
| mab005 scFv-hFc | 2928 | 3001 | 2772 | 1584 | 474 | 197 | 131 | 120 |
| AMG757-4 | 1986 | 2145 | 2025 | 1716 | 603 | 265 | 164 | 195 |
| BI3 | 6301 | 10709 | 7122 | 2244 | 626 | 266 | 166 | 156 |
| SC16.56 | 2327 | 2628 | 2330 | 1896 | 700 | 299 | 189 | 185 |
| hIgG1 | 95 | 83 | 86 | 85 | 84 | 85 | 87 | 94 |
| mab007 scFv-hFc | 739 | 776 | 956 | 706 | 674 | 326 | 144 | 103 |
| mab008 scFv-hFc | 1602 | 2149 | 2092 | 1852 | 1037 | 411 | 169 | 89 |
| AMG757-4 | 628 | 1038 | 1006 | 999 | 838 | 479 | 224 | 142 |
| BI3 | 2165 | 4301 | 4177 | 2436 | 1316 | 497 | 236 | 135 |
| hIgG1 | 125 | 76 | 69 | 63 | 67 | 73 | 54 | 51 |
| mab006 scFv-hFc | 5044 | 5183 | 5132 | 4882 | 2825 | 1015 | 415 | 243 |
| mab010 scFv-hFc | 5107 | 4959 | 4763 | 4129 | 1765 | 658 | 311 | 207 |
| mab012 scFv-hFc | 22248 | 24229 | 19802 | 5946 | 1492 | 506 | 259 | 179 |
| mab013 scFv-hFc | 23070 | 23212 | 22186 | 16182 | 3551 | 1464 | 603 | 304 |
| mab014 scFv-hFc | 26915 | 26400 | 22600 | 10163 | 3139 | 1006 | 422 | 333 |
| AMG757-4 | 5256 | 5473 | 5257 | 4405 | 2287 | 804 | 372 | 230 |
| BI3 | 33799 | 35223 | 23274 | 8163 | 2293 | 807 | 373 | 240 |
| hIgG1 | 159 | 117 | 116 | 112 | 113 | 113 | 108 | 113 |
| mab011 scFv-hFc | 17287 | 17672 | 17231 | 14865 | 5667 | 1893 | 670 | 319 |
| mab015 scFv-hFc | 4336 | 4142 | 3858 | 3673 | 2820 | 1007 | 432 | 291 |
| mab016 scFv-hFc | 2958 | 2984 | 3032 | 2915 | 2211 | 946 | 421 | 322 |
| AMG757-4 | 3435 | 3558 | 3432 | 3353 | 2040 | 820 | 354 | 230 |
| BI3 | 21000 | 23737 | 24047 | 11673 | 3458 | 1063 | 448 | 294 |
| hIgG1 | 126 | 116 | 113 | 113 | 117 | 118 | 120 | 125 |

### Example 6: Humanization Design of DLL3 Antibodies

By comparing the IMGT database (http://imgt.cines.fr) for germline genes of heavy and light chain variable regions of human antibodies with the Molecular Operating Environment (MOE) software, germline genes, with high homology with a murine antibody, of heavy and light chain variable regions were selected as templates, and CDRs of the murine antibody were grafted into corresponding human templates to form a variable region sequence of FR1-CDR1-FR2-CDR2-FR3-CDR3-FR4. Back mutations and/or hotspot mutations were performed as needed. The antibody sequences and CDR sequences in this example were numbered according to the Kabat numbering scheme.

### 6.1 Humanization of antibody mab006

### 6.1.1 Selection of germline sequence for mab006

With IGKV1-39*01 or IGKV4-1*01 and IGKJ4*01 as the humanized light chain templates and IGHV1-3*01 and IGHJ6*01 as the humanized heavy chain templates for the antibody mab006, CDRs of the murine antibody mab006 were grafted into corresponding humanization templates to obtain the humanized antibodies of mab006.

### 6.1.2 Design of back mutations and hotspot mutations for mab006 humanized antibodies

Key amino acids in the FR region sequences of the mab006 humanized antibodies were back-mutated as needed to ensure the original affinity. The detailed mutation design is shown in Table 11 (back mutations were numbered in a natural order).

**Table 11. Back mutation design for mab006 humanized antibodies**

| **VL** | | **VH** | |
|---|---|---|---|
| L1 | Graft(IGKV1-39*01) + Y49F | H1 | Graft(IGHV1-3*01) + R72V |
| - | - | H2 | Graft(IGHV1-3*01) + I70L,R72V |
| L4 | Graft(IGKV4-1*01) + P43T,P44N,Y49F | H4 | Graft(IGHV1-3*01) + I70L,R72V,A76S |
| FR4 | IGKJ4*01 | FR4 | IGHJ6*01 |

### 6.1.3 Sequence combinations of mab006 humanized antibodies

The designs of back mutations for humanized antibodies in Table 11 were combined to eventually obtain multiple mab006 humanized antibodies (see Table 12 for details).

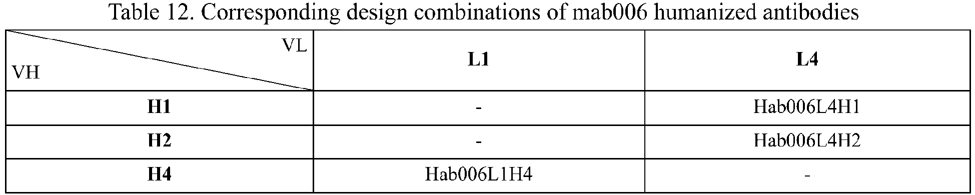

Amino acid sequences of heavy and light chain variable regions after humanization are shown in Table 13:

**Table 13. Amino acid sequences of back-mutated variable regions of mab006 humanized antibodies**

| Heavy and light chains | Sequence No. | Amino acid sequence |
|---|---|---|
| L1 | SEQ ID NO.338 | DIQMTQSPSSLSASVGDRVTITCRATKSISKYLAWYQQKPGKAPKLLIFSGS TLQSGVPSRFSGSGSGTDFTLTISSLQPEDFATYYCQQHHEYPYTFGGGTKV EIK |
| L4 | SEQ ID NO.339 | DIVMTQSPDSLAVSLGERATINCRATKSISKYLAWYQQKPGQTNKLLIFSGS TLQSGVPDRFSGSGSGTDFTLTISSLQAEDVAVYYCQQHHEYPYTFGGGTK VEIK |
| H1 | SEQ ID NO.340 | EVQLVQSGAEVKKPGASVKVSCKASGYTFTSYWMHWVRQAPGQRLEW MGVIDPSDSLANYNQEFKGRVTITVDTSASTAYMELSSLRSEDTAVYYCAR DSTVVAPMDYWGQGTTVTVSS |
| H2 | SEQ ID NO.341 | EVQLVQSGAEVKKPGASVKVSCKASGYTFTSYWMHWVRQAPGQRLEW MGVIDPSDSLANYNQEFKGRVTLTVDTSASTAYMELSSLRSEDTAVYYCAR DSTVVAPMDYWGQGTTVTVSS |
| H4 | SEQ ID NO.342 | EVQLVQSGAEVKKPGASVKVSCKASGYTFTSYWMHWVRQAPGQRLEW |
| | | MGVIDPSDSLANYNQEFKGRVTLTVDTSSSTAYMELSSLRSEDTAVYYCAR DSTVVAPMDYWGQGTTVTVSS |

According to the Kabat numbering scheme, the analysis results for CDR sequences of heavy and light chain variable regions of the humanized antibodies above are shown in Table 14:

**Table 14. Kabat analysis results for CDR sequences of heavy and light chain variable regions of mab006 humanized antibodies**

| Heavy and light chain variable regions | CDR1 | CDR2 | CDR3 |
|---|---|---|---|
| Hab006L1/4 | RATKSISKYLA SEQ ID NO. 83 | SGSTLQS SEQ ID NO. 84 | QQHHEYPYT SEQ ID NO. 85 |
| Hab006H1/2/4 | SYWMH SEQ ID NO. 80 | VIDPSDSLANYNQEFKG SEQ ID NO. 81 | DSTVVAPMDY SEQ ID NO.82 |

### 6.2 Humanization of antibody mab009

### 6.2.1 Selection of germline sequence for mab009

With IGKV2-28*01 and IGKJ2*01 as the humanized light chain templates and IGHV1-3*01 as the humanized heavy chain template for the antibody mab009, CDRs of the murine antibody mab009 were grafted into corresponding humanization templates to obtain the humanized antibodies of mab009.

### 6.2.2 Design of back mutations and hotspot mutations for mab009 humanized antibodies

Key amino acids in FR sequences of mab009 humanized antibodies were back-mutated as needed to ensure the original affinity. In view of a high-risk modifiable site NG on the light chain of mab009, amino acid mutations were performed on the NG by means of computational simulation based on the antibody structure to eliminate modification risks. The detailed mutation design is shown in Table 15 (back mutations were numbered in a natural order).

**Table 15. Design of back mutations and hotspot mutations for mab009 humanized antibodies**

| **VL** | | **VH** | |
|---|---|---|---|
| L1a | Graft(IGKV2-28*01) + Y49K,T69S + N90Q | H1 | Graft(IGHV1-3*01) + R72A,A97T |
| L2 | Graft(IGKV2-28*01) + Q45R,Y49K | | |
| L2a | Graft(IGKV2-28*01) + Q45R,Y49K + N90Q | | |
| L3a | Graft(IGKV2-28*01) + P40S,G41H,Y49K + N90Q | | |
| FR4 | IGKJ2*01 | FR4 | IGHJ6*01 |

### 6.2.3 Sequence combinations of mab009 humanized antibodies

The designs of back mutations and hotspot mutations for the mab009 humanized antibodies in Table 15 were combined to eventually obtain multiple humanized antibodies (see Table 16 for details).

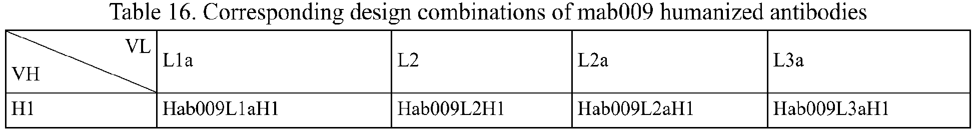

Amino acid sequences of heavy and light chain variable regions after humanization are shown in Table 17:

**Table 17. Amino acid sequences of back-mutated variable regions of mab009 humanized antibodies**

| Heavy and light chains | Sequence No. | Amino acid sequence |
|---|---|---|
| L1a | SEQ ID NO.343 | DIVMTQSPLSLPVTPGEPASISCRASQTISDYLHWYLQKPGQSPQLLIKYAS QSISGVPDRFSGSGSGSDFTLKISRVEAEDVGVYYCQQGHSFPLTFGQGTKL EIK |
| L2 | SEQ ID NO.344 | DIVMTQSPLSLPVTPGEPASISCRASQTISDYLHWYLQKPGQSPRLLIKYAS QSISGVPDRFSGSGSGTDFTLKISRVEAEDVGVYYCQNGHSFPLTFGQGTK LEIK |
| L2a | SEQ ID NO.345 | DIVMTQSPLSLPVTPGEPASISCRASQTISDYLHWYLQKPGQSPRLLIKYAS QSISGVPDRFSGSGSGTDFTLKISRVEAEDVGVYYCQQGHSFPLTFGQGTK LEIK |
| L3a | SEQ ID NO.346 | DIVMTQSPLSLPVTPGEPASISCRASQTISDYLHWYLQKSHQSPQLLIKYAS QSISGVPDRFSGSGSGTDFTLKISRVEAEDVGVYYCQQGHSFPLTFGQGTK LEIK |
| H1 | SEQ ID NO.347 | EVQLVQSGAEVKKPGASVKVSCKASGYTFTSYWMHWVRQAPGQRLEW MGTIYPGNSDTRYNQKFKDRVTITADTSASTAYMELSSLRSEDTAVYYCTR SYDYGDLDYWGQGTTVTVSS |

According to the Kabat numbering scheme, the analysis results for CDR sequences of heavy and light chain variable regions of the humanized antibodies above are shown in Table 18:

**Table 18. Kabat analysis results for CDR sequences of heavy and light chain variable regions of mab009 humanized antibodies**

| Heavy and light chains | CDR1 | CDR2 | CDR3 |
|---|---|---|---|
| Hab009L2 | RASQTISDYLH SEQ ID NO. 101 | YASQSIS SEQ ID NO. 102 | QNGHSFPLT SEQ ID NO.103 |
| Hab009L1a/2a/3a | RASQTISDYLH SEQ ID NO. 101 | YASQSIS SEQ ID NO. 102 | QQGHSFPLT SEQ ID NO. 348 |
| Hab009H1 | SYWMH SEQ ID NO. 98 | TIYPGNSDTRYNQKFKD SEQ ID NO.99 | SYDYGDLDY SEQ ID NO. 100 |

### 6.3 Humanization of antibody mab013

### 6.3.1 Selection of germline sequence for mab013

With IGKV3-11*01 and IGKJ2*01 as the humanized light chain templates and IGHV1-69*02 and IGHJ6*01 as the humanized heavy chain templates for the antibody mab013, CDRs of the murine antibody mab013 were grafted into corresponding humanization templates to obtain the humanized antibodies of mab013.

### 6.3.2 Design of back mutations and hotspot mutations for mab013 humanized antibodies

Key amino acids in FR sequences of the humanized antibodies were back-mutated as needed to ensure the original affinity. In view of a high-risk modifiable site NG on the heavy chain of mab013, amino acid mutations were performed on the NG by means of computational simulation based on the antibody structure to eliminate modification risks. The detailed mutation design is shown in Table 19 (back mutations were numbered in a natural order).

**Table 19. Design of back mutations and hotspot mutations for mab013 humanized antibodies**

| VL | | VH | |
|---|---|---|---|
| L2 | Graft(IGKV3-11*01) + A47P | H2a | Graft(IGHV1-69*02) + G27Y,T28A,A97E + G62S |
| L2+I52V | L2+I52V | - | - |
| L2+A55G | L2+A55G | - | - |
| L2+N57D | L2+N57D | - | - |
| L2+I52V+Q104E | L2+I52V+Q104E | - | - |
| L2+A55G+L108I | L2+A55G+L108I | - | - |
| L2+N57D+Q104E | L2+N57D+Q104E | - | - |
| FR4 | IGKJ2*01 | FR4 | IGHJ6*01 |

### 6.3.3 Sequence combinations of mab013 humanized antibodies

The designs of back mutations and hotspot mutations for the mab013 humanized antibodies in Table 19 were combined to eventually obtain multiple humanized antibodies (see Table 20 for details).

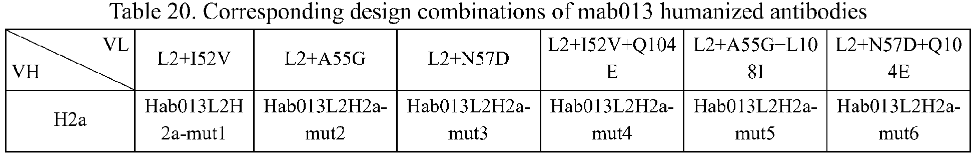

Amino acid sequences of heavy and light chain variable regions after humanization are shown in Table 21:

**Table 21. Amino acid sequences of back-mutated variable regions of mab013 humanized antibodies**

| Heavy and light chains | Sequence No. | Amino acid sequence |
|---|---|---|
| L2 | SEQ ID NO.349 | EIVLTQSPATLSLSPGERATLSCRASKSVSTSGFSYMHWYQQKPGQPPRLLI YLASNLESGIPARFSGSGSGTDFTLTISSLEPEDFAVYYCQHSRELPFTFGQG TKLEIK |
| L2+I52V | SEQ ID NO.350 | EIVLTQSPATLSLSPGERATLSCRASKSVSTSGFSYMHWYQQKPGQPPRLL VYLASNLESGIPARFSGSGSGTDFTLTISSLEPEDFAVYYCQHSRELPFTFGQ GTKLEIK |
| L2+A55G | SEQ ID NO.351 | EIVLTQSPATLSLSPGERATLSCRASKSVSTSGFSYMHWYQQKPGQPPRLLI YLGSNLESGIPARFSGSGSGTDFTLTISSLEPEDFAVYYCQHSRELPFTFGQG TKLEIK |
| L2+N57D | SEQ ID NO.352 | EIVLTQSPATLSLSPGERATLSCRASKSVSTSGFSYMHWYQQKPGQPPRLLI YLASDLESGIPARFSGSGSGTDFTLTISSLEPEDFAVYYCQHSRELPFTFGQG TKLEIK |
| L2+I52V+ Q104E | SEQ ID NO.353 | EIVLTQSPATLSLSPGERATLSCRASKSVSTSGFSYMHWYQQKPGQPPRLL VYLASNLESGIPARFSGSGSGTDFTLTISSLEPEDFAVYYCQHSRELPFTFGE GTKLEIK |
| L2+A55G+ L108I | SEQ ID NO.354 | EIVLTQSPATLSLSPGERATLSCRASKSVSTSGFSYMHWYQQKPGQPPRLLI YLGSNLESGIPARFSGSGSGTDFTLTISSLEPEDFAVYYCQHSRELPFTFGQG TKIEIK |
| L2+N57D+ | SEQ ID NO.355 | EIVLTQSPATLSLSPGERATLSCRASKSVSTSGFSYMHWYQQKPGQPPRLLI |
| Q104E | | YLASDLESGIPARFSGSGSGTDFTLTISSLEPEDFAVYYCQHSRELPFTFGEG TKLEIK |
| H2a | SEQ ID NO.356 | EVQLVQSGAEVKKPGSSVKVSCKASGYAFSNYWINWVRQAPGQGLEWM GQINPGDGDTIYNSKFKGRVTITADKSTSTAYMELSSLRSEDTAVYYCERE GRNYGDFAYWGQGTTVTVSS |

According to the Kabat numbering scheme, the analysis results for CDR sequences of heavy and light chain variable regions of the humanized antibodies above are shown in Table 22:

**Table 22. Kabat analysis results for CDR sequences of heavy and light chain variable regions of mab013 humanized antibodies**

| Heavy and light chains | CDR1 | CDR2 | CDR3 |
|---|---|---|---|
| Hab013L2/ L2+I52V/L2+I52V+Q104E | RASKSVSTSGFSYMH SEQ ID NO. 125 | LASNLES SEQ ID NO.126 | QHSRELPFT SEQ ID NO. 127 |
| Hab013L2+A55G/ L2+A55G+L108I | RASKSVSTSGFSYMH SEQ ID NO. 125 | LGSNLES SEQ ID NO. 357 | QHSRELPFT SEQ ID NO. 127 |
| Hab013L2+N57D/ L2+N57D+Q104E | RASKSVSTSGFSYMH SEQ ID NO. 125 | LASDLES SEQ ID NO. 358 | QHSRELPFT SEQ ID NO. 127 |
| Hab013H2a | NYWIN SEQ ID NO.122 | QINPGDGDTIYNSKFKG SEQ ID NO. 123 | EGRNYGDFAY SEQ ID NO. 124 |

### 6.4 Humanization of antibody mab014

### 6.4.1 Selection of germline sequence for mab014

With IGKV1-27*01, IGKV4-1*01, and IGKJ4*01 as the humanized light chain templates and IGHV1-3*01 and IGHJ6*01 as the humanized heavy chain templates for the antibody mab014, CDRs of the murine antibody mab014 were grafted into corresponding humanization templates to obtain the humanized antibodies of mab014.

### 6.4.2 Design of back mutations and hotspot mutations for mab014 humanized antibodies

Key amino acids in the FR region sequences of the mab014 humanized antibodies were back-mutated as needed to ensure the original affinity. The detailed mutation design is shown in Table 23 (back mutations were numbered in a natural order).

**Table 23. Back mutation design for mab014 humanized antibodies**

| **VL** | | **VH** | |
|---|---|---|---|
| L1 | Graft(IGKV1-27*01) + Y49S | H1 | Graft(IGHV1-3*01) + R72V,T74K |
| L2 | Graft(IGKV1-27*01) + Y49S,T69K | H2 | Graft(IGHV1-3*01) + I70L,R72V,T74K |
| L3 | Graft(IGKV4-1*01) + P43T,Y49S,T69K | - | - |
| FR4 | IGKJ4*01 | FR4 | IGHJ6*01 |

### 6.4.3 Sequence combinations of mab014 humanized antibodies

The designs of back mutations for mab014 humanized antibodies in Table 23 were combined to eventually obtain multiple mab014 humanized antibodies (see Table 24 for details).

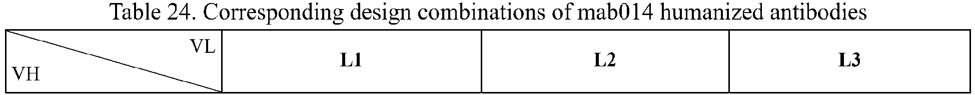

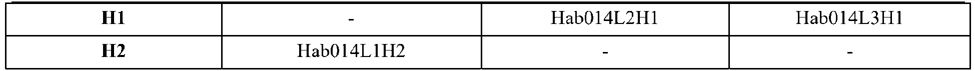

Amino acid sequences of heavy and light chain variable regions after humanization are shown in Table 25:

**Table 25. Amino acid sequences of back-mutated variable regions of mab014 humanized antibodies**

| Heavy and light chains | Sequence No. | Amino acid sequence |
|---|---|---|
| L1 | SEQ ID NO.359 | DIQMTQSPSSLSASVGDRVTITCKASEDIYNRLAWYQQKPGKVPKLLISG ATSLETGVPSRFSGSGSGTDFTLTISSLQPEDVATYYCQQYWSLPWTFGG GTKVEIK |
| L2 | SEQ ID NO.360 | DIQMTQSPSSLSASVGDRVTITCKASEDIYNRLAWYQQKPGKVPKLLISG ATSLETGVPSRFSGSGSGKDFTLTISSLQPEDVATYYCQQYWSLPWTFGG GTKVEIK |
| L3 | SEQ ID NO.361 | DIVMTQSPDSLAVSLGERATINCKASEDIYNRLAWYQQKPGQTPKLLISG ATSLETGVPDRFSGSGSGKDFTLTISSLQAEDVAVYYCQQYWSLPWTFG GGTKVEIK |
| H1 | SEQ ID NO.362 | EVQLVQSGAEVKKPGASVKVSCKASGYTFTDYYMNWVRQAPGQRLE WMGDINPNHGDTGYNQKFTGRVTITVDKSASTAYMELSSLRSEDTAVY YCARNFFYYGTTYGWYFDVWGQGTTVTVSS |
| H2 | SEQ ID NO.363 | EVQLVQSGAEVKKPGASVKVSCKASGYTFTDYYMNWVRQAPGQRLE WMGDINPNHGDTGYNQKFTGRVTLTVDKSASTAYMELSSLRSEDTAVY YCARNFFYYGTTYGWYFDVWGQGTTVTVSS |

According to the Kabat numbering scheme, the analysis results for CDR sequences of heavy and light chain variable regions of the humanized antibodies above are shown in Table 26:

**Table 26. Kabat analysis results for CDR sequences of heavy and light chain variable regions of mab014 humanized antibodies**

| Heavy and light chains | CDR1 | CDR2 | CDR3 |
|---|---|---|---|
| Hab014L1/2/3 | KASEDIYNRLA SEQ ID NO. 131 | GATSLET SEQ ID NO. 132 | QQYWSLPWT SEQ ID NO. 133 |
| Hab014H1/2 | DYYMN SEQ ID NO. 128 | DINPNHGDTGYNQKFTG SEQ ID NO. 129 | NFFYYGTTYGWYFDV SEQ ID NO.130 |

### 6.5 Humanization of antibody mab015

### 6.5.1 Selection of germline sequence for mab015

With IGKV3-11*01, IGKV1-39*01, and IGKJ4*01 as the humanized light chain templates and IGHV3-15*01 and IGHJ6*01 as the humanized heavy chain templates for the antibody mab015, CDRs of the murine antibody mab015 were grafted into corresponding humanization templates to obtain the humanized antibodies of mab015.

### 6.5.2 Design of back mutations and hotspot mutations for mab015 humanized antibodies

Key amino acids in FR sequences of the mab015 humanized antibodies were back-mutated as needed to ensure the original affinity. In view of a high-risk modifiable site DD on the heavy chain of mab015, amino acid mutations were performed on the DD by means of computational simulation based on the antibody structure to eliminate modification risks. The detailed mutation design is shown in Table 27 (back mutations were numbered in a natural order).

**Table 27. Design of back mutations and hotspot mutations for mab015 humanized antibodies**

| **VL** | | **VH** | |
|---|---|---|---|
| L4 | Graft(IGKV3-11*01) +E1N,Y36F,A43S | H1a | Graft(IGHV3-15*01) + F27I,L81V + D75E |
| L5 | Graft(IGKV1-39*01) + D1N,A43S | H1b | Graft(IGHV3-15*01) + F27I,L81V + D56E,D75E |
| | | H5a | Graft(IGHV3-15*01) + A23V,F27I,L81V + D75E |
| | | H6a | Graft(IGHV3-15*01) + F27I,G42E,L81V + D75E |
| | | H6b | Graft(IGHV3-15*01) + F27I,G42E,L81V + D57Q,D75E |
| FR4 | IGKJ4*01 | FR4 | IGHJ6*01 |

### 6.5.3 Sequence combinations of mab015 humanized antibodies

The designs of back mutations and hotspot mutations for the mab015 humanized antibodies in Table 27 were combined to eventually obtain multiple mab015 humanized antibodies (see Table 28 for details).

**Table 28. Corresponding design combinations of mab015 humanized antibodies**

| VH | **L4** | **L5** |
|---|---|---|
| VL | | |
| **H1a** | Hab015L4H1a | Hab015L5H1a |
| **H1b** | Hab015L4H1b | Hab015L5H1b |
| **H5a** | Hab015L4H5a | Hab015L5H5a |
| **H6a** | Hab015L4H6a | Hab015L5H6a |
| **H6b** | Hab015L4H6b | Hab015L5H6b |

Amino acid sequences of heavy and light chain variable regions after humanization are shown in Table 29:

**Table 29. Amino acid sequences of back-mutated variable regions of mab015 humanized antibodies**

| Heavy and light chains | Sequence No. | Amino acid sequence |
|---|---|---|
| L4 | SEQ ID NO.364 | NIVLTQSPATLSLSPGERATLSCKASENVGTYVSWFQQKPGQSPRLLIYG ASNRYTGIPARFSGSGSGTDFTLTISSLEPEDFAVYYCGQFYSFPSTFGGG TKVEIK |
| L5 | SEQ ID NO.365 | NIQMTQSPSSLSASVGDRVTITCKASENVGTYVSWYQQKPGKSPKLLIY GASNRYTGVPSRFSGSGSGTDFTLTISSLQPEDFATYYCGQFYSFPSTFGG GTKVEIK |
| H1a | SEQ ID NO.366 | EVQLVESGGGLVKPGGSLRLSCAASGITFSNFWMNWVRQAPGKGLEW VGQIRLKSDDYATHYAESVKGRFTISREDSKNTVYLQMNSLKTEDTAVY YCTTGYYFDYWGQGTTVTVSS |
| H1b | SEQ ID NO.367 | EVQLVESGGGLVKPGGSLRLSCAASGITFSNFWMNWVRQAPGKGLEW VGQIRLKSEDYATHYAESVKGRFTISREDSKNTVYLQMNSLKTEDTAVY YCTTGYYFDYWGQGTTVTVSS |
| H5a | SEQ ID NO.368 | EVQLVESGGGLVKPGGSLRLSCVASGITFSNFWMNWVRQAPGKGLEWV GQIRLKSDDYATHYAESVKGRFTISREDSKNTVYLQMNSLKTEDTAVYY CTTGYYFDYWGQGTTVTVSS |
| H6a | SEQ ID NO.369 | EVQLVESGGGLVKPGGSLRLSCAASGITFSNFWMNWVRQAPEKGLEWV GQIRLKSDDYATHYAESVKGRFTISREDSKNTVYLQMNSLKTEDTAVYY CTTGYYFDYWGQGTTVTVSS |
| H6b | SEQ ID NO.370 | EVQLVESGGGLVKPGGSLRLSCAASGITFSNFWMNWVRQAPEKGLEWV GQIRLKSDQYATHYAESVKGRFTISREDSKNTVYLQMNSLKTEDTAVYY CTTGYYFDYWGQGTTVTVSS |

According to the Kabat numbering scheme, the analysis results for CDR sequences of heavy and light chain variable regions of the humanized antibodies above are shown in Table 30:

**Table 30. Kabat analysis results for CDR sequences of heavy and light chain variable regions of mab015 humanized antibodies**

| Heavy and light chains | CDR1 | CDR2 | CDR3 |
|---|---|---|---|
| Hab015L4/5 | KASENVGTYVS | GASNRYT | GQFYSFPST |
| | SEQ ID NO. 137 | SEQ ID NO. 138 | SEQ ID NO. 371 |
| Hab015H1a/5a/6a | NFWMN | QIRLKSDDYATHYAESVKG | GYYFDY |
| | SEQ ID NO. 134 | SEQ ID NO. 135 | SEQ ID NO. 136 |
| Hab015H6b | NFWMN | QIRLKSDQYATHYAESVKG | GYYFDY |
| | SEQ ID NO. 134 | SEQ ID NO. 372 | SEQ ID NO. 136 |
| Hab015H1b | NFWMN | QIRLKSEDYATHYAESVKG | GYYFDY |
| | SEQ ID NO. 134 | SEQ ID NO. 373 | SEQ ID NO. 136 |

### 6.6 Humanization of antibody mab008

### 6.6.1 Selection of germline sequence for mab008

With IGKV1-39*01, IGKV4-1*01, and IGKJ4*01 as the humanized light chain templates and IGHV1-3*01 and IGHJ6*01 as the humanized heavy chain templates for the antibody mab008, CDRs of the murine antibody mab008 were grafted into corresponding humanization templates to obtain the humanized antibodies of mab008.

### 6.6.2 Design of back mutations and hotspot mutations for mab008 humanized antibodies

Key amino acids in FR sequences of the mab008 humanized antibodies were back-mutated as needed to ensure the original affinity. In view of a high-risk modifiable site NG on the heavy chain of mab008, amino acid mutations were performed on the NG by means of computational simulation based on the antibody structure to eliminate modification risks. The detailed mutation design is shown in Table 31 (back mutations were numbered in a natural order).

**Table 31. Design of back mutations and hotspot mutations for mab008 humanized antibodies**

| **VL** | | **VH** | |
|---|---|---|---|
| L1 | Graft(IGKV1-39*01) + A43S,Y49F | H2a | Graft(IGHV1-3*01) + I70L,R72V,T74K,A97S + G56S |
| L3 | Graft(IGKV4-1*01) + P43S,Y49F | H5 | Graft(IGHV1-3*01) + Q39R,R72V,T74K,A97S |
| FR4 | IGKJ4*01 | FR4 | IGHJ6*01 |

### 6.6.3 Sequence combinations of mab008 humanized antibodies

The designs of back mutations and hotspot mutations for the mab008 humanized antibodies in Table 31 were combined to eventually obtain multiple mab008 humanized antibodies (see Table 32 for details).

**Table 32. Corresponding design combinations of mab008 humanized antibodies**

| VH | **L1** | **L3** |
|---|---|---|
| VL | | |
| **H2a** | Hab008L1H2a | Hab008L3H2a |
| **H5** | Hab008L1H5 | Hab008L3H5 |

Amino acid sequences of heavy and light chain variable regions after humanization are shown in Table 33:

**Table 33. Amino acid sequences of back-mutated variable regions of mab008 humanized antibodies**

| Heavy and light chains | Sequence No. | Amino acid sequence |
|---|---|---|
| L1 | SEQ ID NO.374 | DIQMTQSPSSLSASVGDRVTITCKASQNVGTAVAWYQQKPGKSPKLLI FSASIRYTGVPSRFSGSGSGTDFTLTISSLQPEDFATYYCQQYSSSPYTF GGGTKVEIK |
| L3 | SEQ ID NO.375 | DIVMTQSPDSLAVSLGERATINCKASQNVGTAVAWYQQKPGQSPKLLI FSASIRYTGVPDRFSGSGSGTDFTLTISSLQAEDVAVYYCQQYSSSPYT FGGGTKVEIK |
| H2a | SEQ ID NO.376 | EVQLVQSGAEVKKPGASVKVSCKASGYTFTDYYMNWVRQAPGQRL EWMGIINPYNSGTSYNQKFRGRVTLTVDKSASTAYMELSSLRSEDTAV YYCSRYDYDYDVEGAMDYWGQGTTVTVSS |
| H5 | SEQ ID NO.377 | EVQLVQSGAEVKKPGASVKVSCKASGYTFTDYYMNWVRRAPGQRL EWMGIINPYNGGTSYNQKFRGRVTITVDKSASTAYMELSSLRSEDTAV YYCSRYDYDYDVEGAMDYWGQGTTVTVSS |

According to the Kabat numbering scheme, the analysis results for CDR sequences of heavy and light chain variable regions of the humanized antibodies above are shown in Table 34:

**Table 34. Kabat analysis results for CDR sequences of heavy and light chain variable regions of mab008 humanized antibodies**

| Heavy and light chains | CDR1 | CDR2 | CDR3 |
|---|---|---|---|
| Hab008L1/L3 | KASQNVGTAVA | SASIRYT | QQYSSSPYT |
| | SEQ ID NO. 95 | SEQ ID NO. 96 | SEQ ID NO. 97 |
| Hab008H2a | DYYMN | IINPYNSGTSYNQKFRG | YDYDYDVEGAMDY |
| | SEQ ID NO. 92 | SEQ ID NO. 378 | SEQ ID NO. 94 |
| Hab008H5 | DYYMN | IINPYNGGTSYNQKFRG | YDYDYDVEGAMDY |
| | SEQ ID NO. 92 | SEQ ID NO. 93 | SEQ ID NO. 94 |

### 6.6 Construction and expression purification of DLL3 humanized full-length antibodies

PCR primers were designed to construct VH/VL gene fragments for each humanized antibody, and then the gene fragments were homologously recombined with vectors to construct full-length expression vectors for the humanized antibodies. The humanized antibodies were expressed in a form that a VL-linker-VH sequence is linked to a human IgG1 Fc fragment. After plasmid construction, Expi293F cells were transiently transfected, and after 7 days, the cells were centrifuged to collect supernatants, and the antibodies were purified by the purification method described in section 1.1 of Example 1.

### Example 7. Identification of DLL3 Humanized Antibodies

### 7.1 Assay on binding of humanized antibodies to human DLL3 protein by ELISA

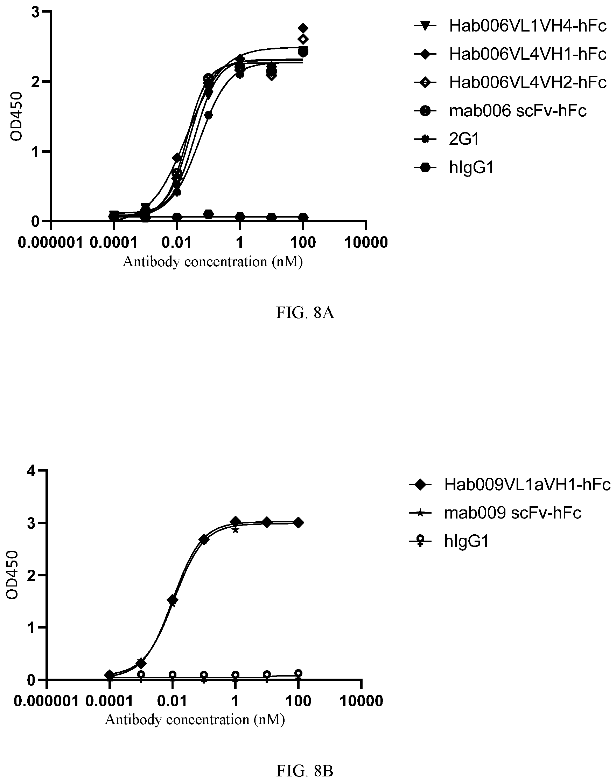

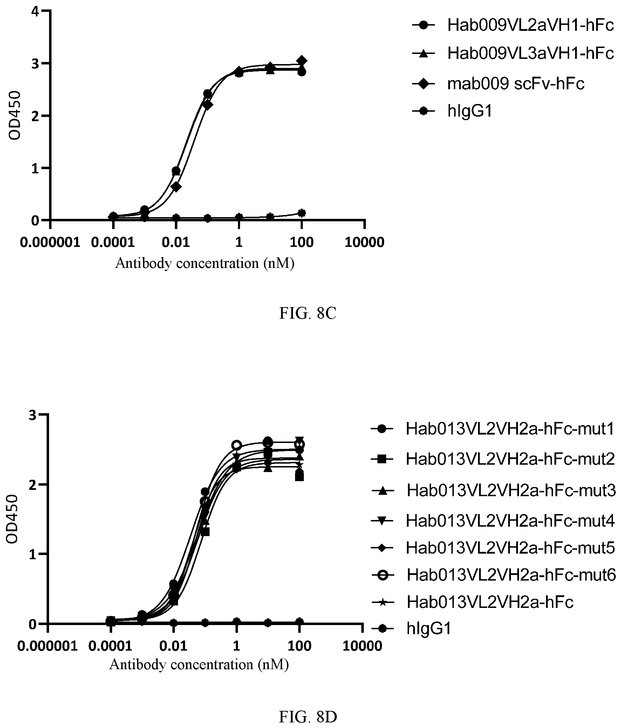

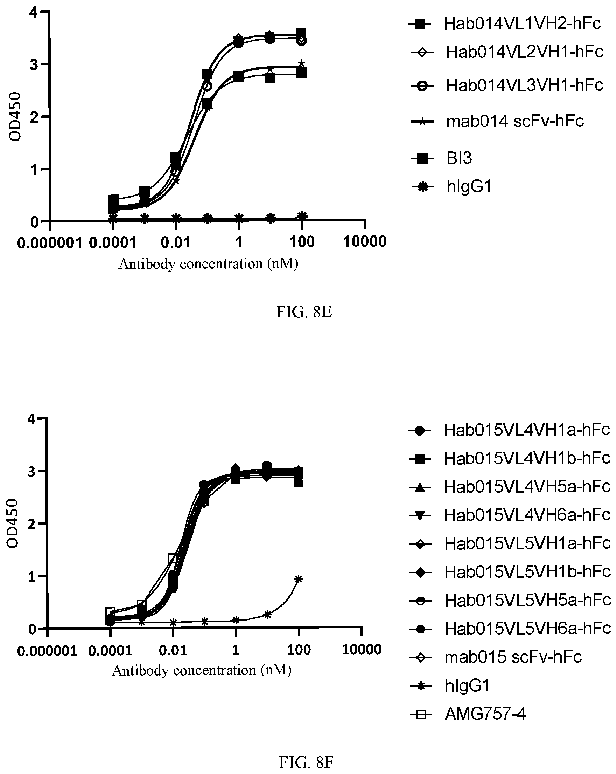

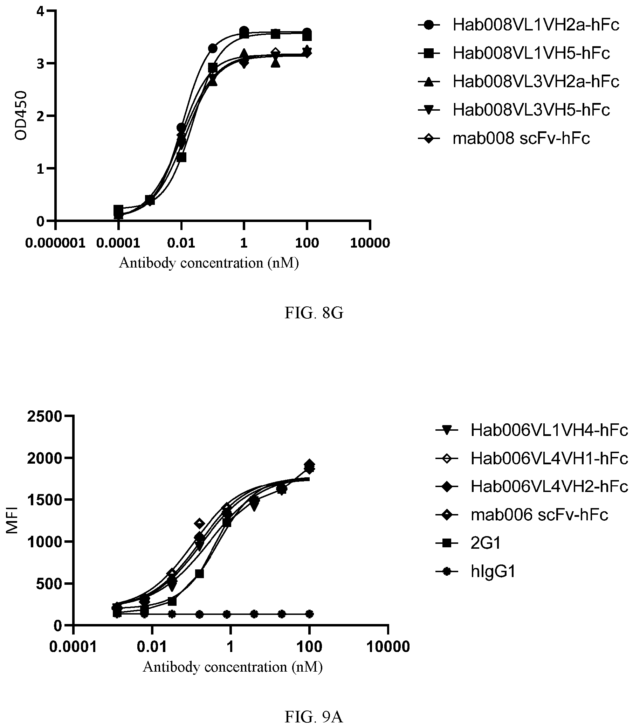

The humanized antibodies obtained above were subjected to ELISA assay and data analysis according to the method in section 1.3 of Example 1. An ELISA plate reader (Multimode Plate Reader, EnSight, purchased from Perkin Elmer) was used to read OD_{450 nm} values, and the results for the binding activity of the humanized antibodies for human DLL3 protein are shown in FIGs. 8A-8G. The results show that the humanized antibodies exhibit good binding activity for human DLL3 protein. The IgG control was hIgG1, and data in the table are OD_{450 nm} values.

### 7.2 Assay on binding of humanized antibodies to human DLL1 and DLL4 proteins by ELISA

The humanized antibodies obtained above were subjected to ELISA assay and data analysis according to the method in section 1.3 of Example 1. An ELISA plate reader (Multimode Plate Reader, EnSight, purchased from Perkin Elmer) was used to read OD_{450 nm} values, and the maximum values of the binding of the humanized antibodies to human DLL1 (purchased from Acro, Cat. No. DL1-H52H8) or DLL4 (purchased from Acro, Cat. No. DL4-H5227) protein at concentrations of 0.1, 1, 10, and 100 nM are shown in Table 35. The results show that the humanized antibodies barely bind to human DLL1 and DLL4.

**Table 35. ELISA assay results for binding reactions of humanized antibodies with human DLL1 and DLL4 proteins**

| Antibody | Emax of OD_{450 nm} binding value | |
|---|---|---|
| | hDLL1 | hDLL4 |
| Hab006VL1VH4-hFc | 0.07 | 0.15 |
| Hab006VL4VH1-hFc | 0.07 | 0.08 |
| Hab006VL4VH2-hFc | 0.05 | 0.06 |
| mab006 scFv-hFc | 0.05 | 0.08 |
| 2G1 | 0.08 | 0.06 |
| hIgG1 | 0.05 | 0.05 |
| Hab009VL1aVH1-hFc | 0.05 | 0.08 |
| mab009 scFv-hFc | 0.10 | 0.06 |
| hIgG1 | 0.04 | 0.06 |
| Hab009VL2aVH1-hFc | 0.07 | 0.06 |
| Hab009VL3aVH1-hFc | 0.06 | 0.06 |
| mab009 scFv-hFc | 0.07 | 0.06 |
| hIgG1 | 0.05 | 0.05 |
| Hab013VL2VH2a-hFc-mut1 | 0.06 | 0.08 |
| Hab013VL2VH2a-hFc-mut2 | 0.07 | 0.20 |
| Hab013VL2VH2a-hFc-mut3 | 0.08 | 0.19 |
| Hab013VL2VH2a-hFc-mut4 | 0.14 | 0.26 |
| Hab013VL2VH2a-hFc-mut5 | 0.11 | 0.15 |
| Hab013VL2VH2a-hFc-mut6 | 0.12 | 0.30 |
| Hab013VL2VH2a-hFc | 0.05 | 0.05 |
| hIgG1 | 0.12 | 0.21 |
| Hab014VL1VH2-hFc | 0.10 | 0.13 |
| Hab014VL2VH1-hFc | 0.17 | 0.25 |
| Hab014VL3VH1-hFc | 0.08 | 0.10 |
| mab014 scFv-hFc | 0.12 | 0.08 |
| BI3 | 0.14 | 0.06 |
| hIgG1 | 0.12 | 0.26 |
| Hab015VL4VH1a-hFc | 0.44 | 0.65 |
| Hab015VL4VH1b-hFc | 0.39 | 0.67 |
| Hab015VL4VH5a-hFc | 0.42 | 0.71 |
| Hab015VL4VH6a-hFc | 0.49 | 0.64 |
| Hab015VL5VH1a-hFc | 0.47 | 0.71 |
| Hab015VL5VH1b-hFc | 0.43 | 0.67 |
| Hab015VL5VH5a-hFc | 0.47 | 0.64 |
| Hab015VL5VH6a-hFc | 0.71 | 0.82 |
| mab015 scFv-hFc | 0.57 | 2.55 |
| AMG757-4 | 0.37 | 0.60 |
| hIgG1 | 0.81 | 0.99 |
| Hab008L1H2a-hFc | 0.06 | 0.07 |
| Hab008L1H5-hFc | 0.24 | 0.69 |
| Hab008L3H2a-hFc | 0.04 | 0.04 |
| Hab008L3H5-hFc | 0.11 | 0.18 |
| mab008 scFv-hFc | 0.05 | 0.05 |

### 7.3 Assay on binding activity of humanized antibodies for cells endogenously expressing DLL3 by FACS

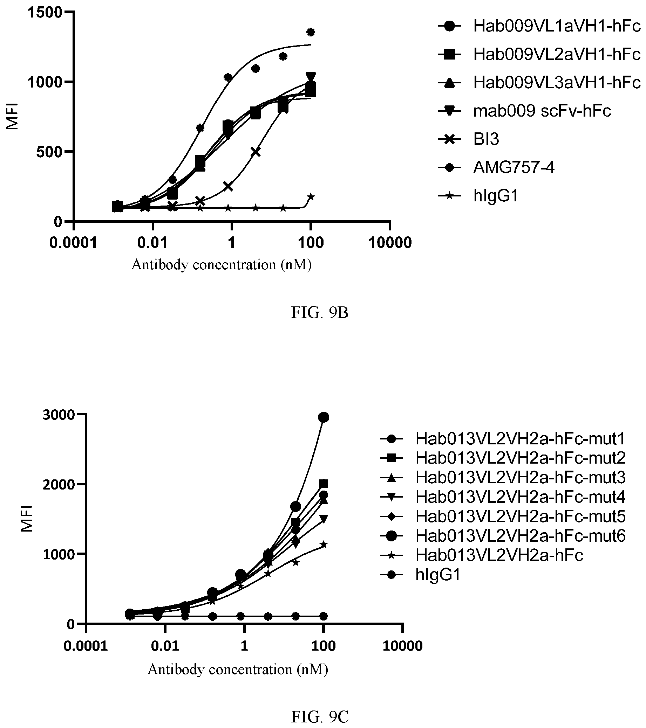

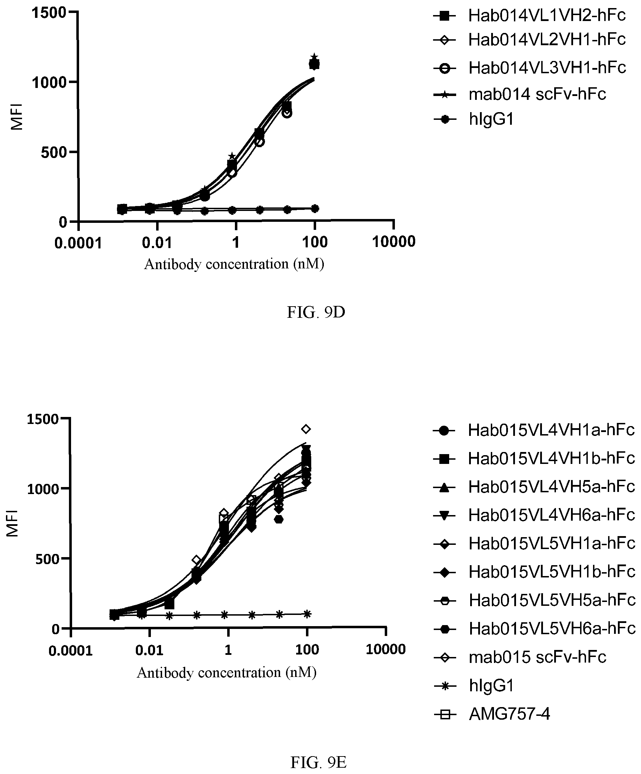

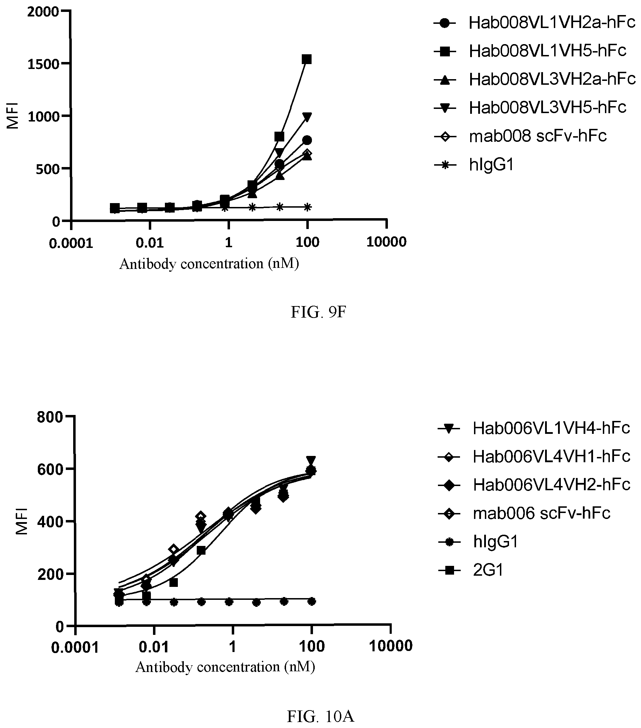

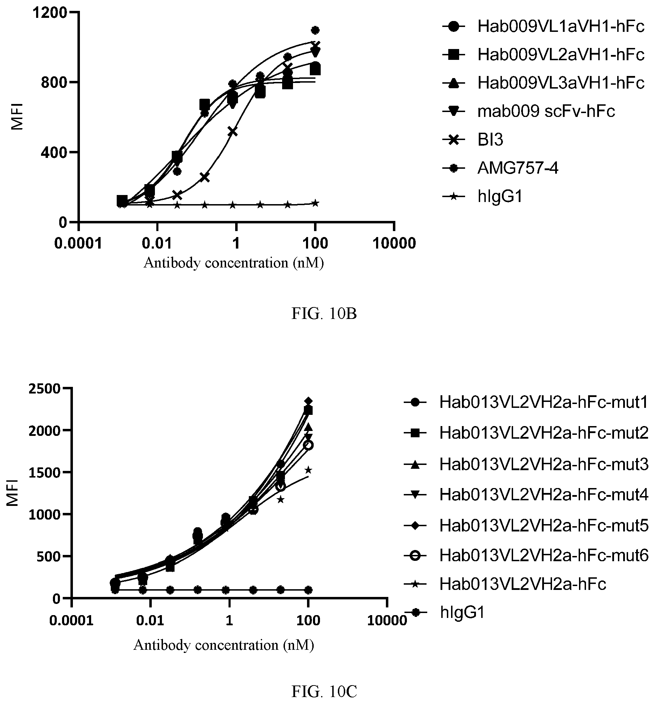

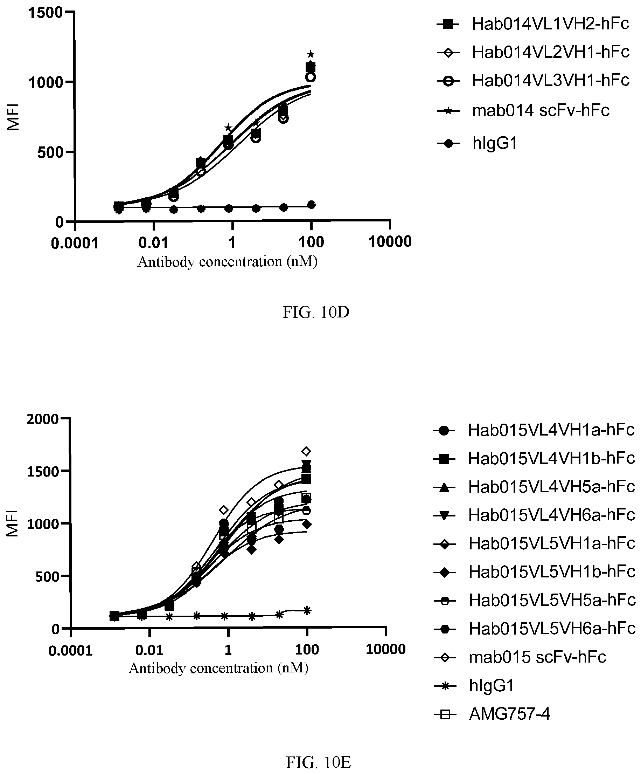

The humanized antibodies obtained above were subjected to FACS assay and data analysis according to the method in Example 4.3. Data analysis was performed by software (FlowJo) to obtain the mean fluorescence intensity (MFI) of the cells. Tables 36-37 and FIGs. 9A-10F show that the humanized antibodies substantially maintain the activity of binding to SHP77 cells and NCI-H82 cells as compared to the chimeric antibodies.

**Table 36. FACS assay results for binding activity of humanized antibodies for SHP77 cells**

| Antibody | Antibody concentration (nM) | | | | | | | |
|---|---|---|---|---|---|---|---|---|
| | 100 | 20 | 4 | 0.8 | 0.16 | 0.032 | 0.0064 | 0.00128 |
| Hab006VL1VH4-hFc | 1889 | 1605 | 1411 | 1309 | 938 | 458 | 275 | 208 |
| Hab006VL4VH1-hFc | 1900 | 1657 | 1492 | 1412 | 1065 | 554 | 305 | 215 |
| Hab006VL4VH2-hFc | 1872 | 1621 | 1497 | 1332 | 1047 | 528 | 281 | 199 |
| mab006 scFv-hFc | 1924 | 1638 | 1484 | 1399 | 1216 | 618 | 323 | 215 |
| 2G1 | 1887 | 1621 | 1491 | 1227 | 618 | 286 | 188 | 153 |
| hIgG1 | 138 | 137 | 136 | 134 | 133 | 137 | 138 | 139 |
| Hab009VL1aVH1-hFc | 969 | 855 | 783 | 692 | 430 | 205 | 129 | 107 |
| Hab009VL2aVH1-hFc | 929 | 825 | 784 | 683 | 432 | 201 | 128 | 108 |
| Hab009VL3aVH1-hFc | 954 | 852 | 769 | 673 | 396 | 196 | 128 | 105 |
| mab009 scFv-hFc | 1033 | 858 | 772 | 619 | 439 | 205 | 126 | 108 |
| BI3 | 967 | 807 | 498 | 252 | 148 | 110 | 105 | 101 |
| AMG757-4 | 1356 | 1182 | 1094 | 1032 | 668 | 299 | 160 | 121 |
| hIgG1 | 175 | 96 | 97 | 97 | 97 | 96 | 97 | 98 |
| Hab013VL2VH2a-hFc-mut1 | 1846 | 1347 | 971 | 703 | 459 | 245 | 162 | 135 |
| Hab013VL2VH2a-hFc-mut2 | 2006 | 1455 | 986 | 685 | 391 | 222 | 152 | 134 |
| Hab013VL2VH2a-hFc-mut3 | 1775 | 1219 | 897 | 685 | 464 | 255 | 166 | 140 |
| Hab013VL2VH2a-hFc-mut4 | 1497 | 1133 | 845 | 653 | 402 | 220 | 158 | 136 |
| Hab013VL2VH2a-hFc-mut5 | 2017 | 1421 | 1020 | 711 | 408 | 220 | 156 | 133 |
| Hab013VL2VH2a-hFc-mut6 | 2955 | 1678 | 979 | 708 | 447 | 243 | 168 | 142 |
| Hab013VL2VH2a-hFc | 1136 | 876 | 719 | 541 | 323 | 183 | 131 | 116 |
| hIgG1 | 109 | 108 | 106 | 108 | 107 | 109 | 107 | 110 |
| Hab014VL1VH2-hFc | 1119 | 818 | 628 | 403 | 192 | 116 | 94 | 90 |
| Hab014VL2VH1-hFc | 1110 | 796 | 610 | 407 | 207 | 120 | 99 | 91 |
| Hab014VL3VH1-hFc | 1122 | 770 | 567 | 348 | 179 | 112 | 96 | 91 |
| mab014 scFv-hFc | 1171 | 800 | 653 | 464 | 227 | 126 | 96 | 87 |
| hIgG1 | 88 | 79 | 79 | 78 | 73 | 77 | 81 | 79 |
| Hab015VL4VH1a-hFc | 1244 | 962 | 762 | 730 | 385 | 174 | 112 | 99 |
| Hab015VL4VH1b-hFc | 1186 | 962 | 829 | 720 | 371 | 169 | 113 | 97 |
| Hab015VL4VH5a-hFc | 1247 | 997 | 812 | 709 | 368 | 179 | 117 | 100 |
| Hab015VL4VH6a-hFc | 1267 | 943 | 821 | 692 | 361 | 173 | 114 | 101 |
| Hab015VL5VH1a-hFc | 1137 | 907 | 768 | 612 | 343 | 185 | 122 | 102 |
| Hab015VL5VH1b-hFc | 1033 | 844 | 714 | 632 | 356 | 178 | 118 | 102 |
| Hab015VL5VH5a-hFc | 1065 | 876 | 785 | 666 | 405 | 189 | 122 | 106 |
| Hab015VL5VH6a-hFc | 1090 | 770 | 726 | 663 | 362 | 171 | 117 | 101 |
| mab015 scFv-hFc | 1415 | 1067 | 917 | 819 | 485 | 184 | 112 | 87 |
| AMG757-4 | 1146 | 1004 | 911 | 773 | 390 | 175 | 117 | 100 |
| hIgG1 | 96 | 92 | 90 | 92 | 89 | 88 | 92 | 89 |
| Hab008L1H2a-hFc | 758 | 532 | 297 | 187 | 144 | 126 | 121 | 119 |
| Hab008L1H5-hFc | 1527 | 793 | 333 | 186 | 139 | 123 | 120 | 121 |
| Hab008L3H2a-hFc | 613 | 426 | 255 | 168 | 129 | 121 | 117 | 117 |
| Hab008L3H5-hFc | 974 | 634 | 330 | 194 | 133 | 122 | 117 | 117 |
| mab008 scFv-hFc | 632 | 495 | 293 | 183 | 136 | 121 | 114 | 114 |
| hIgG1 | 120 | 123 | 122 | 119 | 118 | 118 | 117 | 121 |

**Table 37. FACS assay results for binding activity of humanized antibodies for NCI-H82 cells**

| Antibody | Antibody concentration (nM) | | | | | | | |
|---|---|---|---|---|---|---|---|---|
| | 100 | 20 | 4 | 0.8 | 0.16 | 0.032 | 0.0064 | 0.00128 |
| Hab006VL1VH4-hFc | 626 | 519 | 291 | 412 | 367 | 242 | 155 | 126 |
| Hab006VL4VH1-hFc | 596 | 504 | 454 | 434 | 397 | 252 | 157 | 123 |
| Hab006VL4VH2-hFc | 588 | 494 | 445 | 429 | 385 | 250 | 151 | 117 |
| mab006 scFv-hFc | 586 | 488 | 457 | 422 | 416 | 292 | 177 | 123 |
| 2G1 | 588 | 500 | 472 | 422 | 286 | 165 | 114 | 100 |
| hIgG1 | 91 | 91 | 88 | 90 | 91 | 90 | 92 | 90 |
| Hab009VL1aVH1-hFc | 888 | 815 | 759 | 722 | 673 | 379 | 180 | 122 |
| Hab009VL2aVH1-hFc | 870 | 789 | 747 | 709 | 671 | 377 | 185 | 124 |
| Hab009VL3aVH1-hFc | 872 | 796 | 738 | 709 | 669 | 371 | 179 | 123 |
| mab009 scFv-hFc | 968 | 833 | 737 | 674 | 673 | 346 | 175 | 123 |
| BI3 | 1007 | 882 | 795 | 518 | 256 | 155 | 121 | 112 |
| AMG757-4 | 1097 | 944 | 836 | 789 | 621 | 290 | 160 | 120 |
| hIgG1 | 108 | 99 | 99 | 97 | 97 | 97 | 100 | 99 |
| Hab013VL2VH2a-hFc-mut1 | 2250 | 1599 | 1170 | 966 | 792 | 460 | 246 | 170 |
| Hab013VL2VH2a-hFc-mut2 | 2233 | 1463 | 1162 | 942 | 693 | 368 | 212 | 161 |
| Hab013VL2VH2a-hFc-mut3 | 2044 | 1429 | 1113 | 952 | 785 | 473 | 244 | 174 |
| Hab013VL2VH2a-hFc-mut4 | 1908 | 1356 | 1096 | 918 | 684 | 397 | 224 | 168 |
| Hab013VL2VH2a-hFc-mut5 | 2344 | 1602 | 1166 | 909 | 744 | 399 | 222 | 166 |
| Hab013VL2VH2a-hFc-mut6 | 1823 | 1333 | 1057 | 900 | 741 | 441 | 250 | 183 |
| Hab013VL2VH2a-hFc | 1525 | 1173 | 1032 | 831 | 670 | 356 | 185 | 130 |
| hIgG1 | 99 | 100 | 99 | 99 | 101 | 99 | 99 | 100 |
| Hab014VL1VH2-hFc | 1097 | 784 | 625 | 580 | 419 | 204 | 130 | 110 |
| Hab014VL2VH1-hFc | 1110 | 754 | 624 | 554 | 428 | 217 | 142 | 113 |
| Hab014VL3VH1-hFc | 1028 | 732 | 596 | 547 | 356 | 178 | 125 | 108 |
| mab014 scFv-hFc | 1190 | 829 | 702 | 667 | 429 | 213 | 134 | 112 |
| hIgG1 | 116 | 96 | 90 | 90 | 88 | 86 | 91 | 86 |
| Hab015VL4VH1a-hFc | 1518 | 1199 | 1055 | 993 | 489 | 213 | 137 | 118 |
| Hab015VL4VH1b-hFc | 1412 | 1128 | 1025 | 914 | 461 | 218 | 131 | 114 |
| Hab015VL4VH5a-hFc | 1511 | 1191 | 1031 | 942 | 476 | 212 | 135 | 118 |
| Hab015VL4VH6a-hFc | 1547 | 1177 | 1054 | 913 | 469 | 211 | 133 | 118 |
| Hab015VL5VH1a-hFc | 1217 | 1100 | 867 | 811 | 478 | 219 | 137 | 118 |
| Hab015VL5VH1b-hFc | 983 | 838 | 745 | 711 | 426 | 212 | 139 | 121 |
| Hab015VL5VH5a-hFc | 1111 | 929 | 848 | 779 | 473 | 230 | 139 | 122 |
| Hab015VL5VH6a-hFc | 1218 | 937 | 827 | 755 | 430 | 216 | 127 | 117 |
| mab015 scFv-hFc | 1676 | 1357 | 1194 | 1121 | 588 | 218 | 135 | 113 |
| AMG757-4 | 1230 | 1035 | 942 | 883 | 527 | 218 | 139 | 123 |
| hIgG1 | 160 | 121 | 108 | 110 | 113 | 108 | 108 | 107 |
| Hab008L1H2a-hFc | 617 | 503 | 440 | 312 | 194 | 134 | 106 | 93 |
| Hab008L1H5-hFc | 1864 | 1016 | 539 | 245 | 149 | 114 | 106 | 102 |
| Hab008L3H2a-hFc | 550 | 448 | 307 | 135 | 97 | 90 | 89 | 85 |
| Hab008L3H5-hFc | 1345 | 674 | 388 | 152 | 102 | 91 | 90 | 99 |
| mab008 scFv-hFc | 537 | 438 | 373 | 176 | 107 | 95 | 94 | 113 |
| hIgG1 | 96 | 92 | 92 | 87 | 90 | 92 | 90 | 91 |

### 7.4 Assay on binding activity of humanized antibodies for cells over-expressing DLL3 by FACS

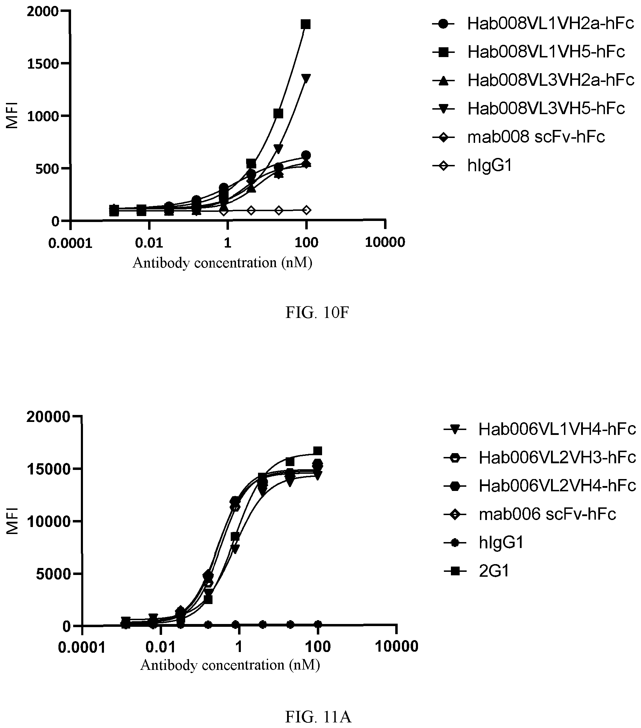

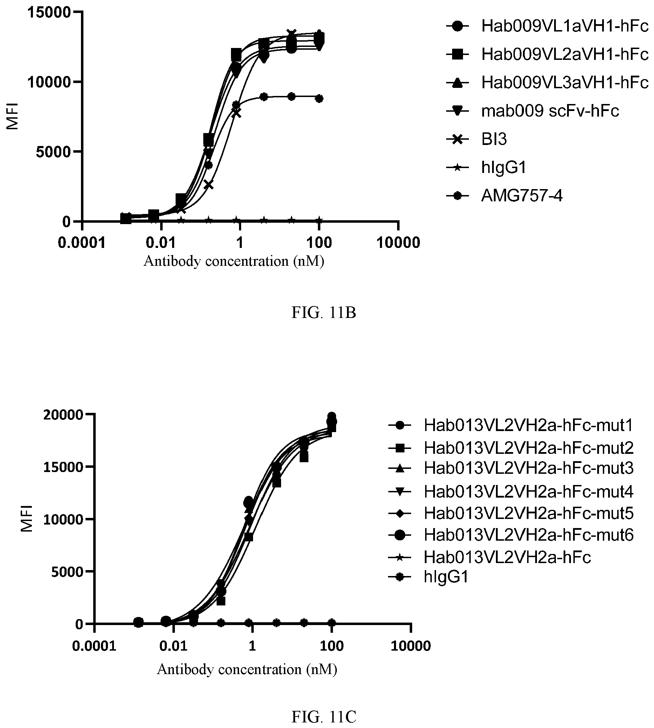

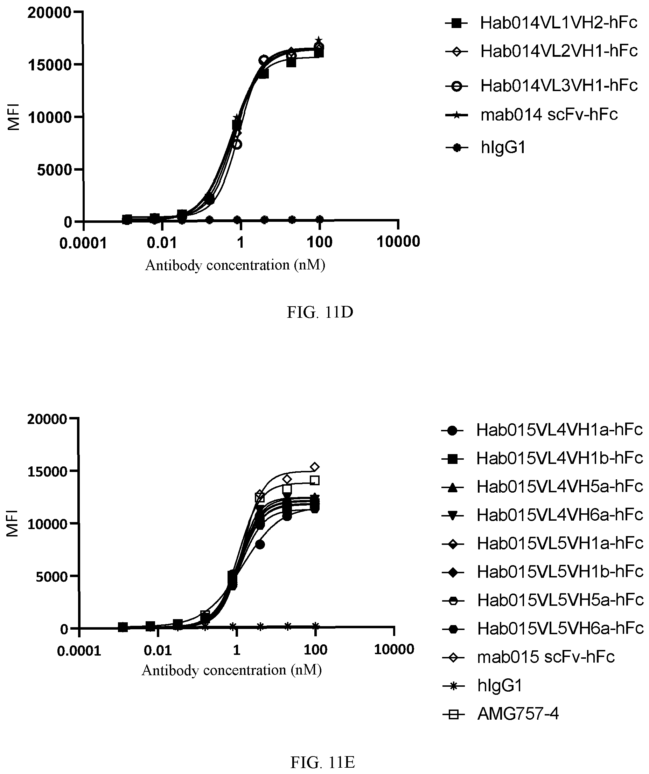

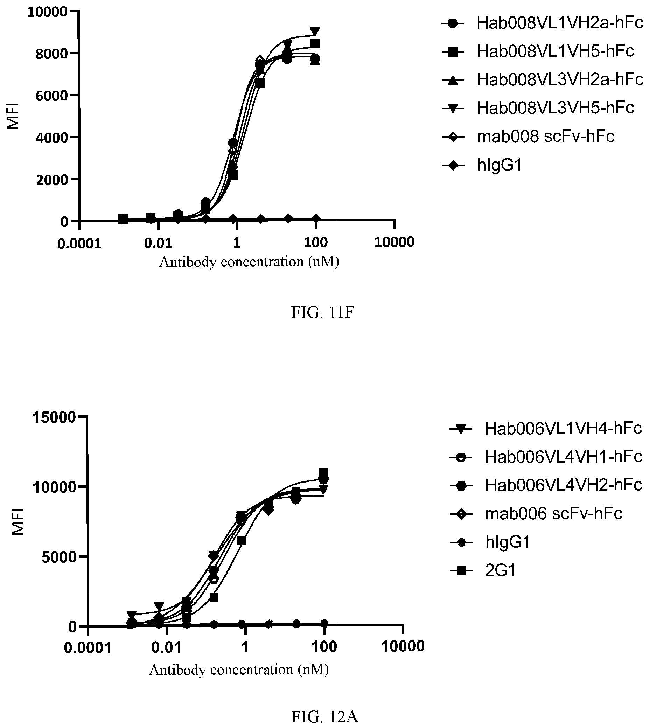

The humanized antibodies obtained above were subjected to FACS assay and data analysis according to the method in Example 4.3. Data analysis was performed by software (FlowJo) to obtain the mean fluorescence intensity (MFI) of the cells. Table 38 and FIGs. 11A-11F show that the humanized antibodies all can bind to CHO-K1 hDLL3 cells.

**Table 38. FACS assay results for binding activity of humanized antibodies for CHO-K1 hDLL3 cells**

| Antibody | Antibody concentration (nM) | | | | | | | |
|---|---|---|---|---|---|---|---|---|
| | 100 | 20 | 4 | 0.8 | 0.16 | 0.032 | 0.0064 | 0.00128 |
| Hab006VL1VH4-hFc | 14277 | 13645 | 12728 | 7266 | 3095 | 1052 | 716 | 419 |
| Hab006VL4VH1-hFc | 15171 | 14516 | 13601 | 11309 | 4116 | 1128 | 411 | 227 |
| Hab006VL4VH2-hFc | 15434 | 14542 | 13783 | 11890 | 4703 | 1340 | 458 | 243 |
| mab006 scFv-hFc | 15215 | 14227 | 13373 | 11809 | 4866 | 1424 | 503 | 251 |
| 2G1 | 16648 | 15605 | 14134 | 8513 | 2512 | 727 | 283 | 159 |
| hIgG1 | 103 | 99 | 99 | 99 | 100 | 100 | 91 | 98 |
| Hab009VL1aVH1-hFc | 12893 | 12391 | 11933 | 11031 | 5836 | 1535 | 452 | 199 |
| Hab009VL2aVH1-hFc | 13126 | 12636 | 12745 | 12027 | 5926 | 1614 | 476 | 210 |
| Hab009VL3aVH1-hFc | 13395 | 13299 | 12788 | 11815 | 5727 | 1499 | 458 | 204 |
| mab009 scFv-hFc | 12528 | 12428 | 11664 | 10599 | 4831 | 1239 | 383 | 194 |
| BI3 | 13213 | 13423 | 12556 | 7768 | 2658 | 924 | 452 | 310 |
| AMG757-4 | 8790 | 8939 | 8931 | 8359 | 4048 | 1204 | 420 | 217 |
| hIgG1 | 95 | 93 | 94 | 93 | 93 | 94 | 94 | 96 |
| Hab013VL2VH2a-hFc-mut1 | 19824 | 16863 | 14203 | 11797 | 3858 | 933 | 306 | 159 |
| Hab013VL2VH2a-hFc-mut2 | 18714 | 15835 | 13447 | 8270 | 2181 | 573 | 218 | 133 |
| Hab013VL2VH2a-hFc-mut3 | 18897 | 16592 | 14477 | 10988 | 3147 | 745 | 253 | 146 |
| Hab013VL2VH2a-hFc-mut4 | 19256 | 16718 | 14318 | 9702 | 2618 | 683 | 243 | 147 |
| Hab013VL2VH2a-hFc-mut5 | 19120 | 16379 | 13933 | 10105 | 3050 | 745 | 265 | 151 |
| Hab013VL2VH2a-hFc-mut6 | 19305 | 17374 | 14975 | 11527 | 3106 | 766 | 261 | 147 |
| Hab013VL2VH2a-hFc | 19484 | 16349 | 14871 | 11187 | 3189 | 789 | 270 | 164 |
| hIgG1 | 96 | 103 | 105 | 94 | 95 | 94 | 95 | 96 |
| Hab014VL1VH2-hFc | 16044 | 15088 | 14032 | 9186 | 2134 | 698 | 306 | 221 |
| Hab014VL2VH1-hFc | 16351 | 16113 | 15426 | 8416 | 2384 | 705 | 322 | 231 |
| Hab014VL3VH1-hFc | 16537 | 15724 | 15319 | 7322 | 2059 | 555 | 299 | 207 |
| mab014 scFv-hFc | 17205 | 15463 | 14171 | 9867 | 2458 | 698 | 328 | 238 |
| hIgG1 | 131 | 109 | 123 | 121 | 118 | 116 | 120 | 120 |
| Hab015VL4VH1a-hFc | 11427 | 10582 | 7922 | 5067 | 941 | 294 | 152 | 122 |
| Hab015VL4VH1b-hFc | 11826 | 11311 | 10266 | 4642 | 937 | 302 | 152 | 120 |
| Hab015VL4VH5a-hFc | 12389 | 12000 | 10882 | 4391 | 870 | 283 | 150 | 122 |
| Hab015VL4VH6a-hFc | 12163 | 12399 | 11205 | 4367 | 861 | 265 | 144 | 120 |
| Hab015VL5VH1a-hFc | 11981 | 11762 | 10624 | 4308 | 936 | 330 | 160 | 129 |
| Hab015VL5VH1b-hFc | 11763 | 11545 | 10301 | 4104 | 980 | 341 | 174 | 137 |
| Hab015VL5VH5a-hFc | 11972 | 11956 | 10720 | 4636 | 1026 | 339 | 166 | 134 |
| Hab015VL5VH6a-hFc | 11266 | 10902 | 9716 | 3958 | 883 | 294 | 153 | 122 |
| mab015 scFv-hFc | 15283 | 14102 | 12704 | 4127 | 757 | 237 | 121 | 107 |
| AMG757-4 | 13975 | 13143 | 12369 | 4962 | 1199 | 368 | 181 | 130 |
| hIgG1 | 104 | 101 | 101 | 100 | 100 | 98 | 101 | 101 |
| Hab008L1H2a-hFc | 7687 | 7663 | 7440 | 3695 | 866 | 316 | 148 | 109 |
| Hab008L1H5-hFc | 8405 | 7753 | 6528 | 2167 | 521 | 221 | 127 | 103 |
| Hab008L3H2a-hFc | 7595 | 8202 | 7186 | 2703 | 715 | 240 | 126 | 98 |
| Hab008L3H5-hFc | 8953 | 8324 | 7139 | 2366 | 671 | 235 | 131 | 100 |
| mab008 scFv-hFc | 7634 | 7730 | 7631 | 3311 | 770 | 276 | 150 | 110 |
| hIgG1 | 78 | 75 | 81 | 78 | 82 | 81 | 82 | 82 |

### Example 8: Assay on Cross-Binding Activity of DLL3 Humanized Antibodies

### (A) Assay on binding activity of humanized antibodies for monkey DLL3 by FACS

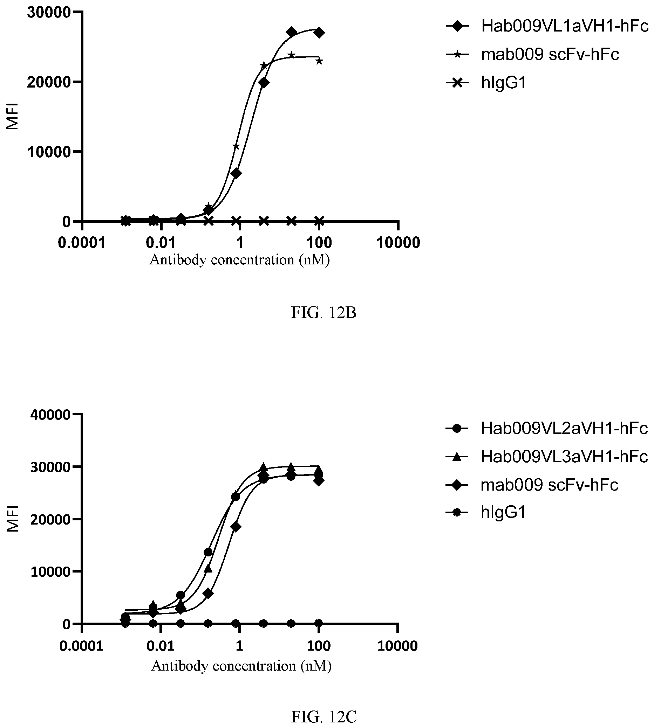

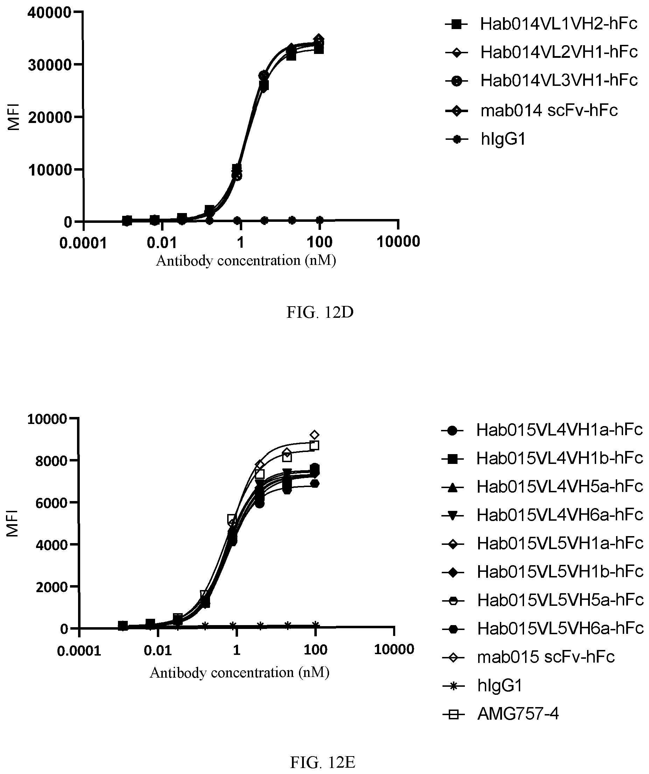

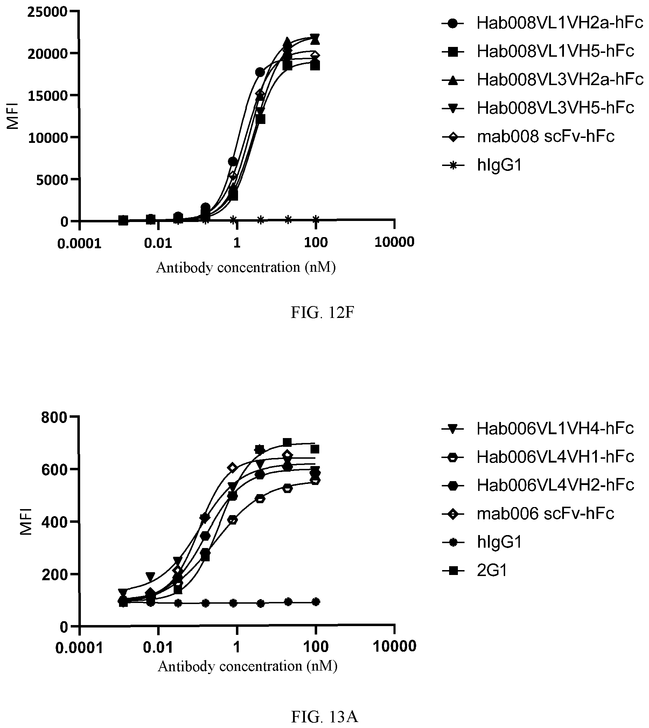

The humanized antibodies obtained above were subjected to FACS assay and data analysis according to the method in Example 4.3. FACS results for the binding of the humanized antibodies to CRO K1-cyno DLL3 cells, a cell line over-expressing monkey DLL3, are shown in Table 39 and FIGs. 12A-12F, and the results show that the humanized antibodies substantially maintain the activity of binding to CRO K1-cyno DLL3 cells as compared to the chimeric antibodies. The IgG control was hIgG1, and the data in the Table are mean fluorescence intensity MFI values.

**Table 39. FACS assay results for binding reactions of humanized antibodies with CHO-K1-cyno DLL3 cells**

| Antibody | Antibody concentration (nM) | | | | | | | |
|---|---|---|---|---|---|---|---|---|
| | 100 | 20 | 4 | 0.8 | 0.16 | 0.032 | 0.0064 | 0.00128 |
| Hab006VL1VH4-hFc | 9719 | 9055 | 8593 | 7836 | 5005 | 1727 | 1377 | 750 |
| Hab006VL4VH 1-hFc | 10453 | 9049 | 8445 | 7497 | 3353 | 1107 | 424 | 239 |
| Hab006VL4VH2-hFc | 10545 | 9185 | 8508 | 7814 | 3966 | 1338 | 494 | 276 |
| mab006 scFv-hFc | 10499 | 9059 | 8275 | 7748 | 4995 | 1675 | 613 | 305 |
| 2G1 | 10947 | 9605 | 8773 | 6089 | 2055 | 642 | 267 | 166 |
| hIgG 1 | 122 | 120 | 120 | 122 | 119 | 118 | 119 | 120 |
| Hab009VL1aVH1-hFc | 27034 | 27113 | 19880 | 6885 | 1641 | 423 | 178 | 120 |
| mab009 scFv-hFc | 22953 | 23811 | 22357 | 10811 | 2152 | 573 | 214 | 125 |
| hIgG1 | 81 | 81 | 78 | 79 | 81 | 79 | 79 | 81 |
| Hab009VL2aVH1-hFc | 28416 | 28152 | 27604 | 24289 | 13692 | 5454 | 3163 | 1338 |
| Hab009VL3aVH1-hFc | 29555 | 30031 | 30012 | 24708 | 10646 | 4022 | 3763 | 1451 |
| mab009 scFv-hFc | 27353 | 28514 | 28366 | 18537 | 5800 | 2888 | 2181 | 787 |
| hIgG1 | 112 | 95 | 90 | 86 | 85 | 88 | 90 | 88 |
| Hab014VL1VH2-hFc | 32760 | 31452 | 25890 | 9963 | 2189 | 692 | 306 | 215 |
| Hab014VL2VH1-hFc | 32994 | 32973 | 25278 | 10116 | 1669 | 661 | 287 | 200 |
| Hab014VL3VH1-hFc | 33920 | 32495 | 27708 | 8681 | 1729 | 565 | 245 | 180 |
| mab014 scFv-hFc | 34649 | 32188 | 27702 | 9545 | 1495 | 498 | 243 | 184 |
| hIgG1 | 131 | 110 | 105 | 104 | 103 | 99 | 100 | 99 |
| Hab015VL4VH1a-hFc | 7592 | 6724 | 5909 | 4467 | 1244 | 399 | 168 | 123 |
| Hab015VL4VH1b-hFc | 7515 | 6825 | 6274 | 4291 | 1195 | 389 | 163 | 117 |
| Hab015VL4VH5a-hFc | 7608 | 7182 | 6520 | 4484 | 1182 | 377 | 164 | 122 |
| Hab015VL4VH6a-hFc | 7481 | 7333 | 6817 | 4259 | 1153 | 352 | 153 | 118 |
| Hab015VL5VH1a-hFc | 7380 | 7041 | 6660 | 4339 | 1191 | 415 | 178 | 131 |
| Hab015VL5VH1b-hFc | 7309 | 6972 | 6505 | 4139 | 1135 | 410 | 195 | 140 |
| Hab015VL5VH5a-hFc | 7381 | 7089 | 6607 | 4588 | 1331 | 428 | 194 | 142 |
| Hab015VL5VH6a-hFc | 6847 | 6527 | 6080 | 4079 | 1147 | 371 | 169 | 125 |
| mab015 scFv-hFc | 9162 | 8329 | 7770 | 4940 | 1173 | 317 | 129 | 95 |
| AMG757-4 | 8658 | 8096 | 7311 | 5171 | 1564 | 469 | 207 | 129 |
| hIgG1 | 95 | 86 | 86 | 85 | 85 | 86 | 85 | 85 |
| Hab008L1H2a-hFc | 18641 | 19504 | 17633 | 7012 | 1545 | 511 | 231 | 129 |
| Hab008L1H5-hFc | 18398 | 18402 | 12082 | 2941 | 711 | 286 | 159 | 116 |
| Hab008L3H2a-hFc | 21436 | 21254 | 14829 | 3976 | 928 | 329 | 148 | 103 |
| Hab008L3H5-hFc | 21590 | 20271 | 12905 | 3466 | 724 | 252 | 142 | 103 |
| mab008 scFv-hFc | 19594 | 20181 | 15104 | 5343 | 879 | 309 | 169 | 113 |
| hIgG1 | 81 | 81 | 78 | 79 | 81 | 79 | 79 | 81 |

### (B) Assay on binding activity of humanized antibodies for mouse DLL3 by FACS

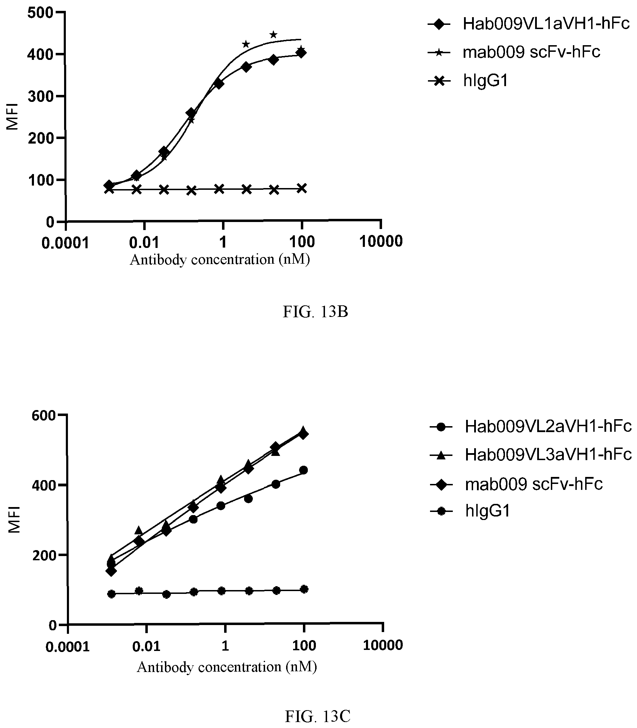

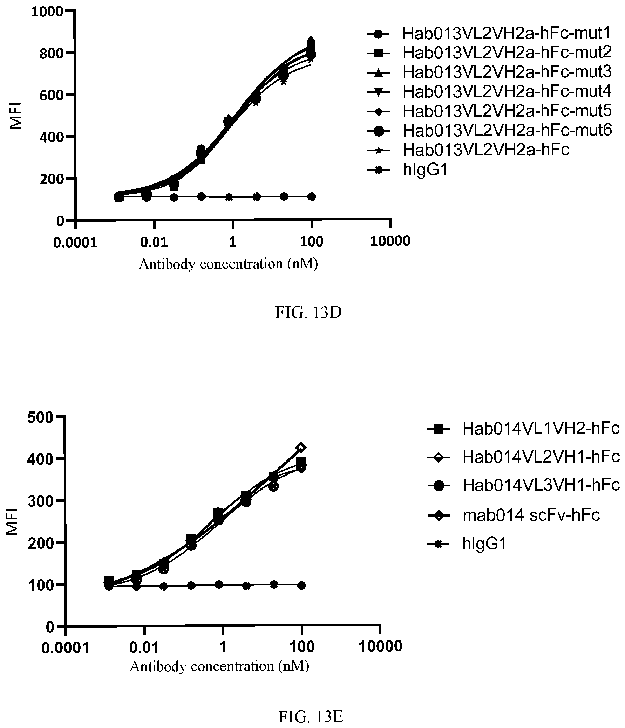

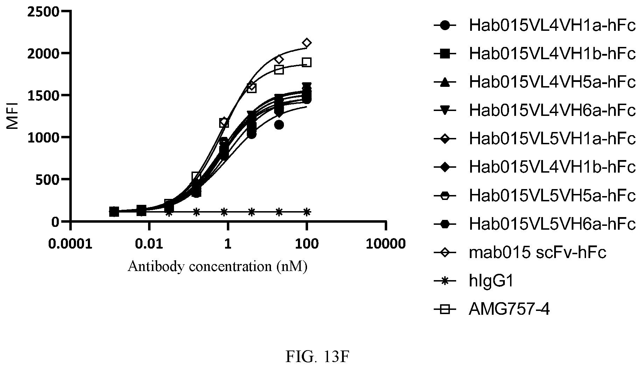

The humanized antibodies obtained above were subjected to FACS assay and data analysis according to the method in Example 4.3. FACS results for the binding of the humanized antibodies to CHO K1-mDLL3 cells, a cell line over-expressing mouse DLL3, are shown in Table 40 and FIGs. 13A-13F, and the results show that the humanized antibodies substantially maintained the activity of binding to CRO K1-mDLL3 cells as compared to the chimeric antibodies and have mouse cross reactivity. The Hab008 humanized molecule has no mouse cross reactivity. The IgG control was hIgG1, and the data in the Table are mean fluorescence intensity MFI values.

**Table 40. FACS assay results for binding reactions of humanized antibodies with CHO-K1-mDLL3 cells**

| Antibody | Antibody concentration (nM) | | | | | | | |
|---|---|---|---|---|---|---|---|---|
| | 100 | 20 | 4 | 0.8 | 0.16 | 0.032 | 0.0064 | 0.00128 |
| Hab006VL1VH4-hFc | 589 | 627 | 615 | 529 | 406 | 246 | 187 | 126 |
| Hab006VL4VH1-hFc | 554 | 523 | 485 | 404 | 278 | 165 | 110 | 98 |
| Hab006VL4VH2-hFc | 580 | 605 | 577 | 495 | 343 | 185 | 118 | 99 |
| mab006 scFv-hFc | 584 | 650 | 670 | 603 | 412 | 212 | 126 | 94 |
| 2G1 | 673 | 698 | 672 | 497 | 263 | 138 | 100 | 92 |
| hIgG1 | 90 | 90 | 85 | 87 | 86 | 88 | 90 | 91 |
| Hab009VL1aVH1-hFc | 400 | 383 | 367 | 327 | 258 | 166 | 109 | 86 |
| mab009 scFv-hFc | 408 | 443 | 421 | 330 | 241 | 153 | 104 | 86 |
| hIgG1 | 77 | 73 | 75 | 76 | 73 | 75 | 76 | 78 |
| Hab009VL2aVH1-hFc | 438 | 398 | 358 | 338 | 300 | 272 | 238 | 172 |
| Hab009VL3aVH1-hFc | 553 | 491 | 458 | 413 | 345 | 287 | 269 | 190 |
| mab009 scFv-hFc | 541 | 504 | 443 | 389 | 334 | 267 | 238 | 153 |
| hIgG1 | 98 | 94 | 94 | 94 | 92 | 85 | 95 | 87 |
| Hab013VL2VH2a-hFc-mut1 | 844 | 723 | 590 | 464 | 339 | 194 | 129 | 112 |
| Hab013VL2VH2a-hFc-mut2 | 811 | 715 | 590 | 467 | 289 | 159 | 118 | 110 |
| Hab013VL2VH2a-hFc-mut3 | 844 | 715 | 605 | 486 | 328 | 183 | 127 | 114 |
| Hab013VL2VH2a-hFc-mut4 | 811 | 698 | 580 | 460 | 302 | 166 | 122 | 112 |
| Hab013VL2VH2a-hFc-mut5 | 854 | 712 | 599 | 480 | 320 | 183 | 122 | 110 |
| Hab013VL2VH2a-hFc-mut6 | 788 | 686 | 577 | 467 | 319 | 172 | 123 | 111 |
| Hab013VL2VH2a-hFc | 764 | 655 | 557 | 463 | 313 | 179 | 125 | 113 |
| hIgG1 | 108 | 109 | 108 | 108 | 110 | 108 | 108 | 109 |
| Hab014VL1VH2-hFc | 389 | 355 | 311 | 268 | 208 | 148 | 122 | 109 |
| Hab014VL2VH1-hFc | 374 | 355 | 308 | 272 | 204 | 152 | 118 | 109 |
| Hab014VL3VH1-hFc | 382 | 332 | 296 | 253 | 192 | 137 | 110 | 103 |
| mab014 scFv-hFc | 424 | 354 | 300 | 260 | 205 | 152 | 117 | 105 |
| hIgG1 | 95 | 98 | 95 | 99 | 96 | 95 | 95 | 96 |
| Hab015VL4VH1a-hFc | 1450 | 1147 | 1036 | 779 | 339 | 172 | 123 | 117 |
| Hab015VL4VH1b-hFc | 1488 | 1344 | 1117 | 813 | 350 | 172 | 123 | 114 |
| Hab015VL4VH5a-hFc | 1529 | 1418 | 1209 | 875 | 350 | 170 | 126 | 117 |
| Hab015VL4VH6a-hFc | 1592 | 1455 | 1257 | 884 | 361 | 170 | 130 | 118 |
| Hab015VL5VH1a-hFc | 1549 | 1290 | 1200 | 922 | 393 | 195 | 131 | 121 |
| Hab015VL5VH1b-hFc | 1456 | 1347 | 1159 | 884 | 361 | 186 | 131 | 122 |
| Hab015VL5VH5a-hFc | 1581 | 1443 | 1236 | 953 | 424 | 199 | 139 | 122 |
| Hab015VL5VH6a-hFc | 1533 | 1409 | 1226 | 870 | 390 | 185 | 131 | 121 |
| mab015 scFv-hFc | 2123 | 1927 | 1613 | 1187 | 458 | 181 | 120 | 113 |
| AMG757-4 | 1890 | 1801 | 1583 | 1167 | 532 | 212 | 138 | 120 |
| hIgG1 | 114 | 114 | 113 | 113 | 113 | 110 | 113 | 113 |

### Example 9: Assay on Affinity of DLL3 Humanized Antibodies

In this experiment, a Biacore 8K (GE) instrument was used to determine the affinity of the anti-DLL3 humanized antibodies to be tested for DLL3 antigen protein using multi-cycle dynamics. In this experiment, the running buffer was 1× HBS-EP+ buffer (10 mM HEPES, 150 mM NaCl, 3 mM EDTA, 0.05% surfactant P20, Cat. BR-1006-69, Cytiva). The flow cell temperature was set at 25 °C, and the sample chamber temperature was set at 16 °C. Both were pretreated with the running buffer. A Protein A biosensor chip (Cat. No. 29-1275-56, Cytiva) was used to affinity capture a certain amount of the antibody to be tested, and then a certain concentration of DLL3 antigen was allowed to flow over the surface of the chip. The reaction signals were detected in real time using a Biacore 8K instrument (GE) to obtain association and dissociation curves. After each cycle of dissociation was completed, the antigen-antibody complex was washed thoroughly and regenerated with a pH 1.5 glycine-hydrochloric acid regeneration solution (Cat. BR-1003-54, Cytiva). The association was measured by injection of DLL3 antigen in various concentrations in solution for 180 s at a flow rate of 30 µL/min; the antigen was 1:1 diluted from 20 nM (see detailed results for actual concentrations tested) and a series of concentration gradients were set. The dissociation time was up to 600 s, and finally the regeneration of the chip surface was completed by washing with a 10 mM glycine-hydrochloric acid solution (pH 1.5) at a flow rate of 30 µL/min for 30 s.

The data obtained from the experiment were fitted with the (1:1) Langmuir model using GE Biacore 8K Evaluation version 2.0 software to give the association rate (Ka), dissociation rate (Kd), and affinity value (KD) of the humanized antibodies for human DLL3 protein, as shown in Tables 41. The results show that the humanized antibodies bind well to human DLL3 protein.

**Table 41. Binding affinity of humanized antibodies for human DLL3 protein**

| Antibody | Ka (1/Ms) | Kd (1/s) | KD (M) |
|---|---|---|---|
| Hab006VL1VL4-hFc | 3.89E+05 | 2.21E-04 | 5.68E-10 |
| Hab006VL4VL1-hFc | 4.15E+05 | 1.19E-04 | 2.86E-10 |
| Hab006VL4VL2-hFc | 3.94E+05 | 1.56E-04 | 3.95E-10 |
| mab006 scFv-hFc | 1.62E+05 | 2.03E-04 | 1.25E-09 |
| Hab009VL1aVH1-hFc | 5.96E+06 | 1.94E-03 | 3.25E-10 |
| Hab009VL2aVH1-hFc | 5.79E+06 | 1.90E-03 | 3.28E-10 |
| Hab009VL3aVH1-hFc | 5.76E+06 | 1.75E-03 | 3.04E-10 |
| mab009 scFv-hFc | 6.13E+06 | 1.32E-03 | 2.16E-10 |
| AMG757-4 | 1.29E+06 | 9.35E-05 | 7.25E-11 |
| BI3 | 9.45E+05 | 4.70E-05 | 4.97E-11 |
| Hab013VL2VH2a-hFc-mut4 | 3.01E+06 | 3.55E-04 | 1.18E-10 |
| Hab013VL2VH2a-hFc-mut6 | 3.09E+06 | 2.63E-04 | 8.51E-11 |
| Hab013VL2VH2a-hFc | 3.32E+06 | 3.71E-04 | 1.12E-10 |
| mab013 scFv -hFc | 3.11E+06 | 8.20E-04 | 2.64E-10 |
| Hab014VL1VH2-hFc | 1.02E+06 | 7.84E-05 | 7.72E-11 |
| Hab014VL2VH1-hFc | 1.21E+06 | 1.35E-04 | 1.11E-10 |
| Hab014VL3VH1-hFc | 1.36E+06 | 1.29E-04 | 9.46E-11 |
| mab014 scFv-hFc | 8.48E+05 | 4.64E-05 | 5.47E-11 |
| Hab015VL4VH1b-hFc | 1.80E+06 | 3.49E-05 | 1.94E-11 |
| Hab015VL4VH6a-hFc | 1.75E+06 | 2.03E-07 | 1.16E-13 |
| mab015 scFv-hFc | 2.45E+06 | 1.46E-06 | 5.97E-13 |
| Hab015VL4VH6b-hFc | 1.91E+06 | 2.50E-05 | 1.31E-11 |
| AMG757-4 | 1.41E+06 | 7.51E-05 | 5.32E-11 |
| Hab008L1H2a-hFc | 6.06E+05 | 3.24E-04 | 5.35E-10 |
| Hab008L3H2a-hFc | 7.66E+05 | 4.05E-04 | 5.29E-10 |
| Hab008L3H5-hFc | 3.45E+05 | 3.12E-04 | 9.02E-10 |
| mab008 scFv-hFc | 8.91E+05 | 3.40E-04 | 3.81E-10 |

## Claims

1. An antibody or an antigen-binding fragment thereof specifically binding to human delta-like ligand 3 (DLL3), comprising a light chain variable region (VL) and a heavy chain variable region (VH), wherein
(1) the light chain variable region comprises an LCDR1, an LCDR2, and an LCDR3, and the LCDR1, the LCDR2, and the LCDR3 are an LCDR1, an LCDR2, and an LCDR3 of a VL domain set forth in any one of SEQ ID NOs:19, 21, 23, 25, 27, 29, 31, 33, 35, 37, 39, 41, 43, 45, 47, 49, 338-339, 343-346, 349-355, 359-361, 364-365, and 374-375; and
(2) the heavy chain variable region comprises an HCDR1, an HCDR2, and an HCDR3, and the HCDR1, the HCDR2, and the HCDR3 are an HCDR1, an HCDR2, and an HCDR3 of a VH domain set forth in any one of SEQ ID NOs:18, 20, 22, 24, 26, 28, 30, 32, 34, 36, 38, 40, 42, 44, 46, 48, 340-342, 347, 356, 362-363, 366-370, and 376-377.

2. The antibody or the antigen-binding fragment thereof according to claim 1, wherein the LCDR1, the LCDR2, and the LCDR3 are determined according to the Kabat numbering scheme, the IMGT numbering scheme, and/or the Chothia numbering scheme.

3. The antibody or the antigen-binding fragment thereof according to claim 1 or 2, wherein the HCDR1, the HCDR2, and the HCDR3 are determined according to the Kabat numbering scheme, the IMGT numbering scheme, and/or the Chothia numbering scheme.

4. The antibody or the antigen-binding fragment thereof according to any one of claims 1 to 3, comprising the following LCDR1, LCDR2, LCDR3, HCDR1, HCDR2, and HCDR3:
(1) according to the Kabat numbering scheme, LCDR1-3 and HCDR1-3 have the amino acid sequences set forth in SEQ ID NOs: 50-145, 348, 357-358, 371-373, and 378 or a sequence combination having 1, 2, 3, or more amino acid insertions, deletions, and/or substitutions compared with the amino acid sequences set forth in SEQ ID NOs: 50-145;
(2) according to the IMGT numbering scheme, LCDR1-3 and HCDR1-3 have the amino acid sequences set forth in SEQ ID NOs: 146-241 or a sequence combination having 1, 2, 3, or more amino acid insertions, deletions, and/or substitutions compared with the amino acid sequences set forth in SEQ ID NOs: 146-241;
(3) according to the Chothia numbering scheme, LCDR1-3 and HCDR1-3 have the amino acid sequences set forth in SEQ ID NOs: 242-337 or a sequence combination having 1, 2, 3, or more amino acid insertions, deletions, and/or substitutions compared with the amino acid sequences set forth in SEQ ID NOs: 242-337.

5. The antibody or the antigen-binding fragment thereof according to any one of claims 1 to 4, wherein:
(1) the light chain variable region comprises the sequences set forth in SEQ ID NOs: 19, 21, 23, 25, 27, 29, 31, 33, 35, 37, 39, 41, 43, 45, 47, 49, 338-339, 343-346, 349-355, 359-361, 364-365, and 374-375 or sequences having 80%, 85%, 90%, 95%, 96%, 97%, 98%, 99%, or higher identity to the sequences set forth in SEQ ID NOs: 19, 21, 23, 25, 27, 29, 31, 33, 35, 37, 39, 41, 43, 45, 47, 49, 338-339, 343-346, 349-355, 359-361, 364-365, and 374-375; and
(2) the heavy chain variable region comprises the sequences set forth in SEQ ID NOs: 18, 20, 22, 24, 26, 28, 30, 32, 34, 36, 38, 40, 42, 44, 46, 48, 340-342, 347, 356, 362-363, 366-370, and 376-377 or sequences having 80%, 85%, 90%, 95%, 96%, 97%, 98%, 99%, or higher identity to the sequences set forth in SEQ ID NOs: 18, 20, 22, 24, 26, 28, 30, 32, 34, 36, 38, 40, 42, 44, 46, 48, 340-342, 347, 356, 362-363, 366-370, and 376-377.

6. The antibody or the antigen-binding fragment thereof according to any one of claims 1 to 5, wherein the antibody or the antigen-binding fragment thereof is chimeric, humanized, or fully human-derived.

7. The antibody or the antigen-binding fragment thereof according to any one of claims 1 to 6, wherein the antibody or the antigen-binding fragment thereof is capable of binding to human, monkey, or murine delta-like ligand 3 (DLL3).

8. The antibody or the antigen-binding fragment thereof according to any one of claims 1 to 7, further comprising a constant region sequence in any one of human or murine IgG1, IgG2, IgG3, IgG4, IgA, IgM, IgE, and IgD antibodies, preferably a constant region sequence of a human or murine IgG1, IgG2, IgG3, or IgG4 antibody or a constant region sequence of a human or murine IgG1, IgG2, IgG3, or IgG4 antibody with a mutation, wherein
further, the antibody or the antigen-binding fragment thereof is also conjugated to a therapeutic agent or a tracer;
preferably, the therapeutic agent is selected from a radioisotope, a chemotherapeutic agent, a cytotoxic agent, or an immunomodulator;
the tracer is selected from a radiocontrast medium, a paramagnetic ion, a metal, a fluorescent label, a chemiluminescent label, an ultrasound contrast agent, and a photosensitizer; more preferably, the cytotoxic agent is selected from alkaloids, methotrexate, anthracycline antibiotics (doxorubicin), pyrrolobenzodiazepine (PBD) or gemcitabine, cytarabine, tegafur, ifosfamide, dacarbazine, and oxaliplatin; more preferably, the cytotoxic agent is taxanes.

9. The antibody or the antigen-binding fragment thereof according to any one of claims 1 to 8, wherein the antigen-binding fragment is selected from one or more of a F(ab')₂, a Fab', a Fab, an Fv, an scFv, a nanobody, or an affibody.

10. A multispecific antigen-binding molecule, comprising the antibody or the antigen-binding fragment thereof according to any one of claims 1 to 9, an antigen-binding molecule that binds to an antigen other than DLL3, and/or an antigen-binding molecule that binds to a DLL3 epitope different from a DLL3 epitope to which the antibody or the antigen-binding fragment thereof according to any one of claims 1 to 9 binds, wherein
preferably, the additional antigen-binding molecule is an antibody or an antigen-binding fragment thereof;
preferably, the multispecific antigen-binding molecule can be bispecific, trispecific, or tetraspecific; preferably, the multispecific antigen-binding molecule can be divalent, trivalent, tetravalent, pentavalent, or hexavalent.

11. An isolated nucleic acid fragment, wherein the isolated nucleic acid fragment encodes the antibody or the antigen-binding fragment thereof according to any one of claims 1 to 9 or the multispecific antigen-binding molecule according to claim 12.

12. A recombinant vector, comprising the isolated nucleic acid fragment according to claim 11.

13. A host cell, comprising the recombinant vector according to claim 12, wherein preferably, the cell is a prokaryotic cell or a eukaryotic cell, e.g., a bacterium (*E. coli*), a fungus (yeast), an insect cell, or a mammalian cell (a CHO cell line or a 293T cell line).

14. A method for preparing the antibody or the antigen-binding fragment thereof according to any one of claims 1 to 9 or the multispecific antigen-binding molecule according to claim 10, comprising: culturing the host cell according to claim 13, and isolating an antibody, an antigen-binding fragment, or a multispecific antigen-binding molecule expressed by the cell.

15. A pharmaceutical composition, comprising the antibody or the antigen-binding fragment thereof according to any one of claims 1-9, the multispecific antigen-binding molecule according to claim 10, the nucleic acid fragment according to claim 11, the vector according to claim 12, or a product prepared by the method according to claim 14, wherein optionally, the pharmaceutical composition further comprises a pharmaceutically acceptable carrier, a diluent, or an adjuvant.

16. The pharmaceutical composition according to claim 15, further comprising an additional antineoplastic agent.

17. A method for treating a tumor or cancer, comprising: administering to a subject an effective amount of the antibody or the antigen-binding fragment thereof according to any one of claims 1 to 9, the multispecific antigen-binding molecule according to claim 10, the nucleic acid fragment according to claim 11, the vector according to claim 12, a product prepared by the method according to claim 14, or the pharmaceutical composition according to claim 15 or 16, wherein preferably, the tumor or cancer is selected from a solid tumor, a hematologic tumor, or a DLL3-expressing infiltrative cancer;
preferably, the tumor or cancer is selected from at least one of small cell lung cancer, glioma, pancreatic cancer, melanoma, breast cancer, pituitary tumor, endometrioma, acute myeloid leukemia, liver cancer, bladder cancer, colon cancer, prostate cancer, kidney cancer, and esophageal cancer.

18. Use of the antibody or the antigen-binding fragment thereof according to any one of claims 1 to 9, the multispecific antigen-binding molecule according to claim 10, the nucleic acid fragment according to claim 11, the vector according to claim 12, a product prepared by the method according to claim 14, or the pharmaceutical composition according to claim 15 or 16 in preparing a medicament for treating a tumor or cancer, wherein preferably, the tumor or cancer is selected from a solid tumor, a hematologic tumor, or a DLL3-expressing infiltrative cancer;
preferably, the tumor or cancer is selected from at least one of small cell lung cancer, glioma, pancreatic cancer, melanoma, breast cancer, pituitary tumor, endometrioma, acute myeloid leukemia, liver cancer, bladder cancer, colon cancer, prostate cancer, kidney cancer, and esophageal cancer.

19. A kit, comprising the antibody or the antigen-binding fragment thereof according to any one of claims 1 to 9, the multispecific antigen-binding molecule according to claim 10, the nucleic acid fragment according to claim 11, the vector according to claim 12, a product prepared by the method according to claim 14, or the pharmaceutical composition according to claim 15 or 16.

20. A method for detecting expression of DLL3 in a biological sample, comprising contacting the biological sample with the antibody or the antigen-binding fragment thereof according to any one of claims 1 to 9 under conditions that allow the antibody or the antigen-binding fragment thereof and DLL3 to form a complex, wherein preferably, the method further comprises detecting formation of the complex and indicating presence or an expression level of DLL3 in the sample.

21. Use of the antibody or the antigen-binding fragment thereof according to any one of claims 1 to 9 in preparing a DLL3 detection reagent.
